# EUROPEAN PATENT APPLICATION

(11) **EP 4 512 805 A1**
(43) Date of publication of application: **26.02.2025**
(21) Application number: 23791259.7
(22) Date of filing: 19.04.2023
(51) Int. Cl.: C07D 405/04, A61K 31/505, A61P 31/14, C07H 19/10

(54) **ANTIVIRAL NUCLEOSIDE ANALOGUE, AND PHARMACEUTICAL COMPOSITION AND USE THEREOF**

(30) Priority: 20.04.2022 CN 202210417953; 19.09.2022 CN 202211140951; 18.01.2023 CN 202310086663
(71) Applicant: Shanghai Institute of Materia Medica, Chinese Academy of Sciences, Shanghai 201203 (CN); Vigonvita Life Sciences Co., Ltd., Suzhou, Jiangsu 215123 (CN); Wuhan Institute Of Virology, Chinese Academy of Sciences, Wuhan, Hubei 430071 (CN); Vigonvita Shanghai Co., Ltd., Shanghai 201210 (CN)
(72) Inventor: SHEN, Jingshan, Shanghai 201203 (CN); XIE, Yuanchao, Shanghai 201203 (CN); ZHANG, Leike, Wuhan, Hubei 430071 (CN); CHENG, Yong, Suzhou, Jiangsu 215123 (CN); PENG, Ke, Wuhan, Hubei 430071 (CN); TIAN, Guanghui, Suzhou, Jiangsu 215123 (CN); XIAO, Gengfu, Wuhan, Hubei 430071 (CN); LI, Jian, Suzhou, Jiangsu 215123 (CN); YU, Jingjin, Suzhou, Jiangsu 215123 (CN); HU, Tianwen, Shanghai 201210 (CN)
(74) Representative: dompatent von Kreisler Selting Werner - Partnerschaft von Patent- und Rechtsanwälten mbB
(86) International application number: PCT/CN2023/089155
(87) International publication number: WO 2023/202604

(57) **Abstract**

The present invention falls within the technical field of medicinal chemistry, and specifically relates to an antiviral nucleoside analogue, and a pharmaceutical composition and the use thereof. The nucleoside analogue of the present invention has a structure as represented by formula (I), and has a good chemical stability, a high oral bioavailability, and a significant antiviral activity. The nucleoside analogue can be used in the preparation of an inhibitor for inhibiting viral replication and/or a drug for preventing, alleviating and/or treating diseases caused by viral infection.

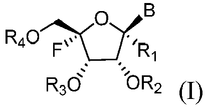

## Description

The present application claims priority to Chinese Patent Application No. 202210417953.2 filed on April 20, 2022, Chinese Patent Application No. 202211140951.X filed on September 19, 2022, and Chinese Patent Application No. 202310086663.9 filed on January 18, 2023, all entitled "ANTIVIRAL NUCLEOSIDE ANALOGUE, AND PHARMACEUTICAL COMPOSITION AND USE THEREOF", which are incorporated herein by reference in their entireties.

### TECHNICAL FIELD

The present invention relates to the technical field of pharmaceutical chemistry, and particularly to an antiviral nucleoside analogue, and a pharmaceutical composition thereof and antiviral use thereof.

### BACKGROUND

Infectious diseases caused by viruses pose a great threat to human life and health. Respiratory syncytial virus RSV belongs to the family Paramyxoviridae, and is the most common viral pathogen causing acute lower respiratory infections in infants and young children, resulting in millions of hospitalizations of children worldwide each year. Influenza viruses belong to the family Orthomyxoviridae and are RNA viruses, which are very easy to mutate, have various subtypes, and have been recorded to cause a plurality of global pandemics, causing a great number of deaths and property losses. Bunya viruses are a class of segmented negative-stranded RNA viruses that contain many important pathogens causing infections in humans and animals. In recent years, death from tick bites resulting in novel bunya virus infections in humans and animals has occurred every year worldwide. In addition to the above viruses, arenaviruses, viruses of the family Flaviviridae (such as a Dengue virus, a hepatitis C virus, and a Zika virus), filoviruses (such as an Ebola virus), and coronaviruses (such as novel coronaviruses and animal coronaviruses) are equally serious health risks to humans.

Nucleoside analogues are the most important class of antiviral drugs. Most of the nucleoside drugs on the market are directed to chronic viral infectious diseases, such as tenofovir, entecavir, lamivudine, and abacavir, while there are few nucleoside drugs on the market directed to acute viral infectious diseases.

The nucleoside analogues remdesivir and molnupiravir have broad-spectrum antiviral effects. Remdesivir was approved by the FDA for the treatment of COVID-19 in October 2020, and molnupiravir received an emergency use authorization in December 2021.

There are no small molecule therapeutic drugs yet available for many diseases caused by RNA virus (such as a respiratory syncytial virus RSV, a bunya virus, and an Ebola virus) infections. 4'-Fluorouracil nucleoside and 4'-fluorocytosine nucleoside have inhibitory effects on various RNA viruses, but the two compounds have poor chemical stability and druggability.

### SUMMARY

### Problems to be solved by the present invention

The present invention aims to provide a nucleoside analogue with good chemical stability, good pharmacokinetic properties, and significant antiviral activity, a pharmaceutical composition comprising the same, and new antiviral use thereof.

### Solutions to the problems

In a first aspect, the present invention provides a compound represented by formula (I), or a pharmaceutically acceptable salt, a solvate, a stereoisomer, a geometric isomer, an isotopically labeled compound or a prodrug thereof 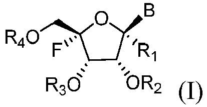 wherein,
B is selected from 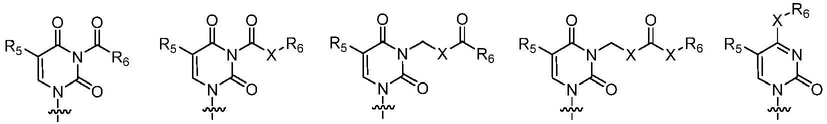 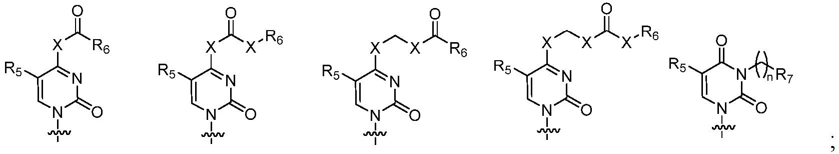
X is selected from O, NH, and S;
R₅ is selected from hydrogen, deuterium, and halogen;
R₆ is selected from C₁₋₂₀ alkyl, C₃₋₂₀ cycloalkyl, 3- to 20-membered heterocycloalkyl, C₂₋₂₀ alkenyl, C₂₋₂₀ alkynyl, C₆₋₂₀ aryl, 5- to 15-membered heteroaryl, amino C₁₋₂₀ alkyl, C₃₋₂₀ cycloalkyl C₁₋₂₀ alkyl, C₁₋₂₀ alkylamino C₁₋₂₀ alkyl, C₁₋₂₀ dialkylamino C₁₋₂₀ alkyl, C₃₋₂₀ cycloalkylamino C₁₋₂₀ alkyl, C₁₋₂₀ alkoxy C₁₋₂₀ alkyl, C₃₋₂₀ cycloalkoxy C₁₋₂₀ alkyl, a group after removal of carboxyl from an amino acid, R₂O-C₁₋₂₀ alkyl, R₂NH-C₁₋₂₀ alkyl, R₂O-C₃₋₂₀ cycloalkyl, and R₂NH-C₃₋₂₀ cycloalkyl;
n is selected from an integer of 1-6;
R₇ is 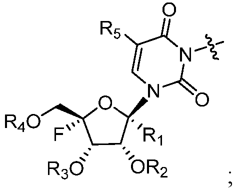
R₁ is selected from hydrogen and deuterium;
R₂ and R₃ are each independently selected from hydrogen, C₁₋₂₀ alkanoyl, C₃₋₂₀ cycloalkanoyl, 3- to 20-membered heterocycloalkanoyl, C₂₋₂₀ alkenylacyl, C₂₋₂₀ alkynylacyl, C₆₋₂₀ aroyl, 5- to 15-membered heteroaroyl, amino C₁₋₂₀ alkanoyl, C₁₋₂₀ alkylamino C₁₋₂₀ alkanoyl, C₁₋₂₀ dialkylamino C₁₋₂₀ alkanoyl, C₃₋₂₀ cycloalkylamino C₁₋₂₀ alkanoyl, C₁₋₂₀ alkoxy C₁₋₂₀ alkanoyl, C₃₋₂₀ cycloalkoxy C₁₋₂₀ alkanoyl, and a group after removal of hydroxyl in carboxyl from an amino acid, wherein the C₁₋₂₀ alkanoyl and the C₃₋₂₀ cycloalkanoyl are unsubstituted or substituted with halogen,
or R₂ and R₃ are connected with each other to form 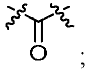
R₄ is selected from hydrogen, C₁₋₂₀ alkanoyl, C₃₋₂₀ cycloalkanoyl, 3- to 20-membered heterocycloalkanoyl, C₂₋₂₀ alkenylacyl, C₂₋₂₀ alkynylacyl, C₆₋₂₀ aroyl, 5- to 15-membered heteroaroyl, amino C₁₋₂₀ alkanoyl, C₁₋₂₀ alkylamino C₁₋₂₀ alkanoyl, C₁₋₂₀ dialkylamino C₁₋₂₀ alkanoyl, C₃₋₂₀ cycloalkylamino C₁₋₂₀ alkanoyl, C₁₋₂₀ alkoxy C₁₋₂₀ alkanoyl, C₃₋₂₀ cycloalkoxy C₁₋₂₀ alkanoyl, a group after removal of hydroxyl in carboxyl from an amino acid, 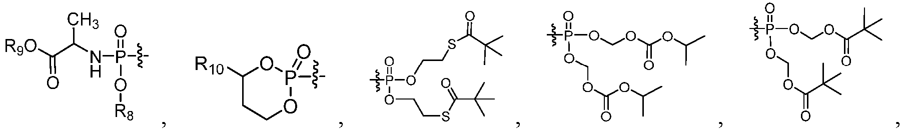 and 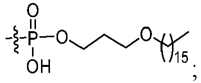
R₈ is selected from C₆₋₂₀ aryl and 5- to 15-membered heteroaryl;
R₉ is selected from C₁₋₂₀ alkyl and C₆₋₂₀ arylmethylene;
R₁₀ is selected from C₁₋₆ alkyl, C₃₋₆ cycloalkyl, C₆₋₂₀ aryl, and 5- to 15-membered heteroaryl. In some preferred embodiments,
B, X, R₁, and R₅ are as defined above;
R₆ is selected from C₁₋₂₀ alkyl, C₃₋₁₀ cycloalkyl, 3- to 10-membered heterocycloalkyl, C₂₋₁₀ alkenyl, C₃₋₁₀ alkynyl, C₆₋₁₂ aryl, 5- to 12-membered heteroaryl, amino C₁₋₁₀ alkyl, C₃₋₁₀ cycloalkyl C₁₋₁₀ alkyl, C₁₋₁₀ alkylamino C₁₋₁₀ alkyl, C₁₋₁₀ dialkylamino C₁₋₁₀ alkyl, C₃₋₁₀ cycloalkylamino C₁₋₁₀ alkyl, C₁₋₁₀ alkoxy C₁₋₁₀ alkyl, C₃₋₁₀ cycloalkoxy C₁₋₁₀ alkyl, a group after removal of carboxyl from an amino acid, R₂O-C₁₋₁₀ alkyl, R₂NH-C₁₋₁₀ alkyl, R₂O-C₃₋₁₀ cycloalkyl, and R₂NH-C₃₋₁₀ cycloalkyl;
n is selected from an integer of 1-4;
R₇ is 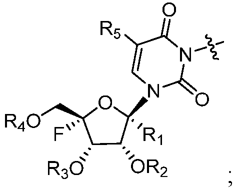
R₂ and R₃ are each independently selected from hydrogen, C₂₋₁₀ alkanoyl, C₃₋₁₀ cycloalkanoyl, 3- to 10-membered heterocycloalkanoyl, C₃₋₁₀ alkenylacyl, C₃₋₁₀ alkynylacyl, C₆₋₁₂ aroyl, 5- to 10-membered heteroaroyl, amino C₁₋₁₀ alkanoyl, C₁₋₁₀ alkylamino C₁₋₁₀ alkanoyl, C₁₋₁₀ dialkylamino C₁₋₁₀ alkanoyl, C₃₋₁₀ cycloalkylamino C₁₋₁₀ alkanoyl, C₁₋₁₀ alkoxy C₁₋₁₀ alkanoyl, C₃₋₁₀ cycloalkoxy C₁₋₁₀ alkanoyl, and a group after removal of hydroxyl in carboxyl from an amino acid, wherein the C₂₋₁₀ alkanoyl and the C₃₋₁₀ cycloalkanoyl are unsubstituted or substituted with halogen;
or R₂ and R₃ are connected with each other to form 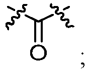
R₄ is selected from hydrogen, C₂₋₂₀ alkanoyl, C₃₋₁₀ cycloalkanoyl, 3- to 10-membered heterocycloalkanoyl, C₃₋₁₀ alkenylacyl, C₃₋₁₀ alkynylacyl, C₆₋₁₅ aroyl, 5- to 12-membered heteroaroyl, amino C₁₋₁₀ alkanoyl, C₁₋₁₀ alkylamino C₁₋₁₀ alkanoyl, C₁₋₁₀ dialkylamino C₁₋₁₀ alkanoyl, C₃₋₁₀ cycloalkylamino C₁₋₁₀ alkanoyl, C₁₋₁₀ alkoxy C₁₋₁₀ alkanoyl, C₃₋₁₀ cycloalkoxy C₁₋₁₀ alkanoyl, a group after removal of hydroxyl in carboxyl from an amino acid, 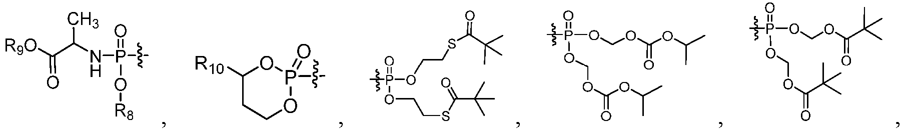 and 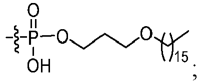
R₈ is selected from C₆₋₁₄ aryl and 5- to 12-membered aryl;
R₉ is selected from C₂₋₁₀ alkyl and C₆₋₁₅ arylmethylene;
R₁₀ is selected from C₁₋₆ alkyl, C₃₋₆ cycloalkyl, C₆₋₁₄ aryl, and 5- to 12-membered heteroaryl.

In some embodiments, the compound represented by formula (I), or the pharmaceutically acceptable salt, the solvate, the stereoisomer, the geometric isomer, the isotopically labeled compound or the prodrug thereof is represented by the following formula (II): 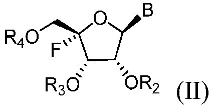 wherein,
B is selected from 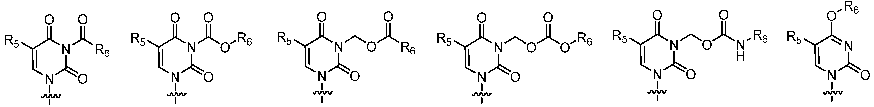 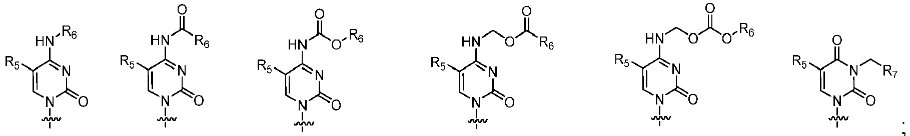
R₅ is selected from hydrogen and deuterium;
R₂, R₃, R₄, R₆, and R₇ have definitions as described above in formula (I).

In some preferred embodiments, R₆ is selected from C₁₋₂₀ alkyl, C₃₋₂₀ cycloalkyl, 3- to 20-membered heterocycloalkyl, C₂₋₂₀ alkenyl, C₆₋₂₀ aryl, 5- to 15-membered heteroaryl, C₃₋₂₀ cycloalkyl C₁₋₂₀ alkyl, C₁₋₂₀ alkylamino C₁₋₂₀ alkyl, C₁₋₂₀ dialkylamino C₁₋₂₀ alkyl, a group after removal of carboxyl from an amino acid, and R₂O-C₁₋₂₀ alkyl; wherein R₂ is C₁₋₂₀ alkanoyl;
preferably, R₆ is selected from C₁₋₂₀ alkyl, C₃₋₁₀ cycloalkyl, 3- to 10-membered heterocycloalkyl, C₃₋₁₀ alkenyl, C₆₋₁₂ aryl, 5- to 12-membered heteroaryl, C₃₋₁₀ cycloalkyl C₁₋₁₀ alkyl, C₁₋₁₀ alkylamino C₁₋₁₀ alkyl, C₁₋₁₀ dialkylamino C₁₋₁₀ alkyl, a group after removal of carboxyl from an amino acid, and R₂O-C₁₋₁₀ alkyl; wherein R₂ is C₂₋₁₀ alkanoyl;
more preferably, R₆ is selected from C₁₋₁₆ alkyl, C₃₋₈ cycloalkyl, 3- to 8-membered heterocycloalkyl, C₃₋₈ alkenyl, C₆₋₁₀ aryl, 5- to 10-membered heteroaryl, C₃₋₈ cycloalkyl C₁₋₈ alkyl, C₁₋₄ alkylamino C₁₋₈ alkyl, C₁₋₄ dialkylamino C₁₋₈ alkyl, a group after removal of carboxyl from an amino acid, and R₂O-C₁₋₈ alkyl; wherein R₂ is C₂₋₈ alkanoyl; preferably, the 3- to 8-membered heterocycloalkyl is azacycloalkyl, e.g., azetidin-3-yl, pyrrolidinyl, or piperidin-4-yl; preferably, the C₆₋₁₀ aryl is selected from phenyl or naphthyl; preferably, the 5-to 10-membered heteroaryl is 5- to 6-membered heteroaryl selected from pyridinyl, pyrimidinyl, pyrazinyl, furanyl, and thienyl, preferably N-containing heteroaryl, including pyridin-2-yl, pyridin-3-yl, pyridin-4-yl, pyrimidin-4-yl, pyrimidin-5-yl, and pyrazin-2-yl; the amino acid is preferably selected from L-alanine, L-valine, and L-phenylglycine;
the other substituents each have definitions as defined above.

In some preferred embodiments, R₆ is selected from C₁₋₂₀ alkyl, C₃₋₂₀ cycloalkyl, 3- to 20-membered heterocycloalkyl, C₂₋₂₀ alkenyl, C₆₋₂₀ aryl, 5- to 15-membered heteroaryl, C₃₋₂₀ cycloalkyl C₁₋₂₀ alkyl, C₁₋₂₀ alkylamino C₁₋₂₀ alkyl, C₁₋₂₀ dialkylamino C₁₋₂₀ alkyl, a group after removal of carboxyl from an amino acid, and R₂O-C₁₋₂₀ alkyl; wherein R₂ is C₁₋₂₀ alkanoyl;
preferably, R₆ is selected from C₁₋₂₀ alkyl, C₃₋₁₀ cycloalkyl, 3- to 10-membered heterocycloalkyl, C₃₋₁₀ alkenyl, C₆₋₁₂ aryl, 5- to 12-membered heteroaryl, C₃₋₁₀ cycloalkyl C₁₋₁₀ alkyl, C₁₋₁₀ alkylamino C₁₋₁₀ alkyl, C₁₋₁₀ dialkylamino C₁₋₁₀ alkyl, a group after removal of carboxyl from an amino acid, and R₂O-C₁₋₁₀ alkyl; wherein R₂ is C₂₋₁₀ alkanoyl;
preferably, the C₆₋₁₂ aryl is selected from phenyl and naphthyl; preferably, the 5- to 12-membered heteroaryl is 5- to 6-membered heteroaryl selected from pyridinyl, pyrimidinyl, pyrazinyl, furanyl, and thienyl, preferably N-containing heteroaryl, including pyridin-2-yl, pyridin-3-yl, pyridin-4-yl, pyrimidin-4-yl, pyrimidin-5-yl, and pyrazin-2-yl; the amino acid is preferably selected from L-alanine, L-valine, and L-phenylglycine;
more preferably, R₆ is selected from C₁₋₁₆ alkyl, C₃₋₈ cycloalkyl, 3- to 8-membered heterocycloalkyl, C₃₋₈ alkenyl, C₃₋₈ cycloalkyl C₁₋₈ alkyl, C₁₋₈ alkylamino C₁₋₈ alkyl, C₁₋₈ dialkylamino C₁₋₈ alkyl, a group after removal of carboxyl from an amino acid, and R₂O-C₁₋₈ alkyl; wherein R₂ is C₂₋₈ alkanoyl; preferably, the 3- to 8-membered heterocycloalkyl is azacycloalkyl, e.g., azetidin-3-yl, pyrrolidinyl, or piperidin-4-yl;
the other substituents each have definitions as defined above.

In some preferred embodiments, R₂ and R₃ are each independently selected from hydrogen, C₁₋₂₀ alkanoyl, C₆₋₂₀ aroyl, and 5- to 20-membered heteroaroyl, or R₂ and R₃ are connected with each other to form 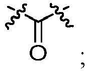 preferably, R₂ and R₃ are each independently selected from hydrogen, C₂₋₁₀ alkanoyl, C₆₋₁₂ aroyl, and 5- to 10-membered heteroaroyl, or R₂ and R₃ are connected with each other to form 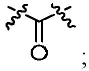 preferably, R₂ and R₃ are each independently selected from hydrogen, C₂₋₁₀ alkanoyl, C₆₋₁₀ aroyl, and 5- to 6-membered heteroaroyl, or R₂ and R₃ are connected with each other to form 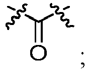 preferably, the C₆₋₁₀ aryl is selected from phenyl and naphthyl; the 5- to 6-membered heteroaryl is selected from pyridinyl, pyrimidinyl, pyrazinyl, furanyl, and thienyl, preferably N-containing heteroaryl, including pyridin-2-yl, pyridin-3-yl, pyridin-4-yl, pyrimidin-4-yl, pyrimidin-5-yl, and pyrazin-2-yl;
the other substituents each have definitions as defined above.

In some preferred embodiments, R₂ and R₃ are each independently selected from hydrogen, C₁₋₂₀ alkanoyl, C₆₋₂₀ aroyl, and 5- to 15-membered heteroaroyl, or R₂ and R₃ are connected with each other to form 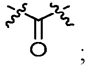 the other substituents each have definitions as defined above.

In some preferred embodiments, R₄ is selected from hydrogen, C₁₋₂₀ alkanoyl, C₃₋₂₀ cycloalkanoyl, 3- to 20-membered heterocycloalkanoyl, C₆₋₂₀ aroyl, 5- to 15-membered heteroaroyl, and a group after removal of hydroxyl in carboxyl from an amino acid; preferably, R₄ is selected from hydrogen, C₂₋₁₆ alkanoyl, C₃₋₁₀ cycloalkanoyl, 3- to 10-membered heterocycloalkanoyl, C₆₋₁₂ aroyl, 5- to 10-membered heteroaroyl, and a group after removal of hydroxyl in carboxyl from an amino acid; preferably, R₄ is selected from hydrogen, C₂₋₁₆ alkanoyl, C₃₋₆ cycloalkanoyl, 3- to 6-membered heterocycloalkanoyl, C₆₋₁₀ aroyl, 5- to 6-membered heteroaroyl, and a group after removal of hydroxyl in carboxyl from an amino acid; preferably, the C₆₋₁₀ aryl is selected from phenyl and naphthyl; preferably, the 3- to 6-membered heterocycloalkanoyl is azacycloalkanoyl, e.g., azetidin-3-ylacyl or piperidin-4-ylacyl; preferably, the 5- to 6-membered heteroaryl is selected from pyridinyl, pyrimidinyl, pyrazinyl, furanyl, and thienyl, preferably N-containing heteroaryl, including pyridin-2-yl, pyridin-3-yl, pyridin-4-yl, pyrimidin-4-yl, pyrimidin-5-yl, and pyrazin-2-yl; the amino acid is selected from L-alanine, L-valine and L-phenylglycine;
the other substituents each have definitions as defined above.

In some preferred embodiments, R₄ is selected from hydrogen, C₁₋₂₀ alkanoyl, C₃₋₂₀ cycloalkanoyl, C₆₋₂₀ aroyl, 5-to 15-membered heteroaroyl, and a group after removal of hydroxyl in carboxyl from an amino acid;
the other substituents each have definitions as defined above.

In some embodiments, the compound represented by formula (I), or the pharmaceutically acceptable salt, the solvate, the stereoisomer, the geometric isomer, the isotopically labeled compound or the prodrug thereof is represented by the following formula (III), formula (IV), or formula (V): 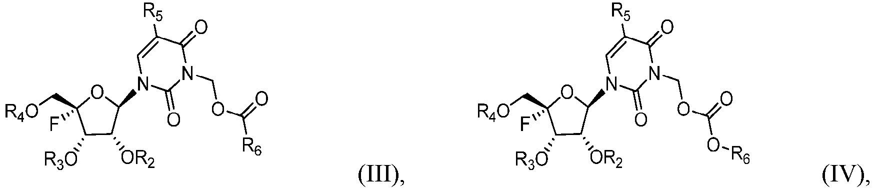 or 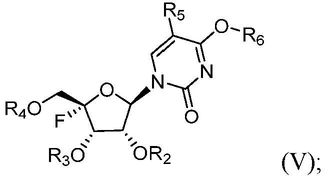 wherein,
R₅ is selected from hydrogen and deuterium;
R₂, R₃, R₄, and R₆ have definitions as described above in formula (I) or formula (II).

In some preferred embodiments, R₂ and R₃ are hydrogen.

In some preferred embodiments, R₂ and R₃ are C₁₋₂₀ alkanoyl, C₆₋₁₂ aryl, or 5- to 12-membered heteroaryl, or R₂ and R₃ are connected with each other to form

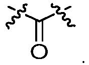

In some preferred embodiments, R₄ is C₁₋₂₀ alkanoyl.

In some preferred embodiments, R₄ is C₆₋₁₂ aryl or 5- to 12-membered heteroaryl.

In some preferred embodiments, R₅ is hydrogen.

In some preferred embodiments, R₆ is C₁₋₂₀ alkyl.

In some preferred embodiments, R₆ is C₆₋₁₂ aryl or 5- to 12-membered heteroaryl.

In a second aspect, the present invention provides compounds, or pharmaceutically acceptable salts, solvates, stereoisomers, geometric isomers, isotopically labeled compounds or prodrugs thereof as follows:

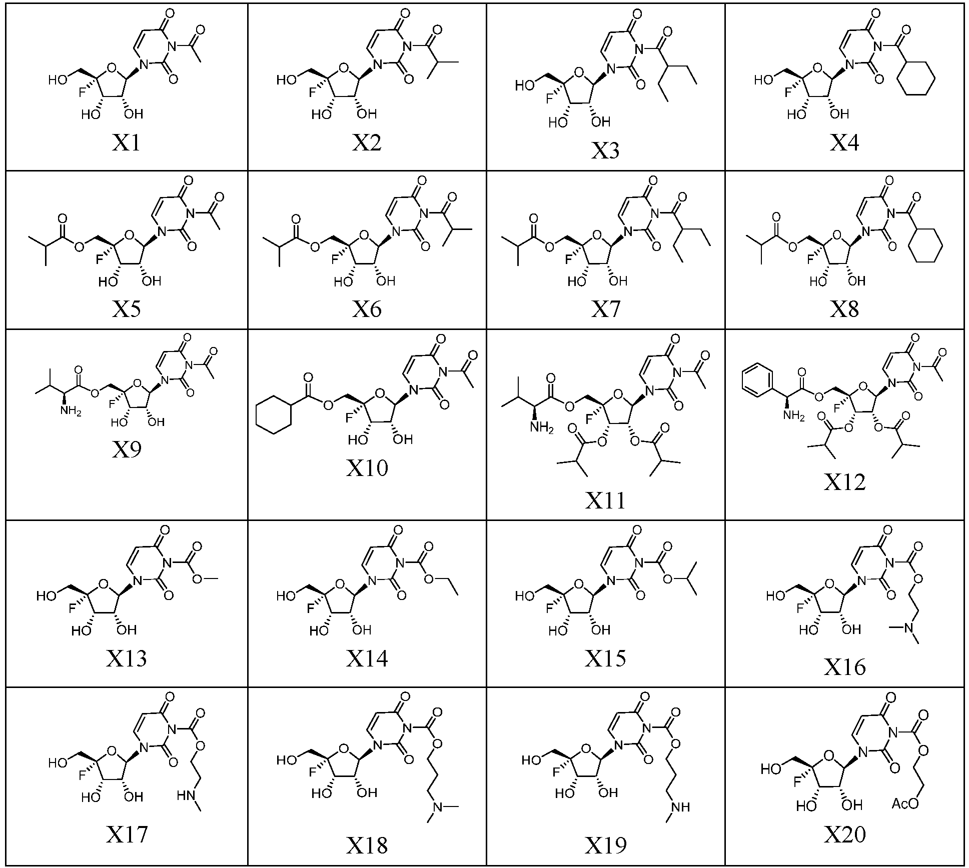

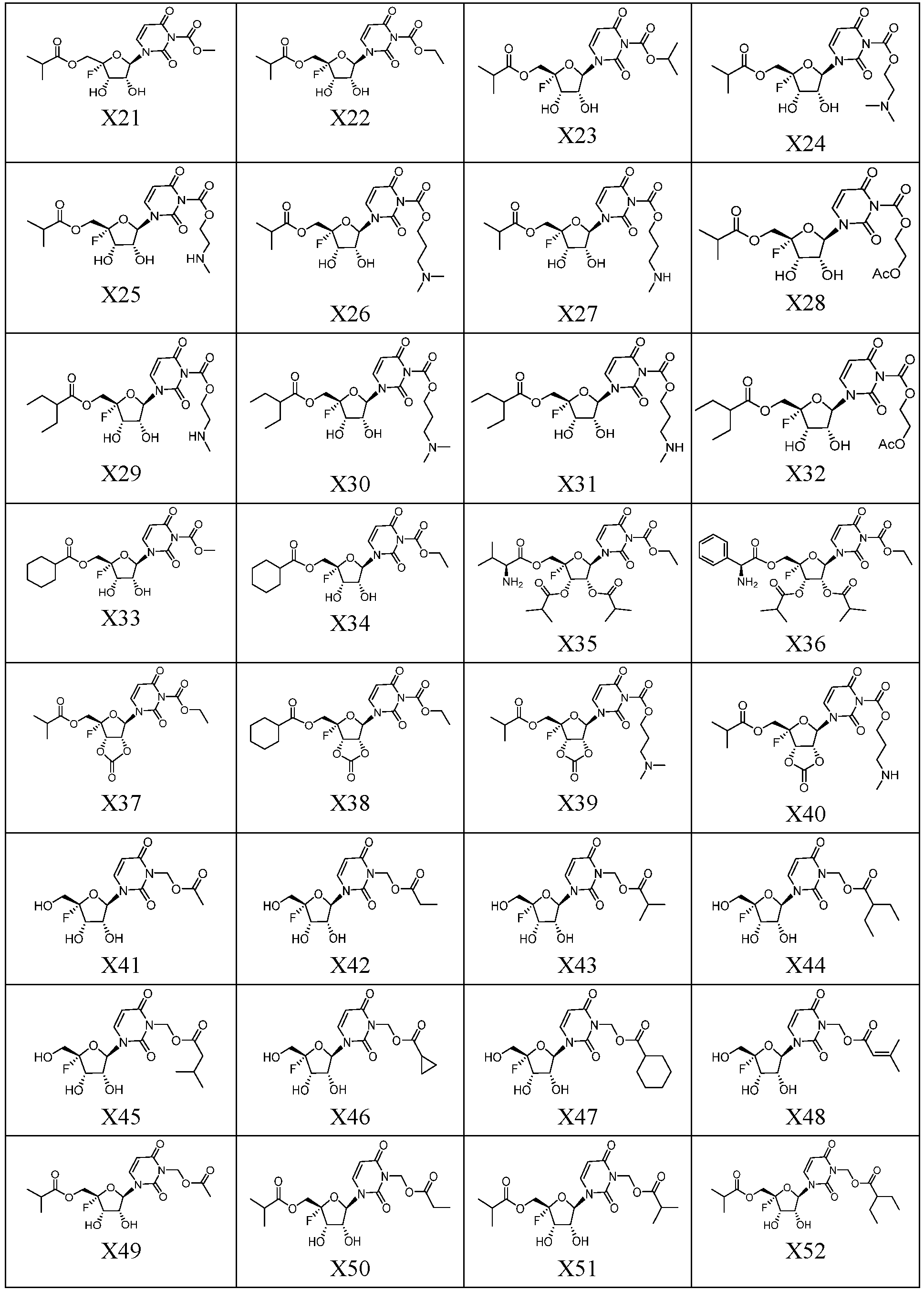

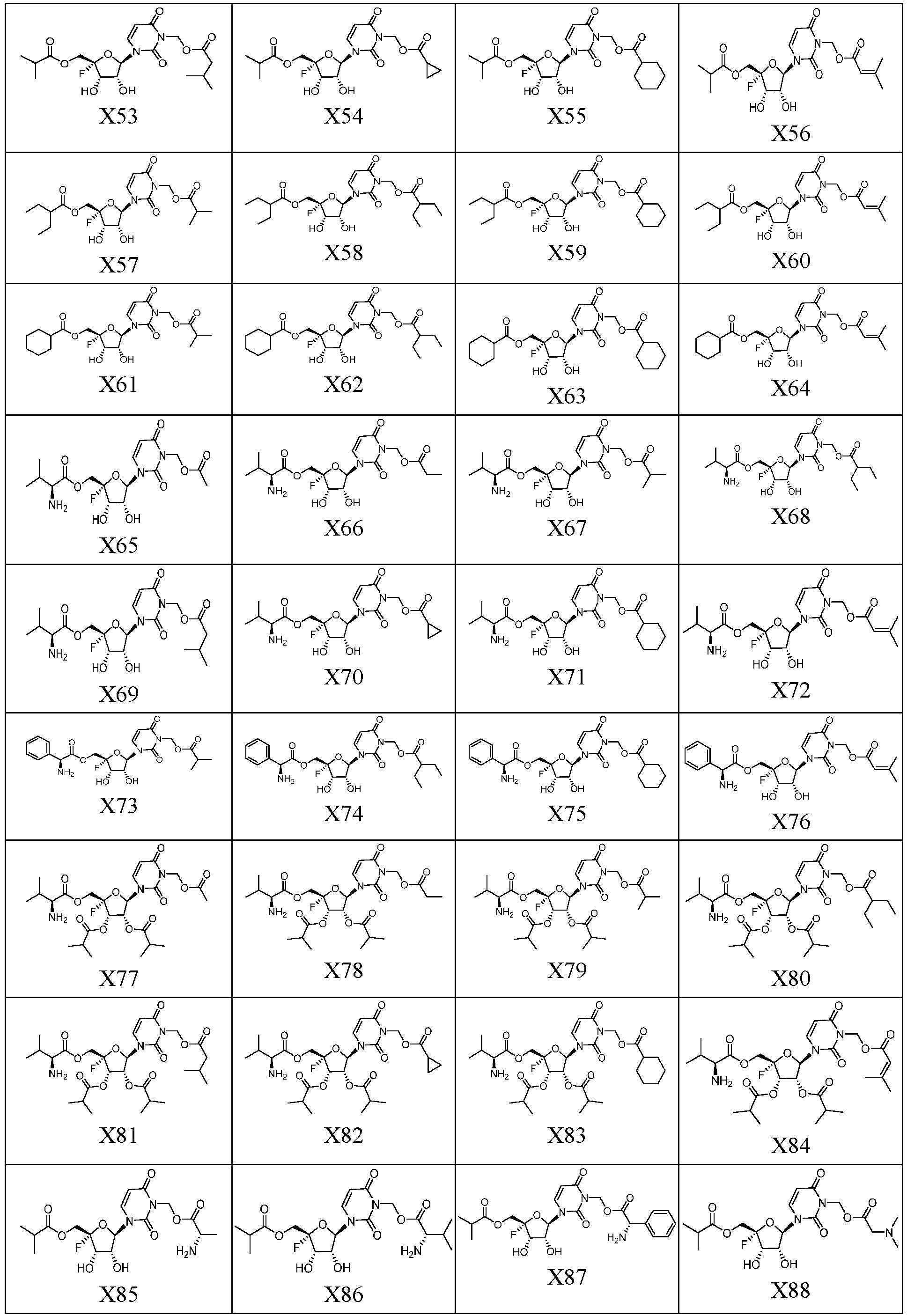

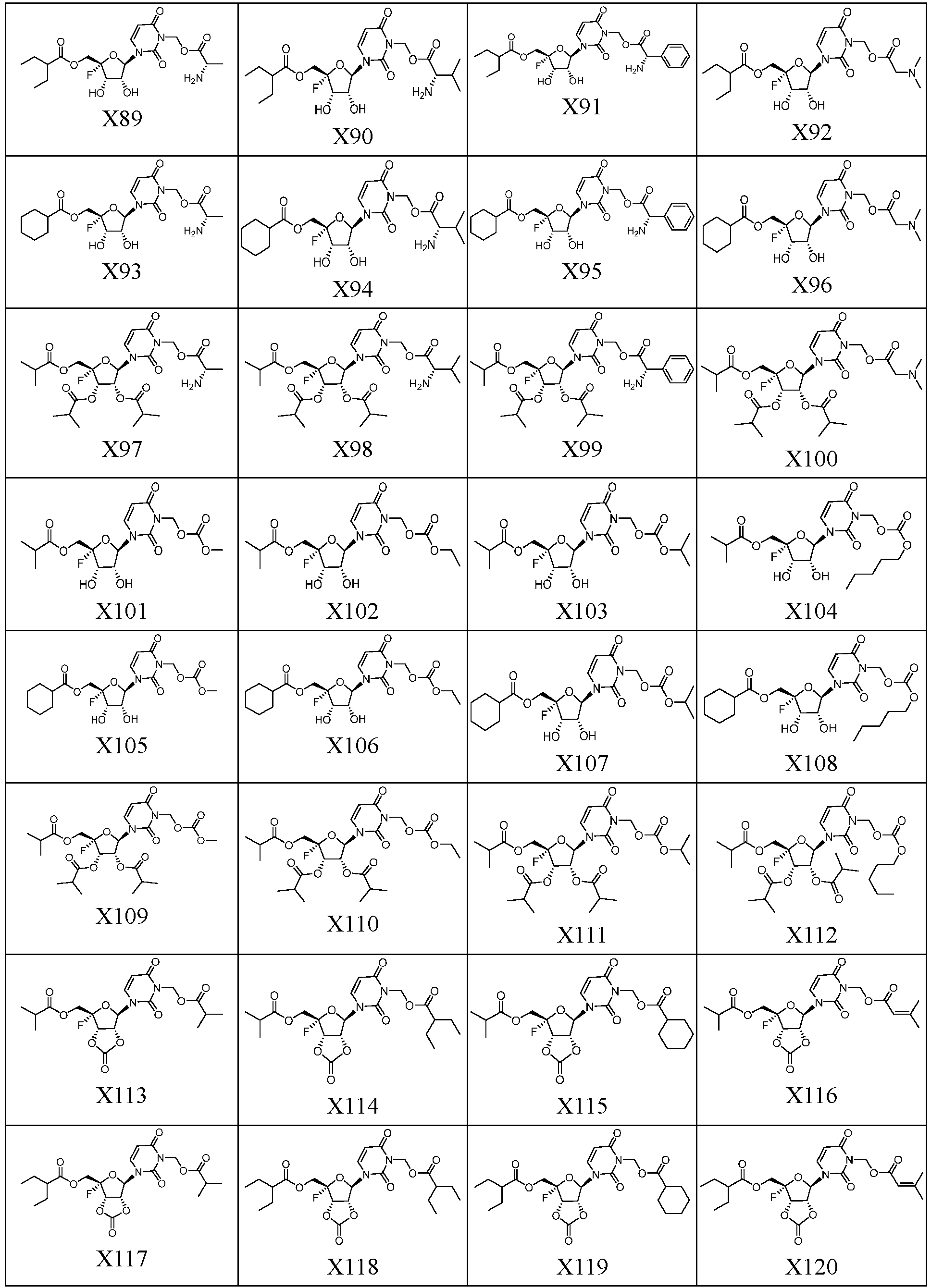

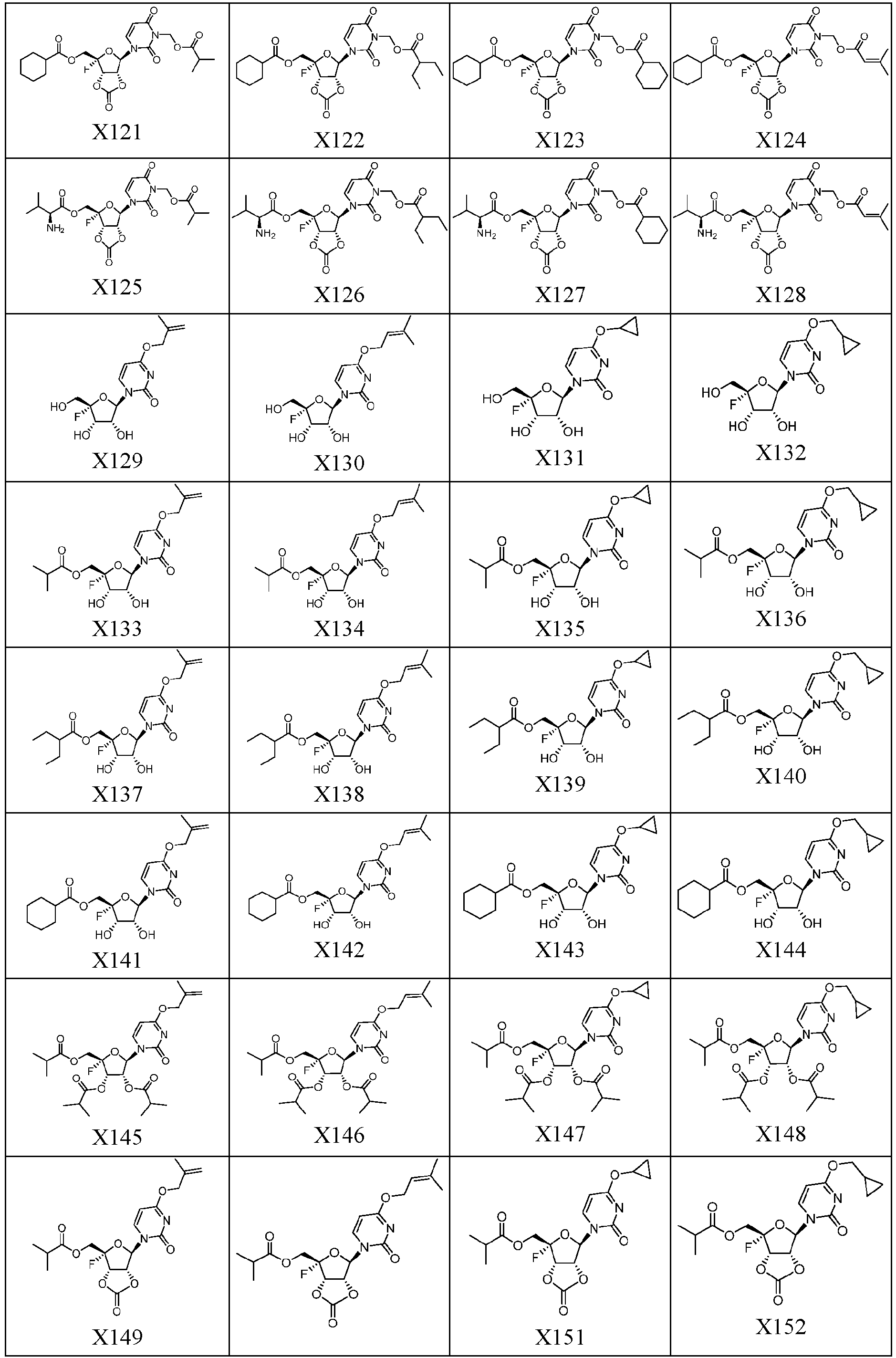

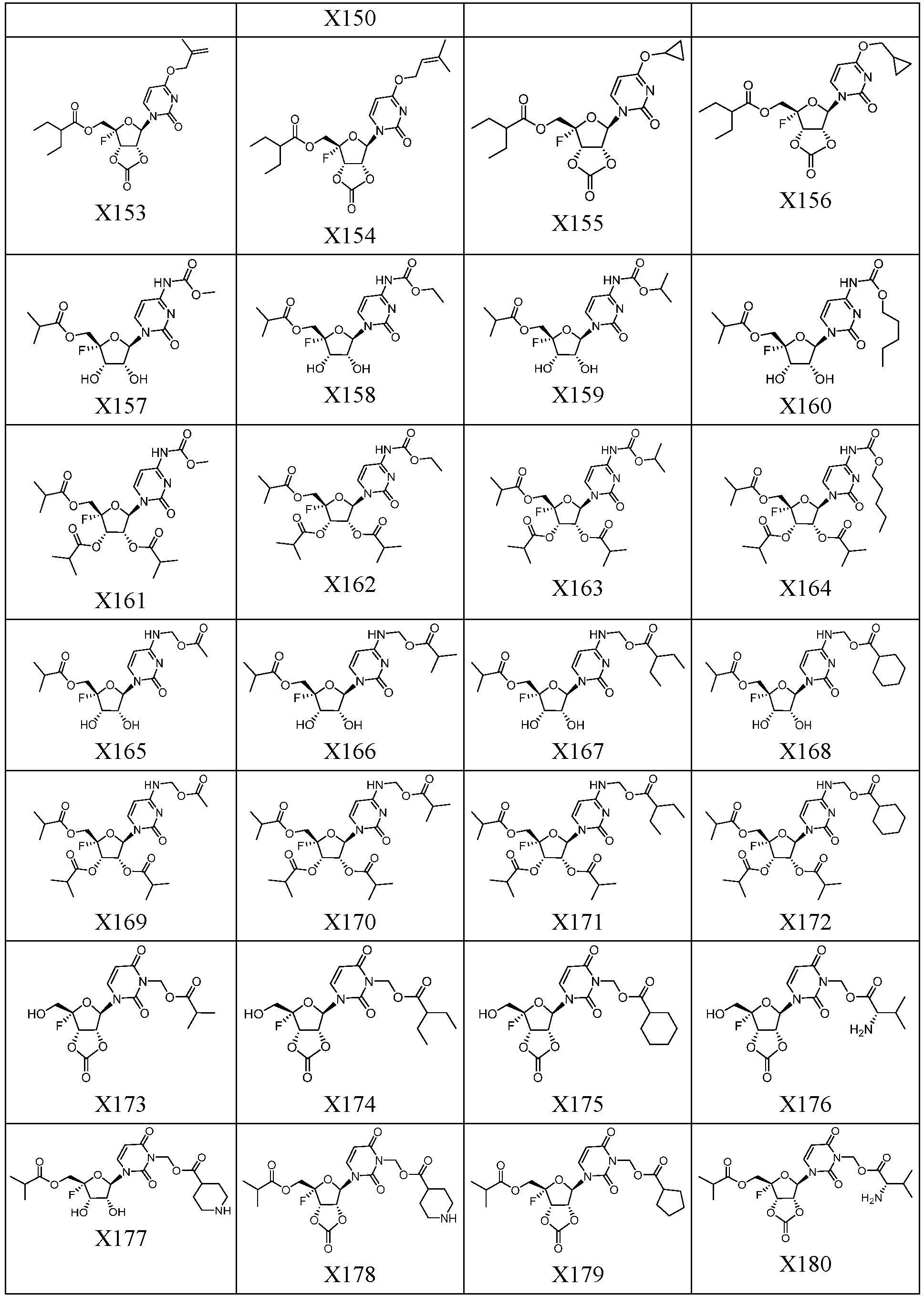

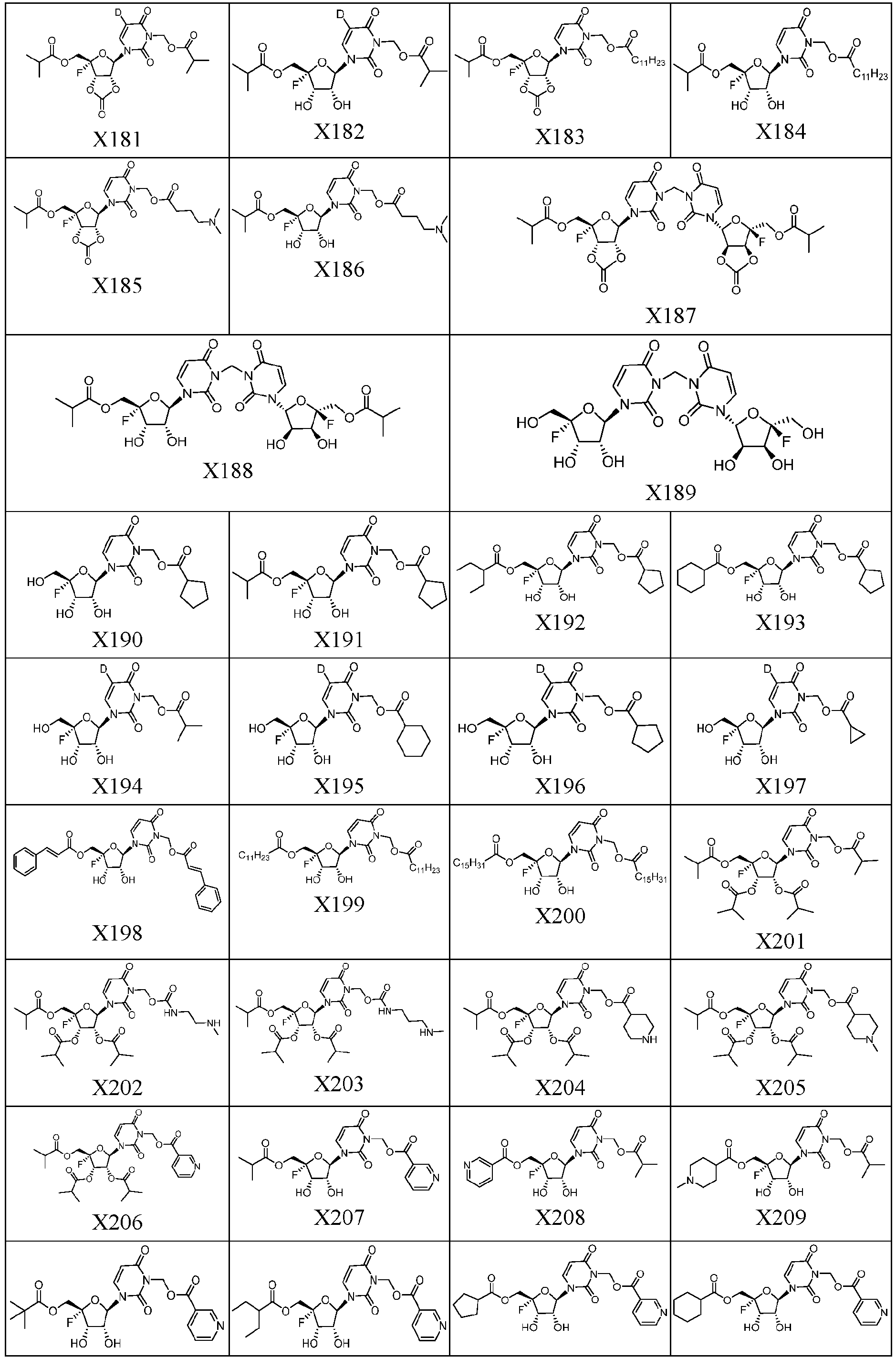

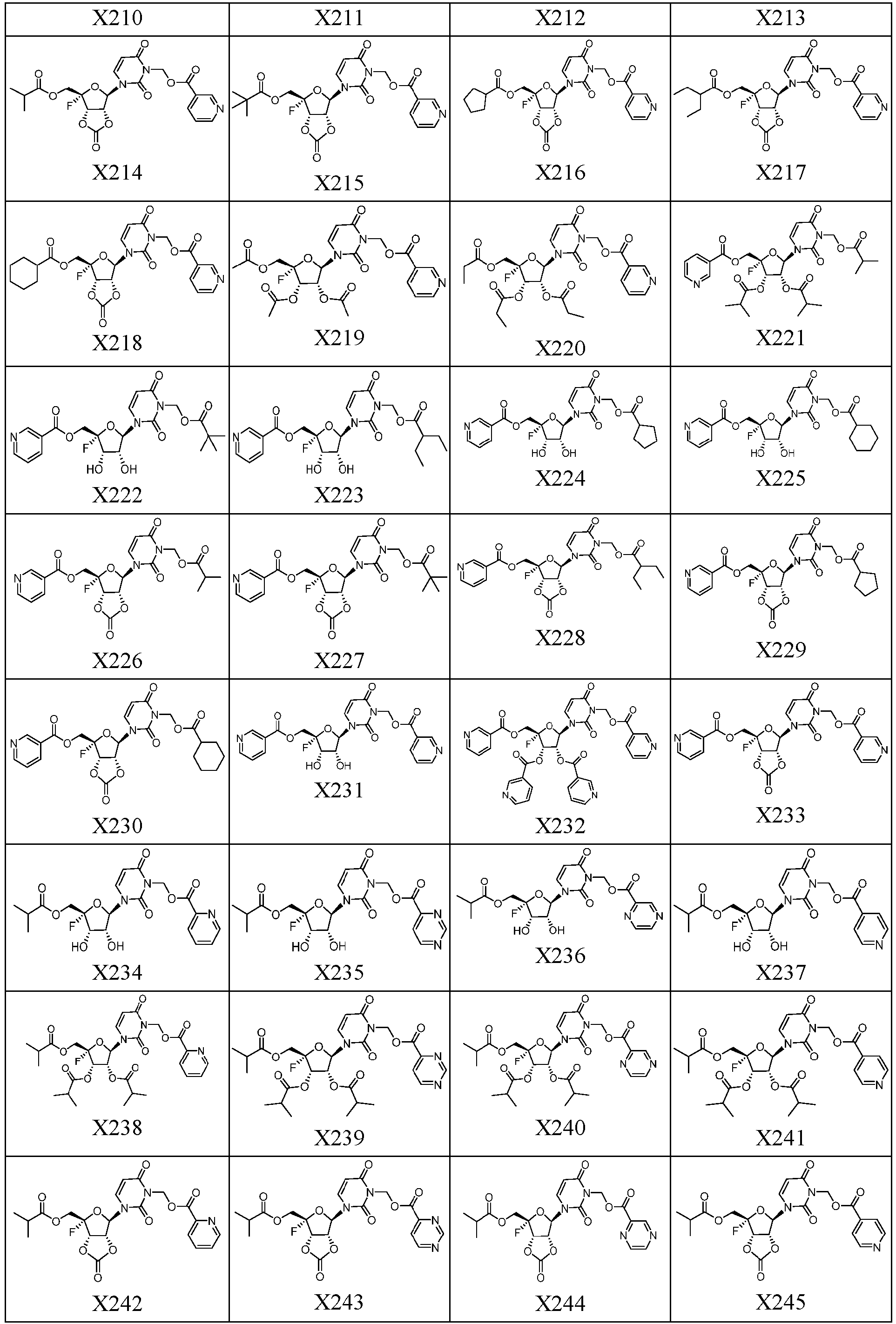

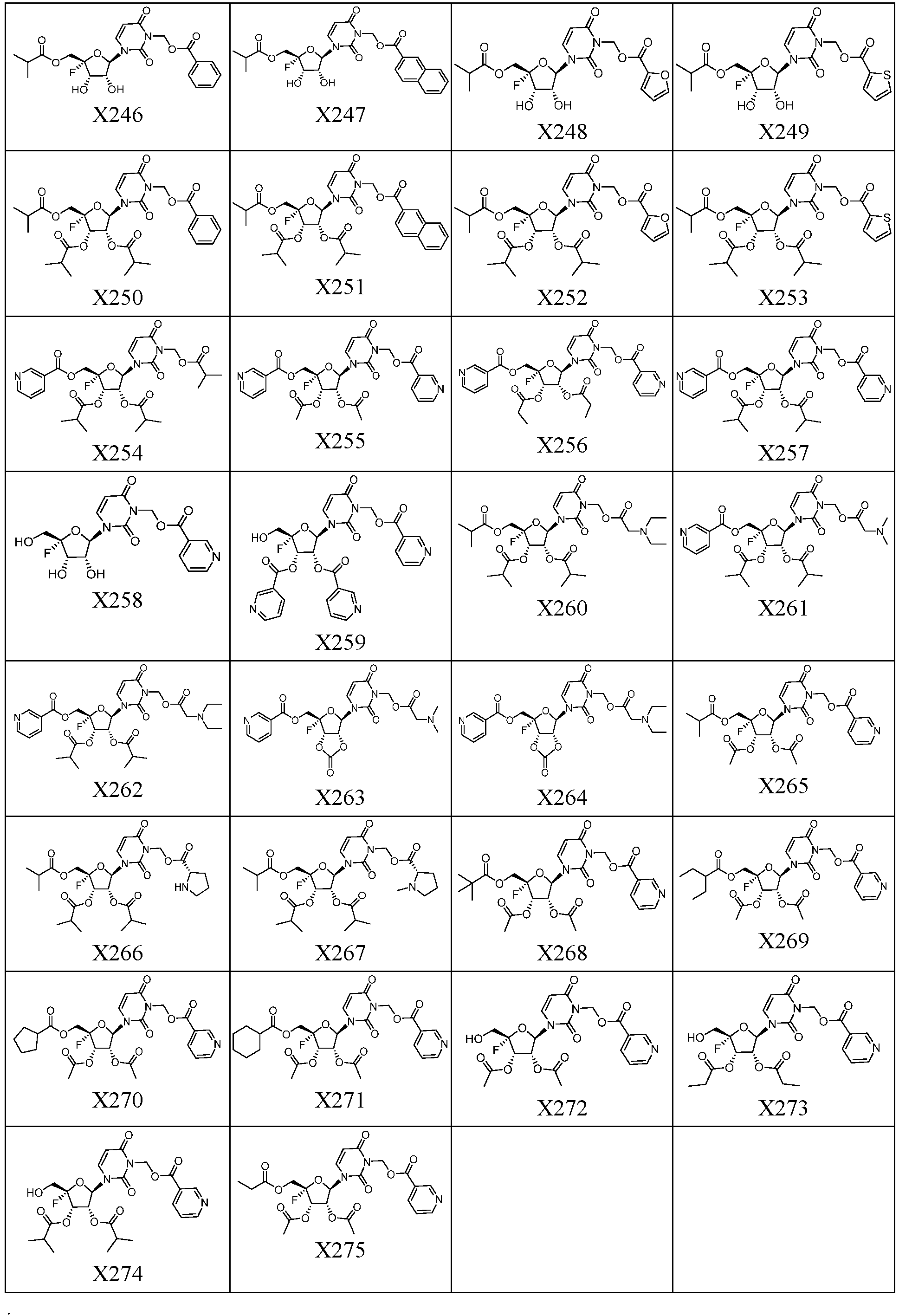

In particular, the present invention provides a pharmaceutically acceptable salt of a compound, which is selected from the following structures: 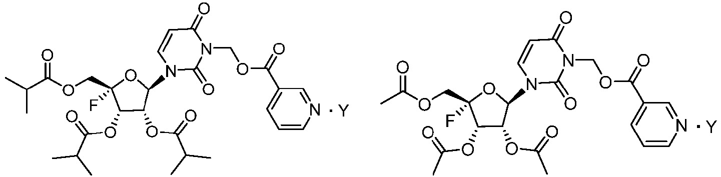 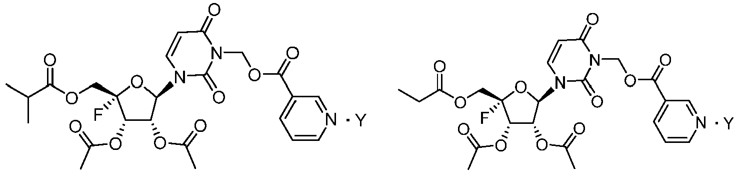 wherein Y are each hydrogen chloride, hydrogen bromide, hydrogen iodide, sulfuric acid, hemisulfuric acid, nitric acid, phosphoric acid, maleic acid, succinic acid, citric acid, tartaric acid, fumaric acid, formic acid, acetic acid, propionic acid, malonic acid, oxalic acid, benzoic acid, phthalic acid, methanesulfonic acid, ethanesulfonic acid, benzenesulfonic acid, toluenesulfonic acid, naphthalenesulfonic acid, 1,5-naphthalenedisulfonic acid, camphoric acid, camphorsulfonic acid, salicylic acid, acetylsalicylic acid, aspartic acid, glutamic acid, lactic acid, gluconic acid, ascorbic acid, gallic acid, mandelic acid, malic acid, sorbic acid, trifluoroacetic acid, taurine, homotaurine, 2-hydroxyethanesulfonic acid, cinnamic acid, or mucic acid, and Y are each preferably hydrogen chloride, hydrogen bromide, sulfuric acid, hemisulfuric acid, or methanesulfonic acid, and more preferably hydrogen chloride or hydrogen bromide.

In some embodiments, the compounds or the pharmaceutically acceptable salts thereof of the present invention described above may exist in the form of crystalline hydrates, solvates, or eutectic compounds, and therefore, these crystalline hydrates, solvates, and eutectic compounds are also included in the scope of the present invention.

In some embodiments, the compounds or the pharmaceutically acceptable salts thereof of the present invention described above may exist in the form of enantiomers, diastereomers, or combinations thereof, and therefore, these enantiomers, diastereomers, and combinations thereof are also included within the scope of the present invention.

In a third aspect, the present invention provides a pharmaceutical composition, which comprises:
a first active ingredient selected from one or more of the compounds, and the pharmaceutically acceptable salts, the solvates, the stereoisomers, the geometric isomers, the isotopically labeled compounds the prodrugs thereof described above;
and a pharmaceutically acceptable carrier.

In some preferred embodiments, the pharmaceutical composition further comprises a second active ingredient selected from one or more of an antiviral active ingredient, a corticosteroid anti-inflammatory drug, an adjuvant therapeutic drug, and the like.

In some embodiments, the antiviral active ingredient is selected from one or more of the following ingredients: interferons, RNA-dependent RNA polymerase inhibitors (such as remdesivir (GS-5734), VV116, favipiravir, galidesivir, GS-441524, NHC (EIDD-1931), EIDD-2801, 5'-isobutyrate of GS-441524, and triisobutyrate of GS-441524), 3CL protease inhibitors (such as GC-376, nirmatrelvir, and VV993), lopinavir, ritonavir, nelfinavir, chloroquine, hydroxychloroquine, cyclosporine, carrimycin, baicalin, baicalein, myricetin, dihydromyricetin, forsythoside, chlorogenic acid, emodin, mycophenolic acid, mycophenolate mofetil, naphthoquine, ciclesonide, ribavirin, penciclovir, leflunomide, teriflunomide, nafamostat, nitazoxanide, darunavir, arbidol, camostat, niclosamide, baricitinib, ruxolitinib, dasatinib, saquinavir, beclabuvir, simeprevir, palivizumab, motavizumab, RSV-IGIV (RespiGam^{®}), MEDI-557, A-60444 (RSV-604), MDT-637, BMS-433771, or pharmaceutically acceptable salts thereof.

In some embodiments, the corticosteroid anti-inflammatory drug is selected from one or more of the following ingredients: dexamethasone, dexamethasone sodium phosphate, fluorometholone, fluorometholone acetate, loteprednol, loteprednol etabonate, hydrocortisone, prednisolone, fludrocortisone, triamcinolone, triamcinolone acetonide, betamethasone, beclomethasone dipropionate, methylprednisolone, fluocinolone, flucinonide, flunisolide, fluocortin-21-butylate, flumethasone, flumethasone pivalate, budesonide, halobetasol propionate, mometasone furoate, fluticasone propionate, ciclesonide, or a pharmaceutically acceptable salt thereof.

In some embodiments, the adjunctive therapeutic drug is selected from one or more of the following ingredients: zinc, fingolimod, vitamin C, olmesartan medoxomil, valsartan, losartan, thalidomide, glycyrrhizic acid, artemisinin, dihydroartemisinin, artesunate, artemisone, azithromycin, escin, naproxen, or a pharmaceutically acceptable salt thereof.

In some embodiments, the pharmaceutical composition is a formulation.

In some preferred embodiments, the formulation is an oral formulation or a non-oral formulation.

In some more preferred embodiments, the formulation is a powder, a granule, a capsule, an injection, an inhalant, a tincture, an oral liquid, a tablet, a troche, or a dropping pill.

In a fourth aspect, the present invention provides use of the nucleoside analogue, or the pharmaceutically acceptable salt, the solvate, the stereoisomer, the geometric isomer, the isotopically labeled compound or the prodrug thereof, or the pharmaceutical composition thereof described above in resisting viruses, such as a paramyxovirus (a parainfluenza virus, a measles virus, a respiratory syncytial virus, a Nipah virus, etc.), an influenza virus, a bunya virus, an arenavirus, viruses of the family Flaviviridae (a hepatitis C virus, a Dengue virus, a Zika virus, etc.), a filovirus (an Ebola virus or a Marburg virus), and/or a coronavirus (SARS, MERS, SARS-CoV-2, a porcine epidemic diarrhea virus, a feline infectious peritonitis virus, etc.), especially in resisting a paramyxovirus, an influenza virus, and a bunya virus.

Specifically, the present invention provides use of the compound, or the pharmaceutically acceptable salt, the solvate, the stereoisomer, the geometric isomer, the isotopically labeled compound or the prodrug thereof, or the pharmaceutical composition thereof described above in the preparation of a medicament, which is (1) an inhibitor for inhibiting viral replication; and/or (2) a medicament for preventing, alleviating, and/or treating a disease caused by a viral infection.

Specifically, the present invention provides a composition for (1) inhibiting viral replication; and/or (2) a method for preventing, alleviating, and/or treating a disease caused by a viral infection, which comprises administering to a human or an animal an effective amount of the compound, or the pharmaceutically acceptable salt, the solvate, the stereoisomer, the geometric isomer, the pharmaceutically acceptable salt or the prodrug thereof described above, or the pharmaceutical composition described above.

Specifically, the present invention provides the compound, or the pharmaceutically acceptable salt, the solvate, the stereoisomer, the geometric isomer, the isotopically labeled compound or the prodrug thereof described above, or the pharmaceutical composition described above, (1) for use as an inhibitor for inhibiting viral replication; and/or (2) for preventing, ameliorating, and/or treating a disease caused by a viral infection.

In some embodiments, the virus is selected from one or more of the following viruses:
(1) a paramyxovirus, preferably a parainfluenza virus, a measles virus, a respiratory syncytial virus (RSV), or a Nipah virus;
(2) an influenza virus, preferably an influenza A virus, an influenza B virus, an influenza C virus, or an influenza D virus;
(3) a virus of the family Bunyaviridae, preferably a virus of the genus *Bunyavirus,* the genus *Phlebovirus,* the genus *Nairovirus,* or the genus *Hantavirus*;
(4) an arenavirus, preferably a Lassa fever virus (LASV), a Junin virus (JUNV), or a Machupo virus (MACV);
(5) a virus of the family Flaviviridae, preferably a hepatitis C virus (HCV), a Dengue virus (DENV), or a Zika virus (Zika);
(6) a filovirus, preferably a Marburg virus (MBV), an Ebola virus (EBV), or a Cuevavirus; and
(7) a coronavirus, preferably a human-infected coronavirus or an animal-infected coronavirus; more preferably, the human-infected coronavirus is severe acute respiratory syndrome coronavirus (SARS-CoV), 2019 novel coronavirus (2019-nCoV or SARS-CoV-2), Middle East respiratory syndrome coronavirus (MERS-CoV), human coronavirus OC43, human coronavirus 229E, human coronavirus NL63, or human coronavirus HKUl, and the animal-infected coronavirus is porcine epidemic diarrhea virus (PEDV) or feline infectious peritonitis virus (FIFV).

In some preferred embodiments, the virus is a paramyxovirus.

In some other preferred embodiments, the virus is an influenza virus.

In some other preferred embodiments, the virus is a bunya virus.

In some preferred embodiments, the disease caused by a viral infection is selected from one or more of the following diseases:
(1) common cold, a high-risk symptom infection, a respiratory tract infection, pneumonia, and complications thereof caused by a respiratory syncytial virus RSV infection;
(2) common cold, a high-risk symptom infection, a respiratory tract infection, pneumonia, and complications thereof caused by an influenza virus infection;
(3) an infection and complications thereof caused by a bunya virus infection;
(4) an infection and complications thereof caused by an arenavirus infection;
(5) chronic hepatitis C and complications thereof caused by a hepatitis C virus infection;
(6) Dengue fever and complications thereof caused by a Dengue virus infection;
(7) an infection and complications thereof caused by a Zika virus infection;
(8) hemorrhagic fever and complications thereof caused by a Marburg virus or Ebola virus infection;
(9) common cold, a high-risk symptom infection, a respiratory tract infection, pneumonia, and complications thereof caused by a human coronavirus infection;
(10) porcine epidemic diarrhea caused by a porcine epidemic diarrhea virus infection; and
(11) feline infectious peritonitis caused by a feline coronavirus infection.

In some preferred embodiments, the disease caused by a viral infection described above is a disease caused by a paramyxovirus infection; preferably, the disease caused by a paramyxovirus infection is selected from one or more of the following diseases: a respiratory tract infection, pneumonia, and complications thereof. More preferably, the paramyxovirus is a respiratory syncytial virus (RSV).

In some preferred embodiments, the disease caused by a viral infection is a disease caused by an influenza virus infection; preferably, the disease caused by an influenza virus infection is selected from one or more of the following diseases: common cold, a high-risk symptom infection, a respiratory tract infection, pneumonia, and complications thereof.

In some preferred embodiments, the disease caused by a viral infection is a disease caused by a bunya virus infection; preferably, the disease caused by a bunya virus infection is selected from one or more of the following diseases: fever with thrombocytopenia syndrome, hemorrhagic fever with renal syndrome, influenza-like diseases, encephalitis, and complications thereof.

It should be understood that within the scope of the present invention, the technical features described above of the present invention and those specifically described below (e.g., in the examples) may be combined with each other to form new or preferred technical solutions. Due to limited space, such solutions are not described herein.

### Effects of the present invention

The nucleoside analogue represented by formula (I) of the present invention, namely the 4'-fluoropyrimidine nucleoside compound, has the advantages of good chemical stability, good pharmacokinetic properties, oral administration, and the like. Experiments show that the compounds of the present invention have good chemical stability, high oral bioavailability, and significant antiviral activity, and are expected to have significant advantages in the treatment of diseases infected by various viruses such as a respiratory syncytial virus, an influenza virus, and a bunya virus.

### BRIEF DESCRIPTION OF THE DRAWINGS

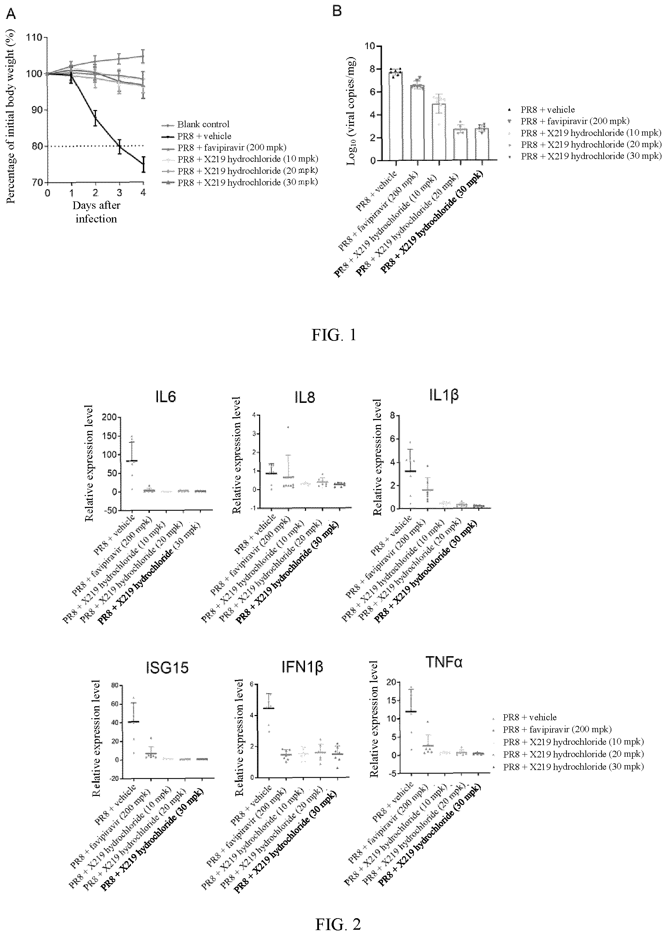

FIGs. 1-2 show the results for evaluation of *in vivo* efficacy of compound X219 hydrochloride in Test Example 10 for an influenza virus in mice. Panel A in FIG. 1 shows the change in body weight over time of Balb/C mice after treatment; panel B in FIG. 1 shows the pulmonary viral load of Balb/C mice on Day 4 after treatment; FIG. 2 shows the expression level of inflammatory factors in the lung tissue of Balb/C mice on Day 4 after treatment.

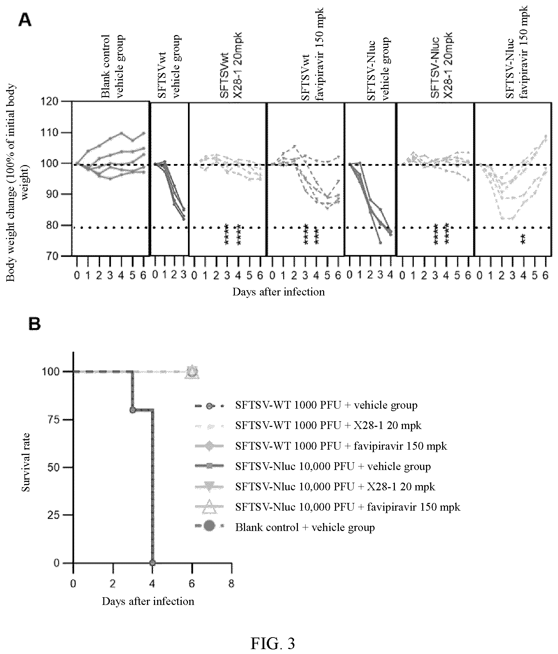

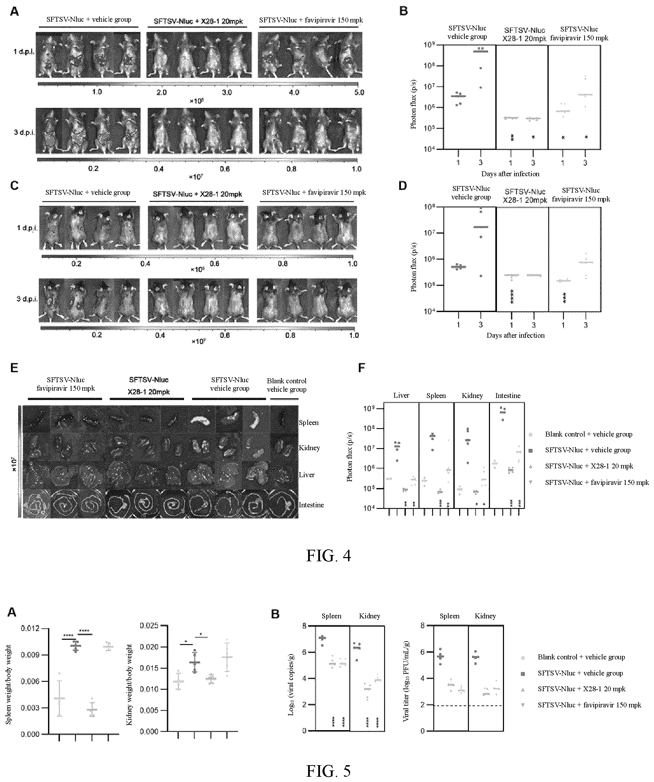

FIGs. 3-5 show the results for evaluation of *in vivo* efficacy of compound X28-1 in Test Example 11 for a severe fever with thrombocytopenia syndrome virus (SFTSV) in mice. Panel A in FIG. 3 shows the change in body weight over time of A129 mice after treatment; panel B in FIG. 3 shows the survival rate curve of A129 mice after treatment; FIG. 4 shows *in vivo* fluorescence imaging and fluorescence intensity for the ventral side (panels A and B in FIG. 4) and dorsal side (panels C and D in FIG. 4) of A129 mice on Day 1 and Day 3 after treatment, and *in vitro* fluorescence imaging and fluorescence intensity for spleen, kidneys, liver, and intestine (panels E and F in FIG. 4) of on A129 mice on Day 3 after treatment; FIG. 5 shows the spleen/body weight ratio and kidney/body weight ratio of A129 mice on Day 3 after treatment (panel A in FIG. 5) and the viral titer and copy number in the spleen and kidneys (panel B in figure 5) of A129 mice.

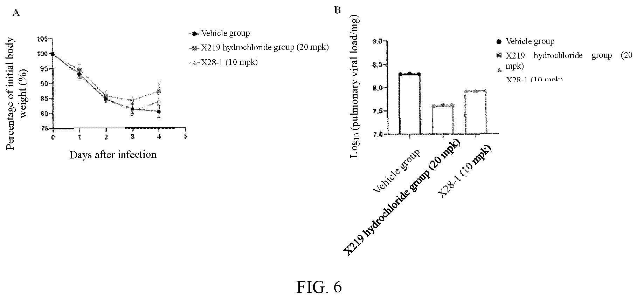

FIG. 6 shows the results for evaluation of *in vivo* efficacy of compound X219 hydrochloride in Test Example 12 for a respiratory syncytial virus (RSV) in mice. Panel A in FIG. 6 shows the change in body weight over time of Balb/C mice after treatment; panel B in FIG. 6 shows the pulmonary viral load of Balb/C mice on Day 4 after treatment.

### DETAILED DESCRIPTION

Through extensive and intensive studies, as a result of extensive screening, the inventors have unexpectedly developed a nucleoside analogue represented by formula (I), namely a 4'-fluoropyrimidine nucleoside compound, which has good chemical stability and pharmacological properties. Experiments show that the 4'-fluoropyrimidine nucleoside compound has good chemical stability, high oral bioavailability, and significant antiviral activity, is expected to have significant advantages in treatment of infections by various viruses such as a respiratory syncytial virus, an influenza virus, and a bunya virus. The present invention is completed on this basis.

Specifically, the present invention provides use of a nucleoside analogue represented by formula (I), or a pharmaceutically acceptable salt, a solvate, a stereoisomer, a geometric isomer, an isotopically labeled compound or a prodrug thereof, or a pharmaceutical composition comprising the same in resisting viruses, preparing an inhibitor for inhibiting viral replication, and/or preparing a medicament for preventing, alleviating, and/or treating a disease, disorder, and/or symptom caused by a viral infection. The viruses are a paramyxovirus (a parainfluenza virus, a measles virus, a respiratory syncytial virus, a Nipah virus, etc.), an influenza virus, a bunya virus, an arenavirus, viruses of the family Flaviviridae (hepatitis C virus, Dengue virus, Zika virus, etc.), a filovirus (an Ebola virus or a Marburg virus), and/or a coronavirus (SARS, MERS, SARS-CoV-2, a porcine epidemic diarrhea virus, a feline infectious peritonitis virus, etc.), especially a paramyxovirus, an influenza virus, and a bunya virus.

The nucleoside analogue represented by formula (I) has good chemical stability and high oral bioavailability, has a relatively strong inhibitory effect on the replication of viruses such as an influenza virus, a respiratory syncytial virus, and a bunya virus, and has good clinical application prospects.

### Definitions of Terms

As used herein, "the compound of the present invention", "the nucleoside analogue of the present invention", "the nucleoside compound of the present invention", "the nucleoside prodrug of the present invention", "the active compound of the present invention", "the antiviral nucleoside analogue of the present invention", and "the nucleoside analogue represented by formula (I)" are used interchangeably and refer to a nucleoside analogue having an excellent antiviral effect *in vivo* or *in vitro,* including a compound represented by formula (I), or a pharmaceutically acceptable salt thereof, or a solvate thereof, or a stereoisomer thereof, or a geometric isomer thereof, or an isotopically labeled compound, or a prodrug thereof, or a combination thereof.

As used herein, "the formulation of the present invention" refers to a formulation containing the nucleoside analogue of the present invention.

As used herein, the term "comprise" or variations thereof such as "comprises" or "comprising" are understood to imply the inclusion of a stated element or component but not the exclusion of any other element or component.

As used herein, the terms "2019 novel coronavirus", "2019-nCoV", or "SARS-CoV-2" are used interchangeably. The 2019 novel coronavirus is the 7th coronavirus known to infect humans and cause novel coronavirus pneumonia (COVID-19), and is one of the serious infectious diseases threatening global human health.

As used herein, "halogen" generally refers to fluorine, chlorine, bromine, and iodine; preferably fluorine, chlorine, or bromine; more preferably fluorine or chlorine.

As used herein, the terms "Cₙ-Cₘ" and "Cₙ₋ₘ" are used interchangeably and refer to a group having n to m carbon atoms, wherein n denotes 1-20, and m denotes 1-20.

As used herein, the term "C₁₋₂₀ alkyl", alone or as part of a complex group, refers to linear or branched saturated hydrocarbyl containing 1-20 carbon atoms, e.g., C₁₋₆ alkyl, e.g., methyl, ethyl, *n*-propyl, isopropyl, *n*-butyl, isobutyl, *tert*-butyl, *sec*-butyl, *n*-pentyl, 1-ethylpropyl, isopentyl, neopentyl, isohexyl, 3-methylpentyl, *n*-hexyl, or the like, preferably methyl, ethyl, *n*-propyl, isopropyl, butyl, isobutyl, or *tert*-butyl.

As used herein, the term "C₁₋₂₀ alkoxy", alone or as part of a complex group, refers to linear or branched alkoxy containing 1-20 carbon atoms, e.g., methoxy, ethoxy, *n*-propoxy, isopropoxy, *n*-butoxy, isobutoxy, *tert*-butoxy, *sec*-butoxy, *n*-pentoxy, isopentoxy, neopentoxy, isohexoxy, pent-3-yloxy, 3-methylpentoxy, *n*-hexoxy, or the like, preferably methoxy, ethoxy, *n*-propoxy, isopropoxy, *n*-butoxy, isobutoxy, pent-3-yloxy, or *tert*-butoxy.

As used herein, the term "C₃₋₂₀ cycloalkyl", alone or as part of a complex group, refers to cyclic saturated hydrocarbyl containing 1-20 carbon atoms, e.g., C₃₋₈ cycloalkyl, e.g., cyclopropyl, cyclobutyl, cyclopentyl, cyclohexyl, cycloheptyl, cyclooctyl, and the like.

As used herein, the term "3- to 20-membered heterocycloalkyl", alone or as part of a complex group, refers to monocyclic or polycyclic (e.g., bicyclic or tricyclic) saturated hydrocarbyl having 3-20 atoms in the ring and including 1-4 ring heteroatoms selected from nitrogen, oxygen, and sulfur, e.g., 3- to 8-membered heterocycloalkyl, e.g., azetidin-3-yl, piperidin-4-yl, tetrahydro-2*H*-pyran-4-yl, and the like.

As used herein, the term "C₂₋₂₀ alkenyl", alone or as part of a complex group, refers to linear or branched unsaturated hydrocarbyl containing 1-3 double bonds and 2-20 carbon atoms, including both cis and trans configurations, e.g., vinyl, 1-propenyl, 2-propenyl, 1-methyl-1-propenyl, 2-methyl-1-propenyl, 2-methyl-2-propenyl, 1-butenyl, 2-butenyl, 3-butenyl, 1-pentenyl, 2-pentenyl, 3-pentenyl, 4-pentenyl, 1,3-butadienyl, 1,3-pentadienyl, 1-hexenyl, 2-hexenyl, 3-hexenyl, 4-hexenyl, 5-hexenyl, 3-methyl-1-butenyl, 2-methyl-1-butenyl, or the like.

As used herein, the term "C₂₋₂₀ alkynyl", alone or as part of a complex group, refers to linear or branched unsaturated hydrocarbyl containing 1-3 triple bonds and 2-20 carbon atoms, e.g., ethynyl, 2-propynyl, 2-butynyl, 3-butynyl, 1-methyl-2-propynyl, 2-pentynyl, 2-hexynyl, or the like.

As used herein, the term "C₁₋₂₀ alkanoyl", alone or as part of a complex group, refers to RC(=O)-, wherein R is selected from hydrogen and C₁₋₁₉ alkyl. Examples of C₁₋₂₀ alkanoyl include formyl, acetyl, propionyl, butyryl, isobutyryl, valeryl, *tert*-butyryl, hexanoyl, or the like. Sometimes alkanoyl may also be referred to as alkylcarbonyl, e.g., C₁₋₆ alkylcarbonyl.

As used herein, the term "C₃₋₂₀ cycloalkanoyl", alone or as part of a complex group, refers to RC(=O)-, wherein R is selected from C₃₋₁₉ cycloalkyl. Examples of C₃₋₂₀ cycloalkanoyl include cyclopropylformyl, cyclobutylformyl, cyclopentylformyl, cyclohexylformyl, cycloheptylformyl, cyclooctylformyl, and the like. Sometimes cycloalkanoyl may also be referred to as cycloalkylcarbonyl, e.g., C₃₋₆ cycloalkylcarbonyl.

As used herein, the term "3- to 20-membered heterocycloalkanoyl", alone or as part of a complex group, refers to RC(=O)-, wherein R is selected from 3- to 19-membered heterocycloalkyl. Examples of 3- to 20-membered heterocycloalkanoyl include azetidine-3-formyl, piperidine-4-formyl, tetrahydro-2*H*-pyran-4-formyl, and the like. Sometimes heterocycloalkanoyl may also be referred to as heterocycloalkylcarbonyl, e.g., 1- to 6-membered heterocycloalkylcarbonyl.

As used herein, the term "C₂₋₂₀ alkenylacyl", alone or as part of a complex group, refers to RC(=O)-, wherein R is selected from C₂₋₁₉ alkenyl. Examples of C₂₋₂₀ alkenylacyl include acryloyl, methacryloyl, 3-methyl-but-2-enoyl, and (*Z*)-9-octadecenoyl.

As used herein, the term "C₂₋₂₀ alkynylacyl", alone or as part of a complex group, refers to RC(=O)-, wherein R is selected from C₂₋₁₉ alkynyl. Examples of C₂₋₂₀ alkynylacyl include propinylacyl, 2-propinylacyl, 2-butynylacyl, 3-butynylacyl, 1-methyl-2-propinylacyl, 2-pentynylacyl, 2-hexynylacyl, and the like.

As used herein, the term "amino C₁₋₂₀ alkanoyl", alone or as part of a complex group, refers to C₁₋₂₀ alkanoyl with one hydrogen substituted with amino (-NH₂), e.g., -CONH₂, -COCH₂NH₂, -COCH₂CH₂NH₂, and the like.

As used herein, the term "C₁₋₂₀ alkylamino", alone or as part of a complex group, refers to amino (-NH₂) with one hydrogen substituted with C₁₋₂₀ alkyl, e.g., -NHCH₃, -NHCH₂CH₃, -NHCH₂CH₂CH₃, and the like.

As used herein, the term "C₁₋₂₀ alkylamino C₁₋₂₀ alkanoyl", alone or as part of a complex group, refers to C₁₋₂₀ alkanoyl with one hydrogen substituted with C₁₋₂₀ alkylamino, e.g., -CONHCH₃, -COCH₂NHCH₃, -COCH₂CH₂NHCH₂CH₃, and the like.

As used herein, the term "C₁₋₂₀ dialkylamino", alone or as part of a complex group, refers to amino (-NH₂) with two hydrogens each being independently substituted with C₁₋₂₀ alkyl, e.g., dimethylamino, diethylamino, methylethylamino, and the like. Sometimes C₁₋₂₀ dialkylamino may also be referred to as di(C₁₋₂₀ alkyl)amino, e.g., di(C₁₋₄ alkyl)amino.

As used herein, the term "C₁₋₂₀ dialkylamino C₁₋₂₀ alkanoyl", alone or as part of a complex group, refers to C₁₋₂₀ alkanoyl with one hydrogen substituted with C₁₋₂₀ dialkylamino, e.g., -CON(CH₃)₂, -CON(CH₂CH₃)₂, -COCH₂N(CH₂CH₃)₂, -COCH₂CH₂N(CH₂CH₃)₂, and the like. As used herein, the term "C₃₋₂₀ cycloalkylamino", alone or as part of a complex group, refers to amino (-NH₂) with one hydrogen substituted with C₃₋₂₀ cycloalkyl, e.g., cyclopropylamino, cyclobutylamino, cyclopentylamino, cyclohexylamino, and the like.

As used herein, the term "C₃₋₂₀ cycloalkylamino C₁₋₂₀ alkanoyl", alone or as part of a complex group, refers to C₁₋₂₀ alkanoyl with one hydrogen substituted with C₃₋₂₀ cycloalkylamino, e.g., cyclopropylamino C₁₋₆ alkanoyl, cyclobutylamino C₁₋₆ alkanoyl, cyclopentylamino C₁₋₆ alkanoyl, cyclohexylamino C₁₋₆ alkanoyl, and the like.

As used herein, the term "C₁₋₂₀ alkoxy C₁₋₂₀ alkanoyl", alone or as part of a complex group, refers to C₁₋₂₀ alkanoyl with one hydrogen substituted with C₁₋₂₀ alkoxy, e.g., -COCH₂OCH₃, -COCH₂CH₂OCH₂CH₃, and the like.

As used herein, the term "C₃₋₂₀ cycloalkoxy", alone or as part of a complex group, refers to RO-, wherein R is selected from C₃₋₂₀ cycloalkyl. Examples of C₃₋₂₀ cycloalkoxy include cyclopropoxy, cyclobutoxy, cyclopentyloxy, cyclohexyloxy, cycloheptyloxy, cyclooctyloxy, and the like. Sometimes cycloalkoxy may also be referred to as cycloalkyloxy, e.g., C₁₋₆ cycloalkyloxy.

As used herein, the term "C₃₋₂₀ cycloalkoxy C₁₋₂₀ alkanoyl", alone or as part of a complex group, refers to C₁₋₂₀ alkanoyl with one hydrogen substituted with C₃₋₂₀ cycloalkoxy, e.g., cyclopropyloxyformyl, cyclobutyloxyformyl, cyclopentyloxyformyl, cyclohexyloxyformyl, cyclohexyloxyacetyl, and the like.

As used herein, the term "amino acid" refers to naturally occurring and synthetic amino acids, in the present application α-amino acids, e.g., L-valine, L-alanine, L-phenylalanine, L-phenylglycine, D-valine, and D-alanine, which can be attached to the parent nucleus structure through the formation of an ester bond between the carbonyl of the amino acid and the oxygen attached to R₂ or R₃.

As used herein, the term "group after removal of carboxyl from an amino acid" refers to a group of remaining after removal of carboxyl connected to the same carbon atom as amino from an amino acid, e.g., a group remaining after removal of carboxyl from L-valine 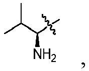 a group remaining after removal of carboxyl from L-alanine 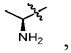 a group remaining after removal of carboxyl from L-phenylglycine 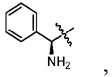 and the like.

As used herein, the term "group after removal of hydroxyl in carboxyl from an amino acid" refers to a group remaining after removal of hydroxyl in carboxyl connected to the same carbon atom as amino from an amino acid, e.g., a group remaining after removal of hydroxyl in carboxyl from L-valine 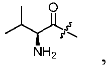 a group remaining after removal of hydroxyl in carboxyl from L-alanine 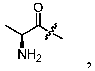 a group remaining after removal of hydroxyl in carboxyl from L-phenylglycine 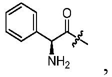 and the like.

As used herein, the term "C₆₋₂₀ aryl", alone or as part of a complex group, refers to a monocyclic or polycyclic (e.g., bicyclic or tricyclic) aromatic ring having 6-20 carbon atoms in the ring and containing no heteroatoms, e.g., C₆₋₁₂ aryl, e.g., C₆₋₁₀ aryl, e.g., phenyl, naphthyl, phenanthryl, anthracyl, and the like.

As used herein, the term "C₆₋₂₀ aroyl", alone or as part of a complex group, may also be referred to as C₆₋₂₀ arylformyl, e.g., C₆₋₁₂ arylformyl, e.g., C₆₋₁₀ arylformyl, e.g., benzoyl, naphthoyl, phenanthroyl, anthracoyl, and the like.

As used herein, the term "5- to 15-membered heteroaryl", alone or as part of a complex group, refers to a monocyclic or polycyclic (e.g., bicyclic or tricyclic) aromatic ring having 5-15 atoms in the ring and containing 1-4 ring heteroatoms selected from nitrogen, oxygen, and sulfur, e.g., 5- to 10-membered heteroaryl or 5- to 6-membered heteroaryl, e.g., pyrrolyl, furanyl, thienyl, imidazolyl, pyrazolyl, oxazolyl, pyridin-2-yl, pyridin-3-yl, pyridin-4-yl, pyrimidin-4-yl, pyrimidin-5-yl, pyrazin-2-yl, isoxazolyl, thiazolyl, isothiazolyl, triazolyl, oxadiazolyl, thiadiazolyl, pyridinyl, pyridazinyl, pyrimidinyl, pyrazinyl, indolyl, isoindolyl, indazolyl, benzotriazolyl, benzothienyl, isobenzothienyl, benzofuranyl, benzisofuranyl, benzimidazolyl, benzoxazolyl, benzisoxazolyl, benzothiazolyl, benzisothiazolyl, benzothiadiazolyl, indolizinyl, quinolinyl, isoquinolinyl, purinyl, imidazopyridinyl, imidazopyrimidinyl, imidazopyrazinyl, imidazopyridazinyl, imidazotriazinyl, pyrazolopyridinyl, pyrazolopyrimidinyl, pyrazolopyrazinyl, pyrazolotriazinyl, pyrrolopyridinyl, pyrrolopyrimidinyl, pyrrolopyridazinyl, pyrrolopyrazinyl, pyrrolotriazinyl, and the like.

As used herein, the term "5- to 15-membered heteroaroyl", alone or as part of a complex group, may also be referred to as "5- to 15-membered heteroarylformyl", which refers to a monocyclic or polycyclic (e.g., bicyclic or tricyclic) aromatic cycloformyl group having 5-15 atoms in the ring and containing 1-4 ring heteroatoms selected from nitrogen, oxygen, and sulfur, e.g., 5- to 10-membered heteroarylformyl or 5- to 6-membered heteroarylformyl, e.g., pyrrolylformyl, furanylformyl, thiophenylformyl, imidazolylformyl, pyrazolylformyl, oxazolylformyl, isoxazolylformyl, thiazolylformyl, isothiazolylformyl, triazolylformyl, oxadiazoylformyl, thiadiazolylformyl, pyridinylformyl, pyridazinylformyl, pyrimidinylformyl, pyrazinylformyl, indolylformyl, isoindolylformyl, indazolylformyl, benzotriazolylformyl, benzothiophenylformyl, isobenzothiophenylformyl, benzofuranylformyl, benzisofuranylformyl, benzimidazolylformyl, benzoxazolylformyl, benzisoxazoylformyl, benzothiazolylformyl, benzisothiazolylformyl, benzothiadiazolylformyl, indolizinylformyl, quinolinylformyl, isoquinolylformyl, purinylformyl, imidazopyridinylformyl, imidazopyrimidinylformyl, imidazopyrazinylformyl, imidazopyridazinylformyl, imidazotriazinylformyl, pyrazolopyridinylformyl, pyrazolopyrimidinylformyl, pyrazolopyrazinylformyl, pyrazolotriazinylformyl, pyrrolopyridinylformyl, pyrrolopyrimidinylformyl, pyrrolopyridazinylformyl, pyrrolopyrazinylformyl, pyrrolotriazinylformyl, and the like.

All features or conditions defined in the form of numerical ranges herein are for brevity and convenience only. Accordingly, the description of numerical ranges should be considered to have covered and specifically disclosed all possible subranges and individual values within the ranges, particularly integer values. For example, the description of a range of "1-6" should be considered to have specifically disclosed all subranges such as 1 to 6, 2 to 6, 2 to 5, 3 to 5, 4 to 6, and 3 to 6, particularly subranges defined by all integer values, and to have specifically disclosed individual values within the ranges such as 1, 2, 3, 4, 5, and 6. Unless otherwise stated, the foregoing methods of interpretation apply to all contents throughout the present invention, whether broad or not.

If a quantity or other value or parameter is expressed as a range, a preferred range, or a list of upper and lower limits, it should be understood that all ranges consisting of any pair of the upper limit or the preferred value of the range and the lower limit or the preferred value of the range have been specifically disclosed herein, whether or not these ranges are separately disclosed. Further, when a numerical range is recited herein, unless otherwise stated, the range is intended to include endpoints thereof, and all integers and fractions within the range.

### Respiratory Syncytial Virus

Respiratory syncytial virus (RSV, abbreviated as syncytial virus, belonging to the family Paramyxoviridae) is the most common pathogen causing viral pneumonia in children and can cause interstitial pneumonia.

RSV is similar to parainfluenza virus, with viral particle size of about 150 nm, slightly smaller than parainfluenza virus, and is an RNA virus.

### Nipah Virus

Nipah virus is a zoonotic RNA virus belonging to the family Paramyxoviridae, which can cause acute respiratory tract infections, fatal encephalitis, and the like, causing serious harm to humans and animals.

### Influenza Virus

Flu virus is referred to as influenza virus. Common influenza viruses are classified into types A, B, C, and D. Influenza viruses can cause infections and diseases in a wide range of animals such as human, poultry, pigs, horses, and bats, and are pathogens of epidemic diseases of human and animals such as human influenza, avian influenza, swine influenza, and horse influenza.

Clinical symptoms caused by influenza viruses include acute hyperthermia, generalized pain, significant fatigue, and respiratory symptoms. Human influenza is mainly caused by influenza A and influenza B viruses. Influenza A virus often has antigenic variations, and can be further classified into subtypes such as H1N1, H3N2, H5N1, and H7N9.

### Bunya Virus

Bunya viruses are a large class of enveloped, segmented, and negative-stranded RNA viruses, which belong to the group of arboviruses. The family Bunyaviridae includes multiple genera (the genus *Bunyavirus,* the genus *Phlebovirus,* the genus *Norovirus,* the genus *Hantavirus,* etc.), which can cause a variety of natural focus infectious diseases, such as hemorrhagic fever with renal syndrome, hantavirus pulmonary syndrome, and sandfly fever.

### Arenavirus

Arenaviruses are a class of enveloped, single-stranded, and negative-stranded RNA viruses shaped like grains of sand and are a small branch of viruses. At present, a plurality of viruses are found to be pathogenic to humans, such as Lassa fever virus (LASV), Junin virus (JUNV), and Machupo virus (MACV), and the three viruses can cause diseases such as hemorrhagic fever, posing a serious threat to human health.

### Viruses of the Family Flaviviridae

Viruses of the family Flaviviridae are a major class of RNA viruses that infect mammals and include 3 genera of viruses, namely the genus *Flavivirus,* the genus *Pestivirus,* and the genus *Hepacivirus.* Dengue virus (DENV) and Zika virus (ZIKV) belong to the genus *Flavivirus* and are transmitted by mosquito vectors. Dengue virus infection can cause significant fever and pain symptoms, and severe dengue fever symptoms include headache, nausea, vomiting, confusion, and even shock. The symptoms of Zika virus (ZIKV) infection are similar to those of dengue fever, but are generally mild. Hepatitis C virus (HCV) belongs to the genus *Hepacivirus,* is the pathogen of chronic hepatitis C, and can cause cirrhosis and liver cancer.

### Viruses of the Family Filoviridae

The family Filovirus currently contains three genera, namely the genus *Ebolavirus,* the genus *Marburgvirus,* and the genus *Cuevavirus.* Both Marburg virus and Ebola virus can cause severe hemorrhagic fever, which can lead to high fever and hemorrhage symptoms in people who are infected, and further shock, organ failure, and death of patients.

### Porcine Epidemic Diarrhea Virus (PEDV)

Porcine epidemic diarrhea virus (PEDV) belongs to the genus *Coronavirus* of the family Coronaviridae. Porcine epidemic diarrhea is an acute intestinal infectious disease of piglets and fattening pigs caused by PEDV virus.

PEDV virus enters the small intestine directly after oral and nasal infections. Replication of PEDV virus can occur in the villous epithelial cytoplasm of the small intestine and colon. PEDV can cause diarrhea, which is osmotic diarrhea. Severe diarrhea causes dehydration, which is the leading cause of death in sick pigs.

### Coronavirus

Coronavirus (CoV) belongs to the family Coronaviridae of the order Nidovirales, and is an enveloped positive-stranded RNA virus, the subfamily of which includes four genera of α, β, δ, and γ.

Among the coronaviruses currently known to infect humans, HCoV-229E and HCoV-NL63 belong to the genus *Alphacoronavirus,* and HCoV-OC43, SARS-CoV, HCoV-HKU1, MERS-CoV, and SARS-CoV-2 are all the genus *Betacoronavirus.*

The genome of the viruses is a single-stranded positive-stranded RNA, which is one of the largest RNA viruses of the genome, and encodes replicase, a spike protein, an envelope protein, a nucleocapsid protein, and the like. In the initial stage of viral replication, the genome is translated into two peptide chains of up to several thousand amino acids, the precursor polyprotein, which is subsequently cleaved by proteases to generate nonstructural proteins (e.g., RNA polymerase and helicase) and structural proteins (e.g., spike protein) and accessory proteins.

Unless otherwise specified, the materials, reagents, and the like adopted in the present application are all available in the market and are conventional in the field, and the adopted equipment and methods are all equipment and method that are conventional in the field.

### Preparation Examples

In the following examples, compound A-1 was prepared by referring to the protocol described in Chinese Patent CN115215914A, compounds X28-1 and X133-1 were prepared by referring to the protocol described in PCT Patent WO2014100505, compounds B and C were prepared by referring to the protocol described in Chinese Patent CN201980030574.4, and uridine was purchased from Wuhu Nuowei Chemical Technology Co., Ltd.

### Preparation Example 1: Preparation of X37 and X22

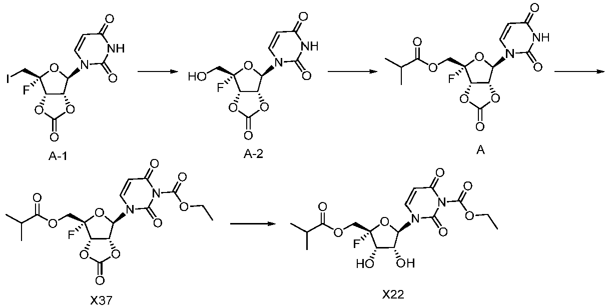

To a 500 mL three-necked flask were added 40% tetrabutylammonium hydroxide (160 g, 247 mmol), trifluoroacetic acid (32 g, 281 mmol), A-1 (20 g, 50.20 mmol), and dichloromethane (150 mL), and the mixture was stirred at room temperature. 85% m-chloroperoxybenzoic acid (50 g, 246 mmol) was added in batches, and the mixture was reacted overnight. After the reaction was completed as detected by TLC, the mixture was filtered, and the filter cake was slurried with 2-butanone (30 mL) and filtered after 1 h. The filter cake was rinsed with 2-butanone and dried at 40 °C for 5 h in vacuum to give intermediate A-2 (11 g, yield: 76%).

To a 500 mL three-necked flask were added A-2 (11 g, 38.20 mmol), 4-dimethylaminopyridine (280 mg, 2.30 mmol), triethylamine (9.7 g, 95.90 mmol), isobutyric anhydride (7.3 g, 46.11 mmol), and ethyl acetate (220 mL), and the mixture was stirred at room temperature for 30 min. After the reaction was completed as detected by TLC, water and ethyl acetate were added, and the mixture was stirred. The organic phase was separated, washed with saturated brine, dried over anhydrous sodium sulfate, and concentrated to give intermediate A (7.6 g, yield: 56%).

A (300 mg, 0.84 mmol) was added to pyridine (3 mL), then *N*,*N*-diisopropylethylamine (433 mg, 3.40 mmol) was added, and ethyl chloroformate (227 mg, 2.11 mmol) was added dropwise in an ice bath. After the dropwise addition, the mixture was heated to room temperature and reacted for 10 min. After the reaction was completed as detected by TLC, an aqueous copper sulfate solution was added to the reaction solution, and the mixture was extracted with ethyl acetate. The organic phase was separated, sequentially washed with water and saturated brine, dried over anhydrous sodium sulfate, and concentrated to give X37 (269 mg), which was directly used in the next step. ¹H NMR (400 MHz, Methanol-*d*₄) δ 7.58 (d, *J* = 8.1 Hz, 1H), 5.88 (d, *J* = 2.1 Hz, 1H), 5.69 (d, *J* = 8.0 Hz, 1H), 4.55 (dd, *J* = 19.2, 6.9 Hz, 1H), 4.43 - 4.35 (m, 2H), 4.29 (dd, *J* = 12.0, 8.2 Hz, 1H), 4.03 - 3.66 (m, 2H), 2.68 - 2.56 (m, 1H), 1.23 - 1.09 (m, 9H). ESI-MS m/z = 431.3 [M+1]⁺.

X37 obtained from the previous step was added to methanol (2 mL), potassium carbonate (17 mg, 0.13 mmol) was added, and the mixture was stirred at room temperature for 5 min. After the reaction was completed as detected by TLC, acetic acid was added to the reaction solution until the pH was about 7, then water and 2-methyltetrahydrofuran were added, and the mixture was stirred. The organic phase was separated, dried over anhydrous sodium sulfate, concentrated, and purified by column chromatography to give compound X22 (96 mg, two-step yield: 28%). ¹H NMR (400 MHz, Chloroform-*d*) δ 7.32 (d, *J* = 8.2 Hz, 1H), 5.81 (d, *J* = 8.3 Hz, 1H), 5.71 (s, 1H), 4.59 - 4.36 (m, 6H), 3.40 - 3.31 (m, 1H), 3.13 - 3.02 (m, 1H), 2.66 - 2.54 (m, 1H), 1.42 (t, *J* = 7.2 Hz, 3H), 1.23 - 1.15 (m, 6H). ¹⁹F NMR (377 MHz, Chloroform-*d*) δ -121.73. ESI-MS m/z = 405.1 [M+1]⁺.

### Preparation Example 2: Synthesis of X28

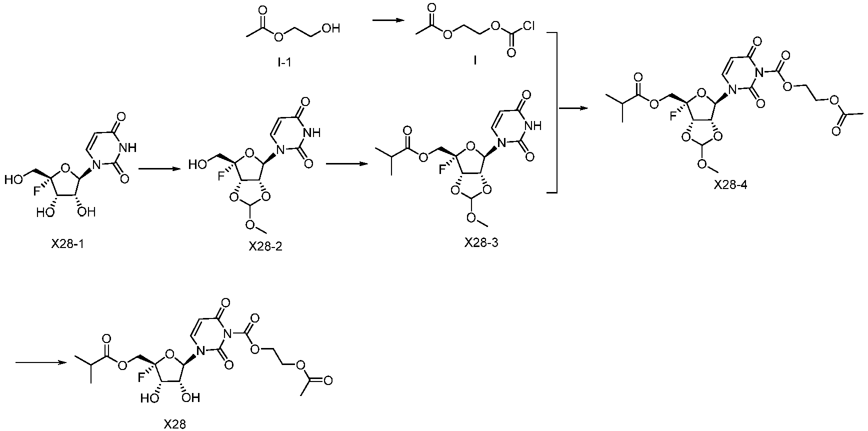

X28-1 (1.59 g, 6.06 mmol) was added to trimethyl orthoformate (15 mL), p-toluenesulfonic acid monohydrate (1.14 g, 6.06 mmol) was added under nitrogen atmosphere, and the mixture was stirred at 23 °C for 1.5 h. After the reaction was completed as detected by TLC, triethylamine (0.61 g, 6.06 mmol) was added dropwise to the reaction solution in an ice bath, the mixture was concentrated by evaporation to remove the solvent, and the residue was purified by column chromatography to give X28-2 (1.51 g, yield: 82%).

X28-2 (150 mg, 0.50 mmol) was dissolved in ethyl acetate (2 mL), then 4-dimethylaminopyridine (4 mg, 0.03 mmol) was added, triethylamine (125 mg, 1.23 mmol) and isobutyric anhydride (94 mg, 0.60 mmol) were sequentially added, and the mixture was reacted at room temperature for 0.5 h. After the reaction was completed as detected by TLC, water was added to the reaction solution, and the mixture was extracted with ethyl acetate. The organic phase was separated, washed with saturated brine, dried over anhydrous sodium sulfate, and concentrated to give intermediate X28-3 (168 mg, yield: 91%).

2-Hydroxyethyl acetate I-1 (1 g, 9.61 mmol) was added to ethyl acetate (10 mL), then a solution of *N*,*N-*dimethylaniline (1.14 g, 9.42 mmol) and triphosgene (1.14 g, 3.81 mmol) in ethyl acetate (10 mL) was added dropwise in an ice bath under nitrogen atmosphere. The mixture was stirred for 2 h in an ice bath, heated to room temperature, and reacted overnight. A 20% aqueous hydrochloric acid solution was added to the reaction solution, and the mixture was extracted with ethyl acetate. The organic phase was separated, washed with saturated brine, dried over anhydrous sodium sulfate, and concentrated to give intermediate I, which was directly used in the next step.

X28-3 (200 mg, 0.54 mmol) was added to pyridine (3 mL), *N*,*N*-diisopropylethylamine (276 mg, 2.11 mmol) was added, and then intermediate I (240 mg, 1.40 mmol) was added in an ice bath. After the addition, the mixture was heated to room temperature and stirred for 10 min. After the reaction was completed as detected by TLC, an aqueous copper sulfate solution was added to the reaction solution, and the mixture was extracted with ethyl acetate. The organic phase was separated, sequentially washed with water and saturated brine, dried over anhydrous sodium sulfate, concentrated and purified by column chromatography to give X28-4 (233 mg), which was directly used in the next step.

The product X28-4 from the previous step was added to dichloromethane (2 mL), and then formic acid (2 mL) was added in an ice bath. After addition, the mixture was heated to room temperature and stirred for 30 min. After the reaction was completed as detected by TLC, a saturated aqueous sodium bicarbonate solution was added to the reaction solution until the pH value was about 7, and the mixture was extracted with ethyl acetate. The organic phase was separated, dried over anhydrous sodium sulfate, concentrated and purified by column chromatography to give X28 (65 mg, two-step yield: 26%). ¹H NMR (400 MHz, Chloroform-*d*) δ 7.32 (d, *J* = 8.2 Hz, 1H), 5.80 (d, *J* = 8.3 Hz, 1H), 5.73 (s, 1H), 4.70 - 4.34 (m, 8H), 3.42 - 3.34 (m, 1H), 3.13 - 3.03 (m, 1H), 2.66 - 2.52 (m, 1H), 2.10 (s, 3H), 1.27 - 1.16 (m, 6H). ¹⁹F NMR (377 MHz, Chloroform-*d*) δ -121.74. ESI-MS m/z = 463.2 [M+1]⁺.

### Preparation Example 3: Preparation of X113 and X51

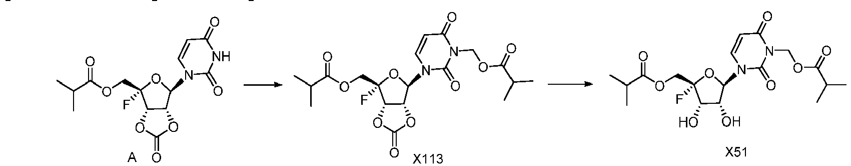

A (200 mg, 0.60 mmol) was added to N,N-dimethylformamide (2 mL), then potassium carbonate (77 mg, 0.60 mmol) was added in an ice bath under nitrogen atmosphere, and the mixture was stirred for 5 min. Chloromethyl isopropyl ester (153 mg, 1.20 mmol) was added, and the mixture was heated to room temperature and stirred overnight. Water and ethyl acetate were added to the reaction solution, and the mixture was stirred. The organic phase was separated, washed with saturated brine, dried over anhydrous sodium sulfate, concentrated, and purified by column chromatography to give X113 (205 mg). ¹H NMR (400 MHz, DMSO-*d*₆) δ 7.91 (d, *J* = 8.1 Hz, 1H), 6.42 (s, 1H), 5.93 (d, *J* = 8.1 Hz, 1H), 5.81 - 5.63 (m, 4H), 4.44 - 4.36 (m, 2H), 2.65 - 2.50 (m, 2H), 1.15 - 1.03 (m, 12H). ESI-MS m/z = 481.2 [M+1]⁺.

X113 (205 mg, 0.45 mmol) was added to methanol (2 mL), then potassium carbonate (38 mg, 0.30 mmol) was added, and the mixture was stirred at room temperature for 7 min. After the reaction was completed as detected by TLC, acetic acid was added dropwise to the reaction solution until the pH value was about 7, then water and 2-methyltetrahydrofuran were added, and the mixture was stirred. The organic phase was separated, dried over anhydrous sodium sulfate, concentrated, and purified by column chromatography to give X51 (90 mg, two-step yield: 37%). ¹H NMR (400 MHz, Chloroform-*d*) δ 7.34 (d, *J* = 8.2 Hz, 1H), 5.92 (s, 2H), 5.82 (d, *J* = 8.2 Hz, 1H), 5.76 (d, *J* = 1.5 Hz, 1H), 4.60 - 4.45 (m, 2H), 4.41 (d, *J* = 7.9 Hz, 2H), 3.39 (d, *J* = 4.3 Hz, 1H), 3.10 (dd, *J* = 9.7, 2.1 Hz, 1H), 2.67 - 2.49 (m, 2H), 1.24 - 1.11 (m, 12H). ¹⁹F NMR (377 MHz, Chloroform-*d*) δ -121.74. ESI-MS m/z = 433.2 [M+1]⁺.

### Preparation Example 4: Synthesis of X102

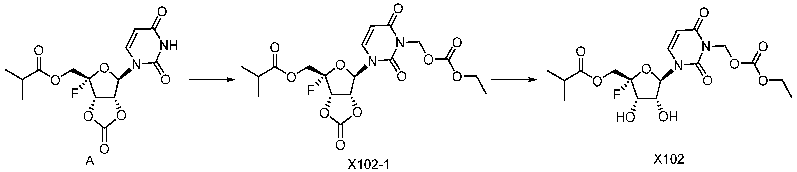

To a 25 mL three-necked flask were added A (200 mg, 0.56 mmol) and *N*,*N*-dimethylformamide (2 mL), then potassium carbonate (77 mg, 0.56 mmol) was added in an ice bath under nitrogen atmosphere, and the mixture was stirred for 5 min. Chloromethyl ethyl carbonate (155 mg, 1.12 mmol) was added, and the mixture was heated to room temperature and stirred overnight. After the reaction was completed as detected by TLC, water and ethyl acetate were added, and the mixture was stirred. The organic phase was separated, dried over anhydrous sodium sulfate, concentrated, and purified by column chromatography to give X102-1 (248 mg, yield: 97%).

To a 25 mL three-necked flask were added X102-1 (200 mg, 0.43 mmol), methanol (2 mL), and potassium carbonate (3 mg, 0.02 mmol), and the mixture was stirred at room temperature for 5 min. After the reaction was completed as detected by TLC, acetic acid was added to the reaction solution until the pH was about 7, then water and ethyl acetate were added, and the mixture was stirred. The organic phase was separated, dried over anhydrous sodium sulfate, concentrated, and purified by column chromatography to give X102 (50 mg, yield: 26%). ¹H NMR (400 MHz, Chloroform-*d*) δ 7.32 (d, *J* = 8.2 Hz, 1H), 5.96 (s, 2H), 5.82 (d, *J* = 8.2 Hz, 1H), 5.73 (d, *J* = 1.4 Hz, 1H), 4.64 - 4.46 (m, 2H), 4.40 (d, *J* = 7.8 Hz, 2H), 4.24 (q, *J* = 7.1 Hz, 2H), 3.30 (d, *J* = 4.2 Hz, 1H), 3.05 (dd, *J* = 9.8, 2.2 Hz, 1H), 2.67 - 2.55 (m, 1H), 1.31 (t, *J* = 7.1 Hz, 3H), 1.23 - 1.17 (m, 6H). ¹⁹F NMR (377 MHz, Chloroform-*d*) δ -121.58. ESI-MS m/z = 435.2 [M+1]⁺.

### Preparation Example 5: Synthesis of X133

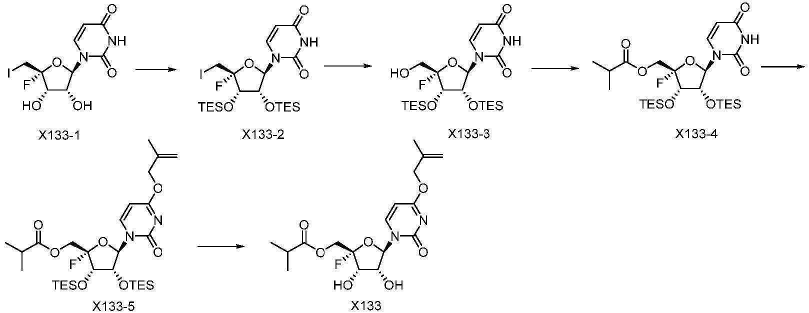

In an ice-water bath, X133-1 (5.00 g, 13.44 mmol) and imidazole (5.50 g, 88.71 mmol) were added to *N*,*N*-dimethylformamide (50 mL), and triethylchlorosilane (8.06 g, 53.73 mmol) was slowly added. The mixture was stirred at room temperature for about 30 min. After the reaction was completed, the reaction solution was concentrated. Water was added, and the mixture was extracted with ethyl acetate. The organic phase was separated, washed with a saturated sodium chloride solution, dried over anhydrous sodium sulfate, concentrated, and purified by silica gel column chromatography to give X133-2 (6.35 g, yield: 79%) as a colorless transparent oil. ¹H NMR (400 MHz, DMSO-*d*₆) δ 11.54 (s, 1H), 7.79 (d, *J* = 8.1 Hz, 1H), 6.05 (d, *J* = 6.2 Hz, 1H), 5.78 (dd, *J* = 8.0, 2.2 Hz, 1H), 4.56 (t, *J* = 5.7 Hz, 1H), 4.43 (t, *J* = 5.5 Hz, 1H), 3.76 - 3.63 (m, 2H), 0.96 (t, *J* = 7.9 Hz, 9H), 0.86 (t, *J* = 7.9 Hz, 9H), 0.66 (q, *J* = 7.7 Hz, 6H), 0.57 - 0.47 (m, 6H).

In an ice-water bath, trifluoroacetic acid was added to a 50% aqueous tetrabutylammonium hydroxide solution (19.72 g, 38 mmol) until the pH was about 4, the solution was added to a solution of X133-2 (4.57 g, 7.61 mmol) in dichloromethane (80 mL), and 85% *m*-chloroperoxybenzoic acid (7.70 g, 38 mmol) was added. The ice bath was removed, the mixture was stirred at room temperature with the pH monitored. After about 1 h of reaction, the pH of the reaction solution was reduced to about 2, and a 1 N aqueous sodium hydroxide solution was added until the pH was about 4. The mixture was stirred for about 12 h. After the reaction was completed, a saturated aqueous sodium thiosulfate pentahydrate solution was added, then a saturated aqueous sodium bicarbonate solution was added, and the mixture was extracted with dichloromethane. The organic phase was separated, dried over anhydrous sodium sulfate, concentrated, and the purified by silica gel column chromatography to give X133-3 (1.67 g, yield: 45%) as a white solid. ¹H NMR (400 MHz, DMSO-*d₆*) δ 11.47 (s, 1H), 7.80 (d, *J =* 8.2 Hz, 1H), 6.04 (d, *J* = 4.7 Hz, 1H), 5.74 (dd, *J* = 8.1, 2.1 Hz, 1H), 5.65 (t, *J* = 5.6 Hz, 1H), 4.38 - 4.29 (m, 2H), 3.63 - 3.51 (m, 2H), 0.95 (t, *J* = 7.9 Hz, 9H), 0.88 (t, *J* = 7.9 Hz, 9H), 0.63 (q, *J* = 7.9 Hz, 6H), 0.58 - 0.48 (m, 6H).

X133-3 (1.01 g, 2.04 mmol), triethylamine (409 mg, 4.05 mmol), and 4-dimethylaminopyridine (124 mg, 1.02 mmol) were sequentially added to dichloromethane (10 mL) at room temperature, and isobutyric anhydride (478 mg, 3.03 mmol) was added. The mixture was stirred for about 30 min. After the reaction was completed, a 1 N diluted hydrochloric acid solution was added to the reaction solution, and the mixture was extracted with dichloromethane. The organic phase was separated, dried over anhydrous sodium sulfate, concentrated, and purified by column chromatography to give X133-4 (894 mg, yield: 78%) as a white foamy solid. ¹H NMR (400 MHz, DMSO-*d*₆) δ 11.55 (s, 1H), 7.78 (d, *J* = 8.1 Hz, 1H), 6.00 (d, *J* = 5.4 Hz, 1H), 5.75 (dd, *J* = 8.1, 2.1 Hz, 1H), 4.55 (t, *J* = 5.4 Hz, 1H), 4.46 - 4.35 (m, 2H), 4.22 - 4.12 (m, 1H), 2.64 - 2.55 (m, 1H), 1.12 (d, *J* = 7.0 Hz, 6H), 0.96 (t, *J* = 7.9 Hz, 9H), 0.88 (t, *J* = 7.9 Hz, 9H), 0.64 (q, *J* = 7.9 Hz, 6H), 0.59 - 0.50 (m, 6H).

In an ice-water bath, X133-4 (305 mg, 0.54 mmol), triethylamine (126 mg, 1.25 mmol), and 4-dimethylaminopyridine (9 mg, 0.07 mmol) were added to dichloromethane (5 mL), then 2,4,6-triisopropylbenzenesulfonyl chloride (326 mg, 1.08 mmol) was slowly added, and the mixture was stirred at room temperature for 4 h. Dichloromethane and a saturated aqueous sodium bicarbonate solution were added, and the mixture was stirred. The organic phase was separated, washed with saturated brine, dried over anhydrous sodium sulfate, and concentrated. The obtained product was dissolved in dichloromethane, 2-methylallyl alcohol (549 mg, 7.63 mmol) was added, then triethylamine (542 mg, 5.37 mmol) was added, and the mixture was stirred for 15 min. 1,8-Diazabicyclo[5.4.0]undec-7-ene (90 mg, 0.59 mmol) was added, and the mixture was stirred for 16 h. After the reaction was completed as detected by TLC, dichloromethane and saturated brine were added to the reaction solution, and the mixture was stirred. The organic phase was separated, dried over anhydrous sodium sulfate, concentrated and purified by silica gel column chromatography to give compound X133-5 (208 mg, yield: 63%) as a white foamy solid. ¹H NMR (500 MHz, DMSO-*d*₆) δ 8.09 (d, *J* = 7.4 Hz, 1H), 6.20 (d, *J* = 7.4 Hz, 1H), 6.02 (d, *J* = 4.4 Hz, 1H), 5.00 (s, 1H), 4.95 (s, 1H), 4.78 - 4.68 (m, 2H), 4.55 (t, *J* = 4.9 Hz, 1H), 4.50 - 4.37 (m, 2H), 4.21 - 4.13 (m, 1H), 2.64 - 2.56 (m, 1H), 1.75 (s, 3H), 1.14 - 1.07 (m, 6H), 0.95 (t, *J* = 7.9 Hz, 9H), 0.86 (t, *J* = 7.9 Hz, 9H), 0.63 (q, *J* = 7.9 Hz, 6H), 0.53 (q, *J* = 7.8 Hz, 6H).

In an ice-water bath, X133-5 (208 mg, 0.34 mmol) was added to tetrahydrofuran (2 mL), then glacial acetic acid (10 mg, 0.17 mmol) and a 1 M tetrabutylammonium fluoride tetrahydrofuran solution (0.34 mL) were added, and the mixture was stirred for 30 min. After the reaction was completed, the reaction solution was concentrated by evaporation to remove the solvent, and the residue was purified by silica gel column chromatography to give compound X133 (124 mg, yield: 94%) as a white solid. ¹H NMR (500 MHz, DMSO-*d*₆) δ 8.00 (d, *J* = 7.4 Hz, 1H), 6.13 (d, *J* = 7.4 Hz, 1H), 5.96 (d, *J* = 2.1 Hz, 1H), 5.77 (d, *J* = 5.1 Hz, 1H), 5.29 (d, *J* = 9.3 Hz, 1H), 5.00 (s, 1H), 4.95 (s, 1H), 4.71 (s, 2H), 4.44 - 4.33 (m, 2H), 4.27 - 4.18 (m, 2H), 2.63 - 2.54 (m, 1H), 1.75 (s, 3H), 1.11 - 1.05 (m, 6H). ESI-MS m/z = 409.3 [M+23]⁺.

### Preparation Example 6: Synthesis of X134

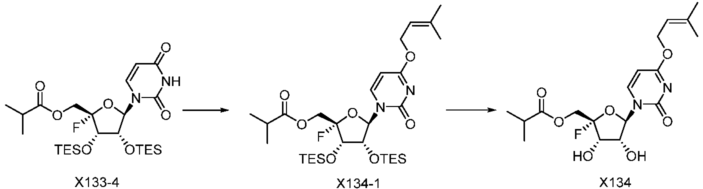

In an ice-water bath, X133-4 (307 mg, 0.55 mmol), triethylamine (129 mg, 1.28 mmol), and 4-dimethylaminopyridine (29 mg, 0.24 mmol) were added to dichloromethane (5 mL), and 2,4,6-triisopropylbenzenesulfonyl chloride (325 mg, 1.08 mmol) was slowly added. The ice-water bath was removed, and the mixture was stirred at room temperature for 4 h. Dichloromethane and a saturated aqueous sodium bicarbonate solution were added, and the mixture was stirred. The organic phase was separated, washed with saturated brine, dried over anhydrous sodium sulfate, and concentrated. The obtained product was dissolved in dichloromethane, then 3-methyl-2-buten-1-ol (660 mg, 7.67 mmol) and triethylamine (566 mg, 5.60 mmol) were added, and the mixture was stirred for 15 min. 1,8-Diazabicyclo[5.4.0]undec-7-ene (89 mg, 0.59 mmol) was added, and the mixture was stirred for 16 h. After the reaction was completed as detected by TLC, dichloromethane and saturated brine were added to the reaction solution, and the mixture was stirred. The organic phase was separated, dried over anhydrous sodium sulfate concentrated, and purified by silica gel column chromatography to give X134-1 (131 mg, yield: 36%) as a white foamy solid. ¹H NMR (500 MHz, DMSO-*d*₆) δ 8.04 (d, *J* = 7.5 Hz, 1H), 6.11 (d, *J* = 7.4 Hz, 1H), 6.00 (d, *J* = 4.3 Hz, 1H), 5.43 - 5.38 (m, 1H), 4.83 - 4.72 (m, 2H), 4.58 - 4.53 (m, 1H), 4.49 - 4.36 (m, 2H), 4.20 - 4.12 (m, 1H), 2.64 - 2.55 (m, 1H), 1.73 (s, 3H), 1.70 (s, 3H), 1.14 -1.08 (m, 6H), 0.95 (t, *J* = 7.9 Hz, 9H), 0.86 (t, *J* = 7.9 Hz, 9H), 0.63 (q, *J* = 7.9 Hz, 6H), 0.53 (q, *J* = 7.7 Hz, 6H).

In an ice-water bath, X134-1 (130 mg, 0.21 mmol) was added to tetrahydrofuran (2 mL), then a drop of glacial acetic acid and a 1 M tetrabutylammonium fluoride tetrahydrofuran solution (0.2 mL) were added, and the mixture was stirred for 30 min. After the reaction was completed, the reaction solution was concentrated by evaporation to remove the solvent, and the residue waspurified by silica gel column chromatography to give compound X134 (54 mg, yield: 64%). ¹H NMR (500 MHz, DMSO-*d*₆) δ 7.96 (d, *J* = 7.4 Hz, 1H), 6.04 (d, *J* = 7.4 Hz, 1H), 5.95 (d, *J* = 2.1 Hz, 1H), 5.45 - 5.38 (m, 1H), 4.82 - 4.73 (m, 2H), 4.44 - 4.32 (m, 2H), 4.28 - 4.17 (m, 2H), 2.63 - 2.54 (m, 1H), 1.74 (s, 3H), 1.70 (s, 3H), 1.10 - 1.07 (m, 6H).

### Preparation Example 7: Synthesis ofX163

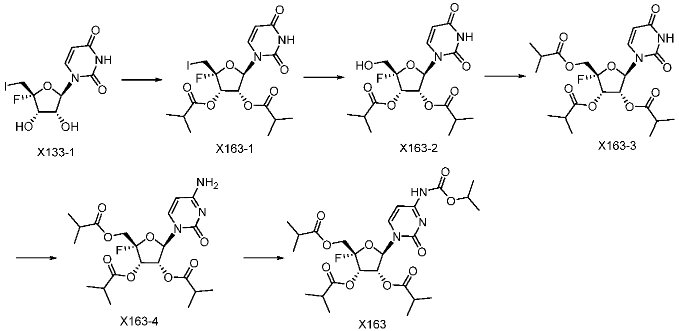

X131-1 (1.50 g, 4.03 mmol) was added to dichloromethane (15 mL), then triethylamine (1.60 g, 16. 12 mmol), 4-dimethylaminopyridine (0.24 g, 2.02 mmol), and isobutyric anhydride (1.30 g, 8.10 mmol) were added, and the mixture was stirred at room temperature for 1 h. After the reaction was completed as detected by TLC, dichloromethane was added to the reaction solution, and the mixture was sequentially washed with 1 M diluted hydrochloric acid, saturated sodium bicarbonate solution, and saturated brine. The organic phase was separated, dried over anhydrous sodium sulfate, concentrated, and purified by column chromatography to give compound X163-1 (1.67 g, yield: 81%) as a white foamy solid. ¹H NMR (500 MHz, DMSO-*d*₆) δ 11.60 (s, 1H), 7.79 (d, *J* = 8.1 Hz, 1H), 6.01 (d, *J* = 2.3 Hz, 1H), 5.79 - 5.71 (m, 2H), 5.58 (dd, *J* = 7.3, 2.3 Hz, 1H), 3.64 - 3.47 (m, 2H), 2.64 - 2.53 (m, 2H), 1.14 - 1.08 (m, 12H).

In an ice bath, trifluoroacetic acid (2.07 g, 18.21 mmol) was added to an aqueous tetrabutylammonium hydroxide solution (8.46 g, 16.30 mmol) to adjust the pH to about 4, then the solution was added to a solution of X163-1 (1.67 g, 3.30 mmol) in dichloromethane (20 mL), and 85% m-chloroperoxybenzoic acid (3.31 g, 16.30 mmol) was added. After the addition, the mixture was heated to room temperature and stirred overnight. After the reaction was completed as detected by TLC, sodium thiosulfate was added to the reaction solution, and the mixture was stirred for 30 min and extracted with ethyl acetate. The organic phase was sequentially washed with saturated sodium bicarbonate solution and saturated brine, dried over anhydrous sodium sulfate, concentrated, and purified by column chromatography to give compound X163-2 (1 g, yield: 77%) as a white foamy solid. ¹H NMR (500 MHz, DMSO-*d*₆) δ 11.54 (s, 1H), 7.79 (d, *J* = 8.1 Hz, 1H), 6.01 (d, *J* = 2.5 Hz, 1H), 5.72 - 5.66 (m, 2H), 5.54 (dd, *J* = 7.1, 2.5 Hz, 1H), 5.50 (t, *J* = 6.3 Hz, 1H), 3.64 - 3.54 (m, 2H), 2.62 - 2.53 (m, 2H), 1.12 - 1.08 (m, 12H).

X163-2 (817.4 mg, 2.03 mmol) was added to dichloromethane (10 mL), then triethylamine (308.2 mg, 3.05 mmol), 4-dimethylaminopyridine (49.6 mg, 0.41 mmol), and isobutyric anhydride (385.9 mg, 2.44 mmol) were sequentially added, and the mixture was stirred at room temperature for 10 min. After the reaction was completed as detected by TLC, dichloromethane was added to the reaction solution, and the mixture was sequentially washed with 1 M diluted hydrochloric acid, saturated sodium bicarbonate solution and saturated brine. The organic phase was separated, dried over anhydrous sodium sulfate and concentrated to give compound X163-3, which was directly used in the next step. ¹H NMR (400 MHz, DMSO-*d*₆) δ 11.58 (d, *J* = 2.1 Hz, 1H), 7.79 (d, *J* = 8.0 Hz, 1H), 6.03 (d, *J* = 2.2 Hz, 1H), 5.82 (dd, *J* = 20.0, 7.2 Hz, 1H), 5.70 (dd, *J* = 8.1, 2.1 Hz, 1H), 5.59 (dd, *J* = 7.3, 2.1 Hz, 1H), 4.45 - 4.21 (m, 2H), 2.67 - 2.52 (m, 3H), 1.20 - 1.00 (m, 18H).

X163-3 (100.0 mg, 0.21 mmol) was added to dichloromethane (1 mL), and triethylamine (42.5 mg, 0.42 mmol), 4-dimethylaminopyridine (51.3 mg, 0.42 mmol), and 2,4,6-triisopropylbenzenesulfonyl chloride (127.2 mg, 0.42 mmol) were sequentially added in an ice bath. The mixture was stirred for 30 min, and 25% ammonia water (856.8 mg, 100.82 mmol) was added. The ice bath was removed, and the mixture was heated to room temperature and stirred overnight. After the reaction was completed as detected by TLC, dichloromethane was added to the reaction solution, and the mixture was washed with saturated brine. The organic phase was separated, dried over anhydrous sodium sulfate, concentrated, and purified by column chromatography to give compound X163-4 (67.6 mg, yield: 68%) as a white solid. ¹H NMR (500 MHz, DMSO-*d*₆) δ 7.68 (d, *J* = 7.4 Hz, 1H), 7.45 (d, *J* = 16.5 Hz, 2H), 5.94 - 5.84 (m, 2H), 5.73 (d, *J* = 7.4 Hz, 1H), 5.56 (dd, *J* = 7.2, 1.9 Hz, 1H), 4.38 - 4.21 (m, 2H), 2.61 - 2.53 (m, 3H), 1.14 - 1.05 (m, 18H).

X163-4 (53.4 mg, 0.11 mmol) was added to dichloromethane (1 mL), and then pyridine (13.1 mg, 0.17 mmol) and isopropyl chloride (16.7 mg, 0.14 mmol) were sequentially added in an ice bath. The mixture was heated to room temperature and stirred for 4 h. After the reaction was completed as detected by TLC, dichloromethane was added to the reaction solution, and the mixture was sequentially washed with diluted hydrochloric acid and saturated brine. The organic phase was separated, dried over anhydrous sodium sulfate, concentrated, and purified by preparative TLC to give compound X163 (32.5 mg, yield: 52%) as a white solid. ¹H NMR (400 MHz, DMSO-*d*₆) δ 10.80 (s, 1H), 8.13 (d, *J* = 7.5 Hz, 1H), 7.08 (d, *J* = 7.5 Hz, 1H), 6.04 (s, 1H), 5.89 (dd, *J* = 20.3, 7.2 Hz, 1H), 5.60 (d, *J* = 7.2 Hz, 1H), 4.95 - 4.87 (m, 1H), 4.42 - 4.25 (m, 2H), 2.63 - 2.52 (m, 3H), 1.26 - 1.23 (m, 6H), 1.14 - 1.06 (m, 18H).

### Preparation Example 8: Synthesis of X181 and X182

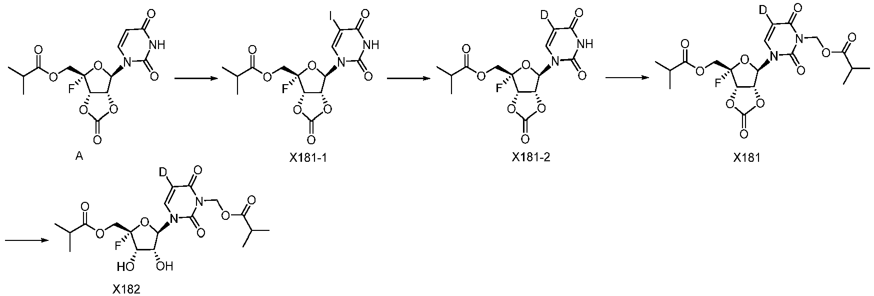

To a three-necked flask were added A (1.5 g, 4.18 mmol) and acetonitrile (15 mL), ceric ammonium nitrate (1.33 g, 2.41 mmol) and iodine (740 mg, 2.92 mmol) were sequentially added under stirring. The mixture was reacted at 90 °C overnight. After the reaction was completed as detected by TLC, the reaction solution was concentrated and the residue was purified by column chromatography to give a crude product, which was slurried with isopropyl ether (6 mL) to give X181-1 (1.2 g, yield: 59%).

To an autoclave were sequentially added tetrahydrofuran (12 mL), X181-1 (600 mg, 1.22 mmol), triethylamine (250 mg, 2.50 mmol), and palladium on carbon (600 mg), and deuterium gas was introduced for pressurization to 1.1 MPa. The mixture was reacted overnight at room temperature. After the reaction was completed as detected by TLC, the reaction solution was filtered, and the filtrate was concentrated to give a crude product of X181-2 (777 mg), which was directly used in the next step.

To a three-necked flask were added the crude product of X181-2 obtained from the previous step, *N*,*N*-dimethylformamide (16 mL) and potassium carbonate (298 mg, 2.20 mmol). Then chloromethyl isobutyrate (442 mg, 3.20 mmol) was added dropwise in an ice bath under nitrogen atmosphere. After the addition, the mixture was heated to room temperature and reacted overnight. After the reaction was completed as detected by TLC, water and ethyl acetate were added to the reaction solution, and the mixture was stirred. The organic phase was separated, dried over anhydrous sodium sulfate, concentrated, and purified by column chromatography to give X181 (265 mg, two-step yield: 48%). ¹H NMR (400 MHz, DMSO-*d*₆) δ 7.91 (s, 1H), 6.42 (s, 1H), 5.81 - 5.63 (m, 4H), 4.40 (d, *J* = 15.4 Hz, 2H), 2.65 - 2.49 (m, 2H), 1.15 - 1.03 (m, 12H). ESI-MS m/z = 504.1 [M+1]⁺.

Referring to the synthetic method for X51, X181 (200 mg, 0.44 mmol) was reacted with potassium carbonate (18 mg, 0.13 mmol) in methanol to give X182 (118 mg, yield: 62%). ¹H NMR (400 MHz, DMSO-*d*₆) δ 7.78 (s, 1H), 5.96 (d, *J* = 2.5 Hz, 1H), 5.82 - 5.73 (m, 3H), 5.37 (d, *J* = 8.8 Hz, 1H), 4.43 - 4.26 (m, 3H), 4.21 (t, *J* = 11.3 Hz, 1H), 2.66 - 2.51 (m, 2H), 1.15 - 0.99 (m, 12H). ESI-MS m/z = 434.2 [M+1]⁺.

### Preparation Example 9: Synthesis of X183 and X184

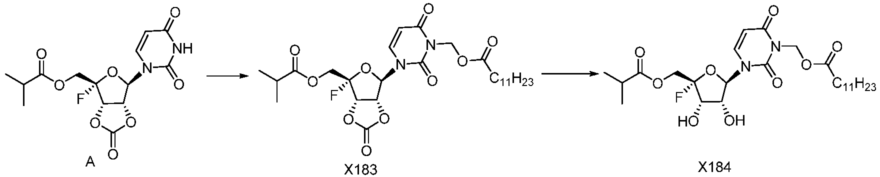

To a 25 mL three-necked flask were sequentially added A (500 mg, 1.40 mmol), *N*,*N*-dimethylformamide (5 mL), potassium carbonate (193 mg, 1.40 mmol), and chloromethyl laurate (697 mg, 2.80 mmol), and the mixture was stirred at 25 °C. After the reaction was completed, water and ethyl acetate were added to the reaction solution, and the mixture was stirred. The organic phase was separated, dried over anhydrous sodium sulfate, concentrated, and purified by column chromatography to give X183 (537 mg, yield: 67%).

To a 25 mL three-necked flask were added X183 (537 mg, 0.94 mmol), methanol (3 mL), and potassium carbonate (13 mg, 0.09 mmol), and the mixture was stirred at room temperature for 15 min. After the reaction was completed as detected by TLC, acetic acid (5.6 mg, 0.09 mmol) was added to the reaction solution, then water and ethyl acetate were added, and the mixture was stirred. The organic phase was separated, dried over anhydrous sodium sulfate, concentrated and purified by column chromatography to give X184 (67 mg, yield: 14%). ¹H NMR (400 MHz, Chloroform-*d*) δ 7.31 (d, *J* = 8.2 Hz, 1H), 5.92 (s, 2H), 5.82 (d, *J* = 8.2 Hz, 1H), 5.73 (s, 1H), 4.62 - 4.46 (m, 2H), 4.41 (d, *J* = 7.8 Hz, 2H), 3.07 - 2.99 (m, 1H), 2.96 - 2.86 (m, 1H), 2.66 - 2.56 (m, 1H), 2.31 (t, *J* = 7.6 Hz, 2H), 1.66 - 1.58 (m, 2H), 1.36 - 1.20 (m, 16H), 1.23 - 1.17 (m, 6H), 0.88 (t, *J* = 6.7 Hz, 3H). ESI-MS m/z = 545.3 [M+1]⁺.

### Preparation Example 10: Synthesis of X185 Hemimaleate and X186 Hydrochloride

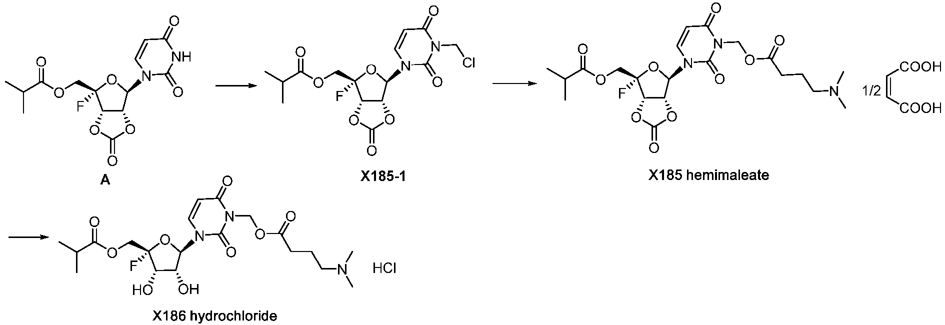

To a 100 mL three-necked flask were added A (4.0 g, 11.20 mmol) and *N*,*N*-dimethylformamide (40 mL), then potassium carbonate (1.5 g, 10.90 mmol) was added in an ice bath under nitrogen atmosphere, and the mixture was stirred for 5 min. Chloroiodomethane (9.8 g, 55.60 mmol) was added, and the mixture was heated to room temperature and stirred overnight. Water and ethyl acetate were added to the reaction solution, and the mixture was stirred. The organic phase was separated, dried over anhydrous sodium sulfate, concentrated, and purified by column chromatography to give X185-1 (1.0 g, yield: 22%).

To a 25 mL three-necked flask were added X185-1 (300 mg, 0.74 mmol), *N*,*N*-dimethylformamide (4 mL), potassium carbonate (306 mg, 2.21 mmol), 4-dimethylaminobutyric acid hydrochloride (248 mg, 1.48 mmol), and sodium iodide (11 mg, 0.07 mmol)The mixture was then stirred at room temperature overnight. After the reaction was completed as detected by TLC, water and ethyl acetate were added to the reaction solution, and the mixture was stirred. The organic phase was separated, dried over anhydrous sodium sulfate, and concentrated to give an oil, which was subjected to salification with maleic acid to give X185 hemimaleate (180 mg, yield: 48%). ¹H NMR (400 MHz, DMSO-*d*₆) δ 9.31 (s, 1H), 7.92 (d, *J* = 8.1 Hz, 1H), 6.44 (s, 1H), 6.04 (s, 2H), 5.95 (d, *J* = 8.1 Hz, 1H), 5.83 - 5.73 (m, 2H), 5.71 - 5.60 (m, 1H), 4.42 (d, *J* = 15.2 Hz, 2H), 3.14 - 2.99 (m, 2H), 2.77 (s, 6H), 2.69 - 2.56 (m, 1H), 2.42 (t, *J* = 7.3 Hz, 2H), 1.93 - 1.79 (m, 2H), 1.17 - 1.06 (m, 6H). ESI-MS m/z = 502.3 [M+1]⁺.

To a 25 mL three-necked flask were added X185 (200 mg, 0.40 mmol), methanol (4 mL), and potassium carbonate (5.0 mg, 0.04 mmol). The mixture was then stirred at room temperature for 5 min. After the reaction was completed as detected by TLC, acetic acid (2 mg, 0.04 mmol) was added to the reaction solution, then water and ethyl acetate were added, and the mixture was stirred. The organic phase was separated, dried over anhydrous sodium sulfate, concentrated, and purified by column chromatography to give X186, which was treated with hydrogen chloride-ethyl acetate to give X186 hydrochloride (130 mg, yield: 64%). ¹H NMR (400 MHz, DMSO-*d*₆) δ 9.68 (s, 1H), 7.78 (d, *J* = 8.1 Hz, 1H), 5.98 (d, *J* = 2.5 Hz, 1H), 5.88 (d, *J* = 8.1 Hz, 1H), 5.83 - 5.75 (m, 3H), 5.41 (d, *J* = 8.7 Hz, 1H), 4.43 - 4.14 (m, 4H), 2.95(t, *J* = 7.4 Hz, 2H), 2.69 (s, 6H), 2.63 - 2.54 (m, 1H), 2.42 (t, *J* = 7.3 Hz, 2H), 1.91 - 1.78 (m, 2H), 1.15 - 1.03 (m, 6H). ESI-MS m/z = 476.2 [M+1]⁺.

### Preparation Example 11: Synthesis of X187 and X188

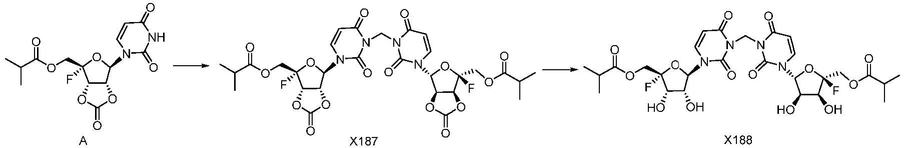

To a 100 mL three-necked flask were added A (4.0 g, 11.20 mmol) and *N*,*N*-dimethylformamide (40 mL), then potassium carbonate (1.5 g, 10.90 mmol) was added in an ice bath under nitrogen atmosphere, and the mixture was stirred for 5 min. Chloroiodomethane (9.8 g, 55.60 mmol) was added, and the mixture was heated to room temperature and stirred overnight. After the reaction was completed as detected by TLC, water and ethyl acetate were added to the reaction solution, and the mixture was stirred. The organic phase was separated, dried over anhydrous sodium sulfate, concentrated, and the purified by column chromatography to give X187 (2.8 g, yield: 34%).

To a 25 mL three-necked flask were added X187 (400 mg, 0.55 mmol), methanol (4 mL), and potassium carbonate (7.6 mg, 0.06 mmol). The mixture was then stirred at room temperature for 5 min. After the reaction was completed as detected by TLC, acetic acid was added to the reaction solution until the pH was about 7, then water and ethyl acetate were added, and the mixture was stirred. The organic phase was separated, dried over anhydrous sodium sulfate, concentrated, and purified by column chromatography to give X188 (217 mg, yield: 58%). ¹H NMR (400 MHz, DMSO-*d*₆) δ 7.66 (d, *J* = 8.1 Hz, 2H), 5.95 (d, *J* = 2.5 Hz, 2H), 5.88 (s, 2H), 5.82 - 5.69 (m, 4H), 5.34 (d, *J* = 9.0 Hz, 2H), 4.43 - 4.28 (m, 4H), 4.26 - 4.10 (m, 4H), 2.64 - 2.53 (m, 2H), 1.14 - 1.03 (m, 12H). ESI-MS m/z = 677.2 [M+1]⁺.

### Preparation Example 12: Synthesis of X114 and X52

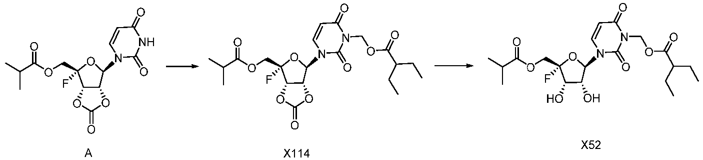

Referring to procedures in Preparation Example 3, A (400 mg, 1.12 mmol) was reacted with chloromethyl 2-ethylbutyrate (368 mg, 2.24 mmol) to give X114; X114 was reacted with potassium carbonate in methanol to give compound X52 (287 mg, two-step yield: 55%). ¹H NMR (400 MHz, Chloroform-*d*) δ 7.33 (d, *J* = 8.2 Hz, 1H), 5.94 (s, 2H), 5.82 (d, *J* = 8.1 Hz, 1H), 5.74 (d, *J* = 1.6 Hz, 1H), 4.60 - 4.44 (m, 2H), 4.41 (d, *J* = 8.1 Hz, 2H), 3.36 - 3.30 (m, 1H), 3.05 (d, *J* = 9.7 Hz, 1H), 2.67 - 2.56 (m, 1H), 2.26 - 2.16 (m, 1H), 1.64 - 1.45 (m, 4H), 1.24 - 1.16 (m, 6H), 0.89 (t, *J* = 7.5 Hz, 6H). ¹⁹F NMR (377 MHz, Chloroform-*d*) δ -121.59. ESI-MS m/z = 461.2 [M+1]⁺.

### Preparation Example 13: Synthesis of X115 and X55

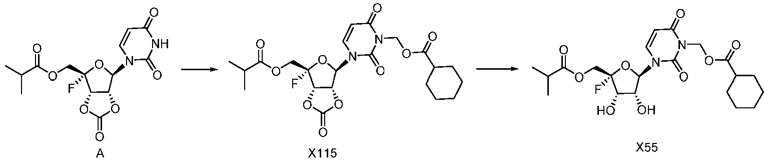

Referring to procedures in Preparation Example 3, A (400 mg, 1.12 mmol) was reacted with chloromethyl cyclohexyl formate (395 mg, 2.24 mmol) to give X115; X115 was reacted with potassium carbonate in methanol to give compound X55 (220 mg, two-step yield: 42%). ¹H NMR (400 MHz, Chloroform-*d*) δ 7.33 (d, *J* = 8.2 Hz, 1H), 5.91 (s, 2H), 5.82 (d, *J* = 8.2 Hz, 1H), 5.76 (s, 1H), 4.59 - 4.44 (m, 2H), 4.41 (d, *J* = 7.8 Hz, 2H), 3.40 (d, *J* = 4.4 Hz, 1H), 3.11 (dd, *J* = 9.7, 2.1 Hz, 1H), 2.66 - 2.54 (m, 1H), 2.37 - 2.24 (m, 1H), 1.96 - 1.82 (m, 2H), 1.79 - 1.70 (m, 2H), 1.66 - 1.58 (m, 1H), 1.50 - 1.36 (m, 2H), 1.32 - 1.14 (m, 9H). ¹⁹F NMR (377 MHz, Chloroform-*d*) δ -121.57. ESI-MS m/z = 473.2 [M+1]⁺.

### Preparation Example 14: Synthesis of X14

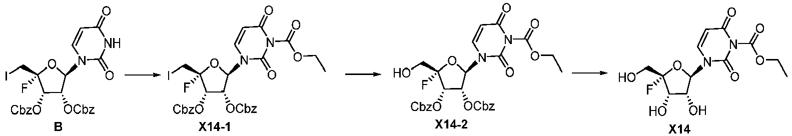

B (1 g, 1.56 mmol) and diisopropylethylamine (DIEA) (808 mg, 6.24 mmol) were dissolved in pyridine (10 mL), then ethyl chloroformate (339 mg, 3.12 mmol) was added dropwise in an ice-water bath under nitrogen atmosphere. The mixture was then heated to room temperature and reacted for 3 h. After there remained a small amount of the starting materials as monitored by TLC, the reaction solution was diluted with ethyl acetate and filtered. The filtrate was then concentrated and purified by column chromatography to give intermediate X14-1 (815 mg), which was directly used in the next step.

To a 50 mL reaction flask were sequentially added 40% tetrabutylammonium hydroxide (3.70 g, 5.70 mmol) and dichloromethane (10 mL), then trifluoroacetic acid (649 mg, 5.70 mmol) was added in an ice-water bath, and the mixture was stirred for 10 min. A solution of X14-1 (815 mg, 1.14 mmol) in dichloromethane was added dropwise at room temperature, then 85% *m*-chloroperoxybenzoic acid (1.16 g, 5.70 mmol) was added in batches, and the mixture was reacted overnight. After the reaction was completed as detected by TLCreaction was completed as detected, water and dichloromethane were added to the reaction solution, and the mixture was left to stand for liquid separation. The organic phase was separated, sequentially washed with water and saturated brine, dried over anhydrous sodium sulfate, concentrated, and the purified by silica gel column chromatography to give intermediate X14-2 (340 mg, two-step yield: 36%) as an oil.

X14-2 (340 mg) was dissolved in tetrahydrofuran (6.8 mL), then 10% palladium on carbon (170 mg) was added, and the mixture was purged with hydrogen and reacted at room temperature for 1 h under normal pressure. After the reaction was completed as detected by TLCreaction was completed as detected, the reaction solution was filtered to remove palladium on carbon. The filtrate was then concentrated and purified by silica gel column chromatography to give compound X14 (120 mg, yield: 63%) as a white solid. ¹H NMR (400 MHz, DMSO-*d*₆) δ 7.87 (d, *J* = 8.2 Hz, 1H), 5.97 (d, *J* = 1.8 Hz, 1H), 5.90 (d, *J* = 8.2 Hz, 1H), 5.75 (d, *J* = 5.1 Hz, 1H), 5.56 (t, *J* = 5.8 Hz, 1H), 5.21 (d, *J* = 8.5 Hz, 1H), 4.41 (q, *J* = 7.1 Hz, 2H), 4.30 - 4.17 (m, 2H), 3.61 -3.54 (m, 2H), 1.30 (t, *J* = 7.1 Hz, 3H). ESI-MS m/z = 335.1 [M+1]⁺.

### Preparation Example 15: Synthesis of X43

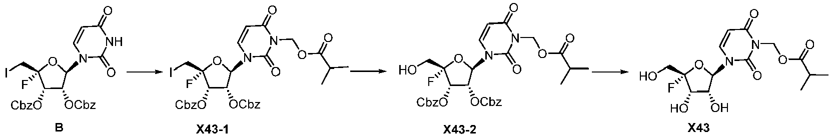

B (800 mg, 1.25 mmol) was dissolved in acetone (8 mL), then potassium carbonate (173 mg, 1.25 mmol) was added, and chloromethyl isobutyrate (257 mg, 1.88 mmol) was added dropwise in an ice-water bath under nitrogen atmosphere. The mixture was reacted at room temperature for 3 h. After the reaction was completed as detected by TLC, the reaction solution was diluted with ethyl acetate, filtered, concentrated, and purified by column chromatography to give intermediate X43-1 (770 mg, yield: 83%) as an oil.

40% tetrabutylammonium hydroxide (3.37 g, 5.20 mmol) was dissolved in dichloromethane (7.7 mL), then trifluoroacetic acid (593 mg, 5.20 mmol) was added in an ice-water bath, and the mixture was stirred for 10 min. A solution of X43-1 (770 mg, 1.04 mmol) in dichloromethane (7.7 mL) was added dropwise, then 85% *m*-chloroperoxybenzoic acid (1.06 g, 5.20 mmol) was added in batches, and the mixture was reacted at room temperature overnight. After the reaction was completed as detected by TLC, water and dichloromethane were added to the reaction solution, and the mixture was stirred. The organic phase was separated, sequentially washed with water and saturated brine, dried over anhydrous sodium sulfate, concentrated, and purified by silica gel column chromatography to give intermediate X43-2 (320 mg, yield: 49%) as an oil.

X43-2 (320 mg, 0.51 mmol) was dissolved in tetrahydrofuran (3.2 mL), then 10% palladium on carbon (160 mg) was added, and the mixture was purged with hydrogen three times and reacted at room temperature for 2 h under normal pressure. After the reaction was completed as detected by TLC, the reaction solution was filtered, and the filtrate was concentrated. The residue was purified by silica gel column chromatography to give compound X43 (90 mg, yield: 49%). ¹H NMR (400 MHz, DMSO-*d*₆) δ 7.83 (d, *J* = 8.2 Hz, 1H), 6.01 (d, *J* = 2.4 Hz, 1H), 5.86 (d, *J* = 8.2 Hz, 1H), 5.77 (s, 2H), 5.73 (d, *J* = 5.5 Hz, 1H), 5.53 (t, *J* = 5.8 Hz, 1H), 5.21 (d, *J* = 8.7 Hz, 1H), 4.31 - 4.14 (m, 2H), 3.61 - 3.54 (m, 2H), 2.58 - 2.50 (m, 1H), 1.06 (d, *J* = 6.9 Hz, 6H). ESI-MS m/z = 363.2 [M+1]⁺.

### Preparation Example 16: Synthesis of X44

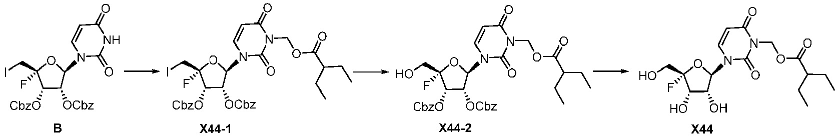

Referring to procedures in Preparation Example 15, B (1 g, 1.56 mmol) was reacted with chloromethyl 2-ethylbutyrate (770 mg, 4.68 mmol) to give intermediate X44-1; X44-1 was oxidized with *m*-chloroperoxybenzoic acid to give intermediate X44-2; X44-2 was deprotected by hydrogen/palladium on carbon, and the obtained product was purified by silica gel column chromatography to give compound X44 (100 mg, three-step yield: 16%). ¹H NMR (400 MHz, DMSO-*d*₆) δ 7.84 (d, *J* = 8.2 Hz, 1H), 6.01 (d, *J* = 2.6 Hz, 1H), 5.86 (d, *J* = 8.2 Hz, 1H), 5.79 (s, 2H), 5.73 (d, *J* = 5.4 Hz, 1H), 5.53 (t, *J* = 5.9 Hz, 1H), 5.22 (d, *J* = 8.7 Hz, 1H), 4.31 - 4.12 (m, 2H), 3.62 - 3.53 (m, 2H), 2.23 - 2.14 (m, 1H), 1.56 - 1.37 (m, 4H), 0.82 (t, *J* = 7.4 Hz, 6H). ESI-MS m/z = 391.2 [M+1]⁺.

### Preparation Example 17: Synthesis of X47

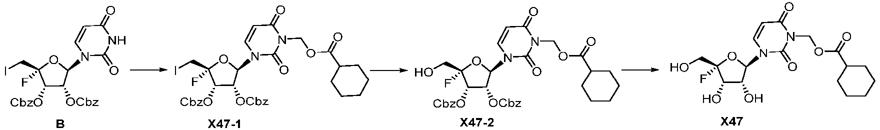

Referring to procedures in Preparation Example 15, B (1 g, 1.56 mmol) was reacted with chloromethyl cyclohexyl formate (828 mg, 4.68 mmol) to give intermediate X47-1; X47-1 was oxidized with *m*-chloroperoxybenzoic acid to give intermediate X47-2; X47-2 was deprotected by hydrogen/palladium on carbon, and the obtained product was purified by silica gel column chromatography to give compound X47 (130 mg, three-step yield: 21%). ¹H NMR (400 MHz, DMSO-*d*₆) δ 7.83 (d, *J* = 8.2 Hz, 1H), 6.00 (d, *J* = 2.5 Hz, 1H), 5.85 (d, *J* = 8.2 Hz, 1H), 5.77 (s, 2H), 5.73 (d, *J* = 5.3 Hz, 1H), 5.53 (t, *J* = 5.9 Hz, 1H), 5.21 (d, *J* = 8.7 Hz, 1H), 4.31 - 4.13 (m, 2H), 3.62 - 3.54 (m, 2H), 2.36 - 2.26 (m, 1H), 1.83 - 1.72 (m, 2H), 1.69 - 1.59 (m, 2H), 1.40 - 1.10 (m, 6H). ESI-MS m/z = 403.2 [M+1]⁺.

### Preparation Example 18: Synthesis of X57

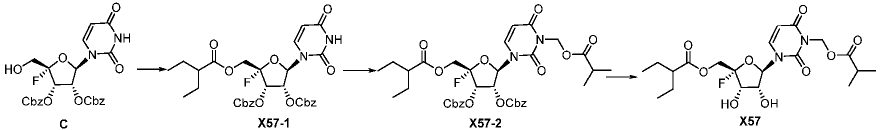

To a 100 mL three-necked flask were added C (1.1 g, 2.07 mmol), 4-dimethylaminopyridine (127 mg, 1.04 mmol), diisopropylethylamine (677 mg, 5.18 mmol), and dichloromethane (11 mL), and the mixture was stirred until a clear solution was obtained. 2-Ethylbutyrylchloride (339 mg, 2.48 mmol) was added dropwise in an ice-water bath, and the mixture was reacted at this temperature for 30 min. After the reaction was completed as detected by TLC, dichloromethane was added to the reaction solution, the mixture was washed with saturated sodium bicarbonate solution four times, dried over anhydrous sodium sulfate, filtered, and concentrated to give compound X57-1 (crude product, 1.40 g), which was directly used in the next step.

To a 100 mL three-necked flask were added X57-1 (1.40 g, 2.07 mmol), potassium carbonate (286 mg, 2.07 mmol), and *N*,*N*-dimethylformamide (13 mL), and the mixture was stirred. Chloromethyl isobutyrate (565 mg, 4.14 mmol) was added dropwise in an ice-water bath. After the addition, the mixture was heated to room temperature and reacted overnight. After the reaction was completed as detected by TLC, water and ethyl acetate were added to the reaction solution, and the mixture was left to stand for liquid separation. The organic phase was separated and concentrated, and purified by silica gel column chromatography to give compound X57-2 (620 mg, two-step yield: 41%).

To a 25 mL single-necked flask were added X57-2 (620 mg, 0.85 mmol), 10% Pd/C (310 mg), and tetrahydrofuran (12 mL), and the mixture was replaced with hydrogen 3 times and reacted at room temperature for 3 h under normal pressure. After the reaction was completed as detected by TLC, the reaction solution was filtered, and the filtrate was concentrated. The residue was purified by silica gel column chromatography to give compound X57 (250 mg, yield: 64%). ¹H NMR (400 MHz, Chloroform-*d*) δ 7.28 (d, *J* = 8.2 Hz, 1H), 5.86 (s, 2H), 5.75 (d, *J* = 8.2 Hz, 1H), 5.72 (s, 1H), 4.42 - 4.28 (m, 4H), 3.38 (s, 1H), 3.03 (s, 1H), 2.55 - 2.41 (m, 1H), 2.25 - 2.12 (m, 1H), 1.58 - 1.42 (m, 4H), 1.10 (d, *J* = 7.0 Hz, 6H), 0.83 (t, *J* = 7.4 Hz, 6H). ESI-MS m/z = 459.2 [M-1]⁻.

### Preparation Example 19: Synthesis of X58

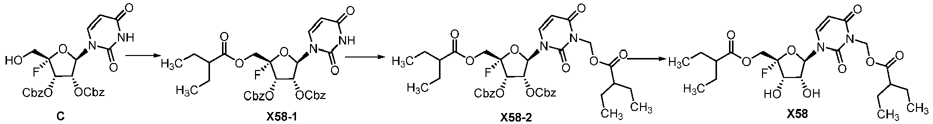

Referring to procedures in Preparation Example 18, C (300 mg, 0.57 mmol) was reacted with 2-ethylbutyrylchloride (100 mg, 0.74 mmol) to give intermediate X58-1; X58-1 was reacted with chloromethyl 2-ethylbutyrate to give intermediate X58-2; finally, the protecting group of X58-2 was removed under the condition of hydrogen/palladium carbon to give compound X58 (70 mg, three-step yield: 25%). ¹H NMR (400 MHz, DMSO-*d*₆) δ 7.80 (d, *J* = 8.1 Hz, 1H), 5.95 (s, 1H), 5.87 (d, *J* = 8.1 Hz, 1H), 5.82 - 5.74 (m, 3H), 5.38 (d, *J* = 8.5 Hz, 1H), 4.44 - 4.16 (m, 4H), 2.30 - 2.13 (m, 2H), 1.61 - 1.39 (m, 8H), 0.90 - 0.73 (m, 12H). ESI-MS m/z = 487.2 [M-1]⁻.

### Preparation Example 20: Synthesis of X61

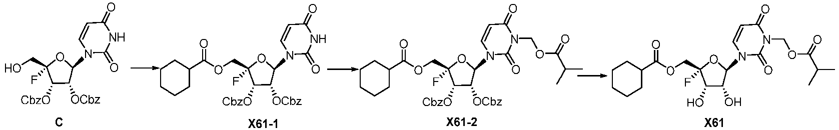

Referring to procedures in Preparation Example 18, C (600 mg, 1.13 mmol) was reacted with cyclohexanecarbonyl chloride (215 mg, 1.47 mmol) to give intermediate X61-1; X61-1 was reacted with chloromethyl isobutyrate to give intermediate X61-2; X61-2 was deprotected by hydrogen/palladium on carbon to give compound X61 (200 mg, three-step yield: 37%). ¹H NMR (400 MHz, Chloroform-*d*) δ 7.35 (d, *J* = 8.2 Hz, 1H), 5.93 (s, 2H), 5.84 - 5.76 (m, 2H), 4.56 - 4.35 (m, 4H), 3.49 (s, 1H), 3.14 (s, 1H), 2.62 - 2.50 (m, 1H), 2.41 - 2.30 (m, 1H), 1.97 - 1.86 (m, 2H), 1.83 - 1.72 (m, 2H), 1.52 - 1.38 (m, 2H), 1.36 - 1.21 (m, 4H), 1.16 (d, *J* = 7.0 Hz, 6H). ESI-MS m/z = 471.2 [M-1]⁻.

### Preparation Example 21: Synthesis of X79 Hydrochloride

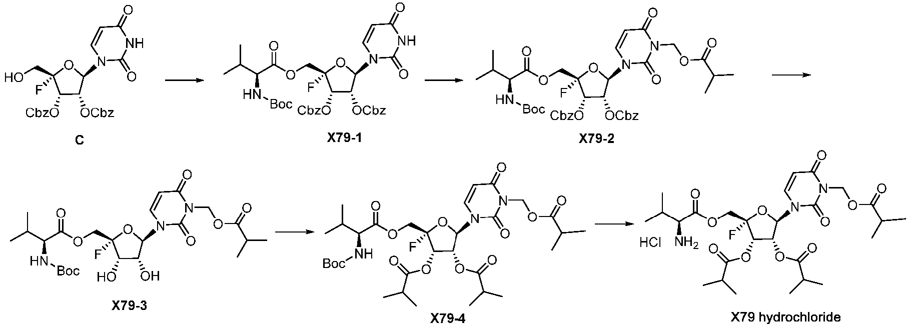

C (680 mg, 1.28 mmol), N-tert-butoxycarbonyl-L-valine (361 mg, 1.66 mmol), *N*,*N*-diisopropylethylamine (497 mg, 3.84 mmol), and 4-dimethylaminopyridine (156 mg, 1.28 mmol) were dissolved in dichloromethane (3 mL), then 2-(7-azabenzotriazol-1-yl)-*N,N,N',N'*-tetramethyluronium hexafluorophosphate (HATU) (731 mg, 1.92 mmol) were added in an ice-water bath under nitrogen atmosphere, and the mixture was heated to room temperature and reacted for 1 h. After the reaction was completed as monitored by TLC, water was added to the reaction solution, and the mixture was stirred and left to stand for liquid separation. The organic phase was separated, concentrated, and purified by silica gel column chromatography to give intermediate X79-1 (870 mg, yield: 93%).

X79-1 (830 mg, 1.14 mmol) was dissolved in *N*,*N* dimethylformamide (9 mL), potassium carbonate (158 mg, 1.14 mmol) was added, then chloromethyl isobutyrate (233 mg, 1.14 mmol) was slowly added in an ice-water bath under nitrogen atmosphere, and the mixture was reacted at room temperature overnight. After the starting materials disappeared as monitored by TLC, water and ethyl acetate were added to the reaction solution, and the mixture was left to stand for liquid separation. The organic phase was separated, dried, and concentrated to give intermediate X79-2 (crude product, 1.28 g), which was directly used in the next step.

X79-2 (1.28 g, 1.14 mmol) was dissolved in tetrahydrofuran (12 mL), then 10% palladium on carbon (640 mg) was added, and the mixture was purged with hydrogen three times and reacted at room temperature for 4 h under normal pressure. After the starting materials disappeared as monitored by TLC, the reaction solution was filtered and concentrated to give intermediate X79-3 (crude product, 890 mg), which was directly used in the next step.

X79-3 (200 mg, 0.36 mmol), 4-dimethylaminopyridine (44 mg, 0.36 mmol), and triethylamine (182 mg, 1.80 mmol) were dissolved in dichloromethane, then isobutyric anhydride (171 mg, 1.08 mmol) was added dropwise in an ice-water bath under nitrogen atmosphere, and the mixture was stirred at room temperature for 2 h. After the reaction was completed as detected by TLC, water was added to the reaction solution, and the mixture was stirred and left to stand for liquid separation. The organic phase was separated, dried over anhydrous sodium sulfate, concentrated, and purified by silica gel column chromatography to give intermediate X79-4 (180 mg, yield: 72%).

X79-4 (180 mg) was dissolved in ethyl acetate (1 mL), then a 4 N hydrogen chloride-ethyl acetate solution (2 mL) was added, and the mixture was stirred at room temperature for 2 h. After the reaction was completed as detected by TLC, the reaction solution was concentrated to give compound X79 hydrochloride (105 mg, yield: 64%). ¹H NMR (400 MHz, DMSO-*d*₆) δ 7.92 (d, *J* = 8.4 Hz, 1H), 7.80 (s, 3H), 6.13 (d, *J* = 2.1 Hz, 1H), 5.94 (d, *J* = 8.1 Hz, 1H), 5.86 - 5.72 (m, 3H), 5.62 (dd, *J* = 7.3, 2.1 Hz, 1H), 4.54 - 4.44 (m, 2H), 3.82 (d, *J* = 4.2 Hz, 1H), 2.66 - 2.52 (m, 3H), 2.27 - 2.14 (m, 1H), 1.16 - 1.03 (m, 18H), 0.95 (d, *J* = 6.9 Hz, 6H). ESI-MS m/z = 602.3 [M+1]⁺.

### Preparation Example 22: Synthesis of X97 Hydrochloride

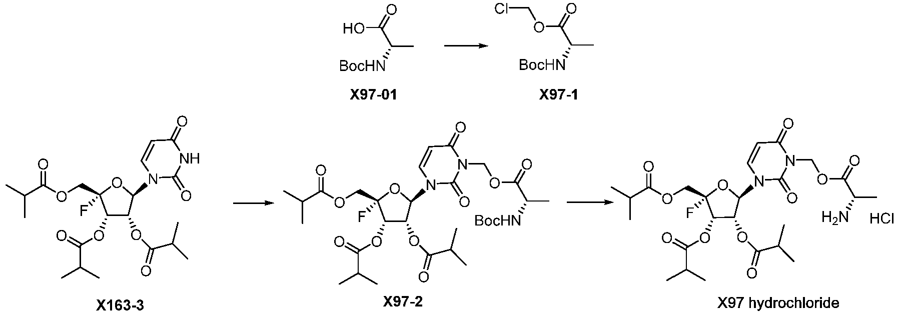

X97-01 (2.0 g, 10.57 mmol) was added to ethanol (20 mL), then a solution of cesium carbonate (3.4 g, 10.57 mmol) in water (7 mL) was added dropwise, and the mixture was stirred at room temperature for 30 min and concentrated to dryness. Toluene was added, and the mixture was concentrated to dryness. This operation was repeated twice. *N*,*N*-dimethylformamide (10 mL) was added, then chloroiodomethane (8 mL, 105.70 mmol) was added dropwise at 0-10 °C, and the mixture was stirred at room temperature overnight. Water and ethyl acetate were added to the reaction solution, and the mixture was left to stand for liquid separation. The organic phase was separated, washed with a saturated sodium chloride solution, dried over anhydrous sodium sulfate, filtered, concentrated, and purified by silica gel column chromatography to give compound X97-1 (1.20 g, yield: 48%).

X163-3 (300 mg, 0.63 mmol), X97-1 (449 mg, 1.89 mmol), potassium carbonate (174 mg, 1.26 mmol), and sodium iodide (94 mg, 0.63 mmol) were added to acetone (10 mL), and the mixture was stirred at room temperature overnight. Water and ethyl acetate were added to the reaction solution, and the mixture was left to stand for liquid separation. The organic phase was separated, washed with a saturated sodium chloride solution, dried over anhydrous sodium sulfate, filtered, concentrated, and purified by column chromatography to give compound X97-2 (380 mg, yield: 89%).

X97-2 (380 mg, 0.56 mmol) was dissolved in ethyl acetate (2 mL), then a 4 M hydrogen chloride-ethyl acetate solution (5 mL) was added, and the mixture was stirred at room temperature for 2 h. The reaction solution was concentrated to dryness. Water and ethyl acetate were added, and the mixture was left to stand for liquid separation. The aqueous phase was isolated and lyophilized to give compound X97 hydrochloride (200 mg, yield: 58%). ¹H NMR (400 MHz, DMSO-*d*₆) δ 8.53 (s, 3H), 8.02 (d, *J* = 8.2 Hz, 1H), 6.22 (d, *J* = 2.0 Hz, 1H), 6.06 - 5.82 (m, 4H), 5.68 (dd, *J* = 7.2, 2.0 Hz, 1H), 4.51 - 4.33 (m, 2H), 4.18 (q, *J* = 7.2 Hz, 1H), 2.77 - 2.57 (m, 3H), 1.45 (d, *J* = 7.1 Hz, 3H), 1.24 - 1.12 (m, 18H). ESI-MS m/z = 574.3 [M+1]⁺. Preparation Example 23: Synthesis of X98 Hydrochloride

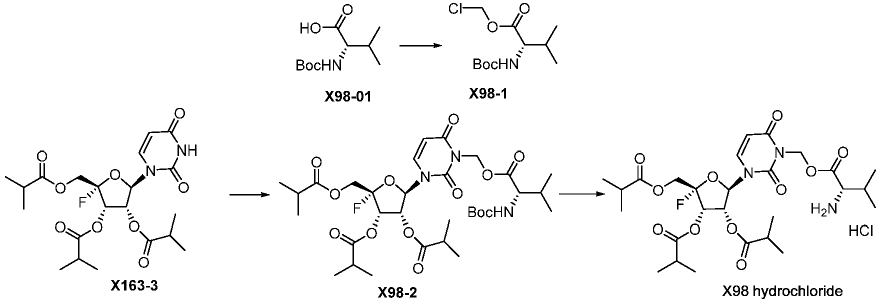

Referring to procedures in Preparation Example 22, compound X163-3 (300 mg, 0.63 mmol) was reacted with self-made X98-1 (502 mg, 1.89 mmol) to give compound X98-2; compound X98-2 was deprotected by a hydrogen chloride-ethyl acetate solution to give compound X98 hydrochloride (240 mg, two-step yield: 63%). ¹H NMR (400 MHz, DMSO-*d*₆) δ 8.50 (s, 3H), 7.95 (d, *J* = 8.2 Hz, 1H), 6.13 (d, *J* = 2.0 Hz, 1H), 6.00 - 5.93 (m, 2H), 5.86 - 5.73 (m, 2H), 5.58 (dd, *J* = 7.3, 2.0 Hz, 1H), 4.42 - 4.24 (m, 2H), 3.92 (d, *J* = 4.4 Hz, 1H), 2.66 - 2.53 (m, 3H), 2.20 - 2.08 (m, 1H), 1.17 - 1.04 (m, 18H), 0.98 - 0.88 (m, 6H). ESI-MS m/z = 602.3 [M+1]⁺.

### Preparation Example 24: Synthesis of X100 Hydrochloride

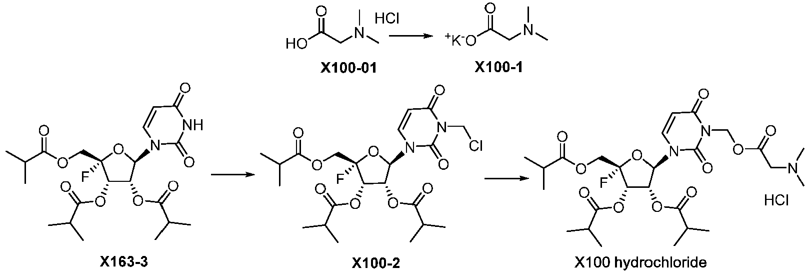

X100-01 (1.0 g, 7.16 mmol) was dissolved in methanol (10 mL), then a solution of potassium hydroxide (402 mg, 7.16 mmol) in methanol (2 mL) was added dropwise, and the mixture was stirred for 30 min. The reaction solution was concentrated to dryness to give compound X100-1 (crude product, 800 mg), which was directly used in the next step.

X163-3 (600 mg, 1.27 mmol) was dissolved in *N*,*N*-dimethylformamide (3 mL), then 60% sodium hydride (102 mg, 2.54 mmol) was added in an ice-water bath, and the mixture was reacted for 30 min. The above reaction solution was then slowly added to a solution of chloroiodomethane (2.23 g, 12.70 mmol) in *N*,*N*-dimethylformamide (1 mL), and the mixture was reacted for 2 h. After there remained a small amount of the starting materials as monitored by TLC, water and ethyl acetate were added to the reaction solution, and the mixture was stirred and left to stand for liquid separation. The organic phase was separated, dried over anhydrous sodium sulfate, filtered, concentrated, and purified by silica gel column chromatography to give compound X100-2 (400 mg, yield: 60%).

X100-2 (470 mg, 0.90 mmol), potassium iodide (150 mg, 0.90 mmol), and X100-1 (255 mg, 1.80 mmol) were added to *N*,*N*-dimethylformamide (5 mL), and the mixture was reacted at room temperature overnight. After the starting materials were completely reacted as detected by TLC, the reaction solution was concentrated. Water and ethyl acetate were added, and the mixture was stirred and left to stand for liquid separation. The organic phase was separated, dried over anhydrous sodium sulfate, filtered, concentrated, and purified by silica gel column chromatography to give compound X100, which was treated with hydrogen chloride-ethyl acetate to give X100 hydrochloride (96 mg, yield: 17%). ¹H NMR (400 MHz, DMSO-*d*₆) δ 10.17 (s, 1H), 7.94 (d, *J* = 8.2 Hz, 1H), 6.13 (s, 1H), 5.97 - 5.89 (m, 3H), 5.80 (dd, *J* = 20.1, 7.2 Hz, 1H), 5.61 (dd, *J* = 7.3, 1.9 Hz, 1H), 4.41 - 4.17 (m, 4H), 2.83 (s, 6H), 2.65 - 2.52 (m, 3H), 1.16 - 1.05 (m, 18H). ESI-MS m/z = 588.3 [M+1]⁺.

### Preparation Example 25: Synthesis of X173

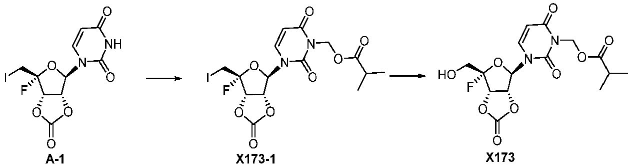

A-1 (500 mg, 1.26 mmol) was dissolved in *N*,*N*-dimethylformamide (10 mL), then potassium carbonate (174 mg, 1.26 mmol) was added, and the mixture was stirred. Chloromethyl isobutyrate (258 mg, 1.89 mmol) was added dropwise in an ice-water bath, and the mixture was reacted at room temperature overnight. After the starting materials were completely reacted as detected by TLC, water and ethyl acetate were added to the reaction solution, and the mixture was left to stand for liquid separation. The organic phase was separated, concentrated, and purified by silica gel column chromatography to give compound X173-1 (300 mg, yield: 48%).

40% tetrabutylammonium hydroxide (1.95 g, 3.00 mmol) was dissolved in dichloromethane (10 mL), then trifluoroacetic acid (342 mg, 3.00 mmol) was added in an ice-water bath, and the mixture was stirred for 10 min. X173-1 (300 mg, 0.60 mmol) and 85% *m*-chloroperoxybenzoic acid (600 mg, 3.00 mmol) were added to the reaction solution, and the mixture was reacted at room temperature overnight. After the starting materials were completely reacted as detected by TLC, water was added to the reaction solution, and the mixture was left to stand for liquid separation. The organic phase was separated , concentrated, and purified by silica gel column chromatography to give compound X173 (85 mg, yield: 36%). ¹H NMR (400 MHz, DMSO-*d*₆) δ 7.91 (d, *J* = 8.2 Hz, 1H), 6.34 (s, 1H), 5.92 (d, *J* = 8.1 Hz, 1H), 5.81 - 5.67 (m, 3H), 5.64 - 5.51 (m, 2H), 3.80 - 3.53 (m, 2H), 2.57 - 2.49 (m, 1H), 1.06 (d, *J* = 7.0 Hz, 6H). ESI-MS m/z = 389.2 [M+1]⁺.

### Preparation Example 26: Synthesis of X174

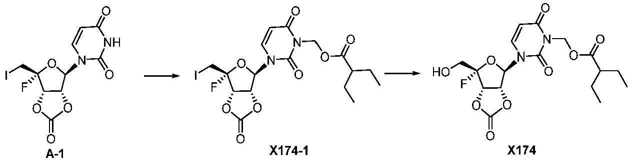

Referring to Preparation Example 25, A-1 (500 mg, 1.26 mmol) was reacted with chloromethyl 2-ethylbutyrate (311 mg, 1.89 mmol) to give compound X174-1; compound X174-1 was oxidized with *m*-chloroperoxybenzoic acid to give compound X174 (85 mg, two-step yield: 16%). ¹H NMR (400 MHz, DMSO-*d*₆) δ 7.90 (d, *J* = 8.0 Hz, 1H), 6.34 (s, 1H), 5.92 (d, *J* = 8.1 Hz, 1H), 5.81 - 5.69 (m, 3H), 5.63 - 5.51 (m, 2H), 3.78 - 3.54 (m, 3H), 2.24 - 2.12 (m, 1H), 1.55 - 1.37 (m, 4H), 0.81 (t, *J* = 8.0 Hz, 6H). ESI-MS m/z = 417.2 [M+1]⁺.

### Preparation Example 27: Synthesis of X198

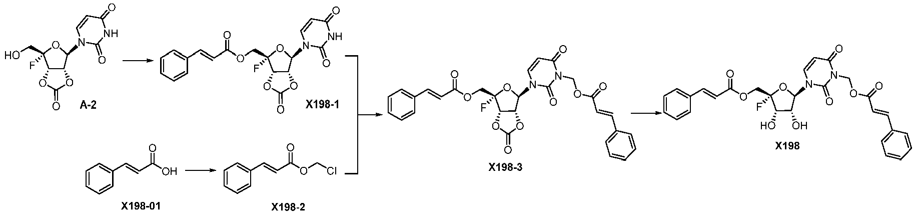

A-2 (600 mg, 2.08 mmol) and *N*,*N*-diisopropylethylamine (673 mg, 5.20 mmol) were added to dichloromethane (10 mL), then cinnamoyl chloride (416 mg, 2.50 mmol) was added dropwise in an ice-water bath, and the mixture was reacted at room temperature for 2 h. After there remained a small amount of the starting materials as detected by TLC, water and ethyl acetate were added to the reaction solution, and the mixture was left to stand for liquid separation. The organic phase was separated, washed with saturated brine, dried over anhydrous sodium sulfate, filtered, concentrated, and purified by column chromatography to give compound X198-1 (450 mg, yield: 52%).

*Trans*-cinnamic acid (2.0 g, 13.50 mmol), tetrabutylammonium hydrogen sulfate (458 mg, 1.35 mmol), and solid sodium bicarbonate (4.31 g, 51.30 mmol) were added to dichloromethane (14 mL) and water (14 mL), and the mixture was stirred. Chloromethyl chlorosulfonate (2.45 g, 14.85 mmol) was added dropwise in an ice-water bath, and the mixture was reacted at room temperature for 2 h. After there were no starting materials remaining as detected by TLC, water was added to the reaction solution, and the mixture was left to stand for liquid separation, The organic phase was separated, dried over sodium sulfate, filtered, concentrated, and purified by column chromatography to give compound X198-2 (2 g, yield: 75%).

X198-1 (450 mg, 1.08 mmol) and potassium carbonate (149 mg, 1.08 mmol) were added to *N*,*N*-dimethylformamide (5 mL), then X198-2 (425 mg, 2.16 mmol) was added dropwise at 0-10 °C, and the mixture was reacted at room temperature for 5 h. After there were no starting materials remaining as detected by TLC, water and ethyl acetate were added to the reaction solution, and the mixture was left to stand for liquid separation. The organic phase was separated, washed with a saturated sodium chloride solution, dried over anhydrous sodium sulfate, filtered, concentrated, and purified by column chromatography to give compound X198-3 (380 mg, yield: 61%).

X198-3 (380 mg, 0.66 mmol) was added to 1,4-dioxane (10 mL) and water (3 mL), then a solution of potassium hydroxide (37 mg, 0.66 mmol) in water (3 mL) was added dropwise in an ice-water bath, and the mixture was stirred for 2 h. After there were starting materials remained as detected by TLC, water and ethyl acetate were added to the reaction solution, and the mixture was left to stand for liquid separation. The organic phase was separated, washed with a saturated sodium chloride solution, dried over anhydrous sodium sulfate, filtered, concentrated, and purified by column chromatography to give compound X198 (200 mg, yield: 55%). ¹H NMR (400 MHz, Chloroform-*d*) δ 7.81 - 7.64 (m, 2H), 7.56 - 7.44 (m, 4H), 7.44 - 7.33 (m, 7H), 6.50 - 6.31 (m, 2H), 6.11 - 6.01 (m, 2H), 5.86 - 5.76 (m, 2H), 4.71 - 4.48 (m, 4H), 3.26 (s, 1H), 3.09 - 2.96 (m, 1H). ESI-MS m/z = 551.1 [M-1]⁻.

### Preparation Example 28: Synthesis of X199

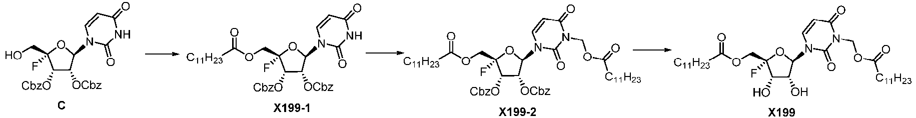

Referring to procedures in Preparation Example 18, C (600 mg, 1.13 mmol) was reacted with lauryl chloride (297 mg, 1.36 mmol) to give compound X199-1; X199-1 was reacted with chloromethyl laurate to give X199-2; finally, X199-2 was deprotected by hydrogen/palladium on carbon to give compound X199 (100 mg, three-step yield: 13%). ¹H NMR (400 MHz, Chloroform-*d*) δ 7.32 (d, *J* = 8.2 Hz, 1H), 5.93 (s, 2H), 5.82 (d, *J* = 8.0 Hz, 1H), 5.75 (s, 2H), 4.58 - 4.37 (m, 3H), 3.29 (s, 1H), 3.00 (d, *J* = 9.3 Hz, 1H), 2.43 - 2.23 (m, 4H), 1.69 - 1.54 (m, 4H), 1.37 - 1.19 (m, 32H), 0.88 (t, *J* = 6.7 Hz, 6H). ESI-MS m/z = 323.2 [M-1]⁻.

### Preparation Example 29: Synthesis of X200

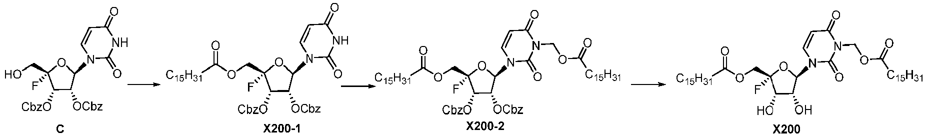

Referring to procedures in Preparation Example 18, C (400 mg, 0.75 mmol) was reacted with palmitoyl chloride (247 mg, 0.90 mmol) to give compound X200-1; X200-1 was reacted with chloromethyl palmitate to give X200-2; finally, X200-2 was deprotected by hydrogen/palladium carbon to give compound X200 (50 mg, three-step yield: 9%). ¹H NMR (400 MHz, Chloroform-*d*) δ 7.32 (d, *J* = 8.2 Hz, 1H), 5.93 (s, 2H), 5.82 (d, *J* = 8.2 Hz, 1H), 5.75 (s, 1H), 4.57 - 4.37 (m, 4H), 3.42 - 2.88 (m, 2H), 2.42 - 2.26 (m, 4H), 1.81 - 1.50 (m, 4H), 1.38 - 1.15 (m, 48H), 0.88 (t, *J* = 6.7 Hz, 6H). ESI-MS m/z = 379.3 [M+1]⁺.

### Preparation Example 30: Synthesis of X201

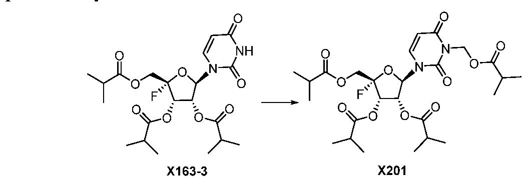

X163-3 (250 mg, 0.53 mmol), potassium carbonate (147 mg, 1.06 mmol), and sodium iodide (79 mg, 0.53 mmol) were added to acetone (5 mL), then chloromethyl isobutyrate (145 mg, 1.06 mmol) was added dropwise, and the mixture was reacted at room temperature overnight. After the starting materials were completely converted as monitored by TLC, the reaction solution was filtered, and the filtrate was concentrated. The residue was purified by silica gel column chromatography to give compound X201 (270 mg, yield: 89%) as an oil. ¹H NMR (400 MHz, DMSO-*d*₆) δ 7.91 (d, *J* = 8.2 Hz, 1H), 6.11 (d, *J* = 2.1 Hz, 1H), 5.97 - 5.74 (m, 4H), 5.61 (dd, *J* = 7.2, 2.1 Hz, 1H), 4.42 - 4.25 (m, 2H), 2.67 - 2.50 (m, 4H), 1.17 - 1.02 (m, 24H). ESI-MS m/z = 573.3 [M+1]⁺.

### Preparation Example 31: Synthesis of X202 Hydrochloride

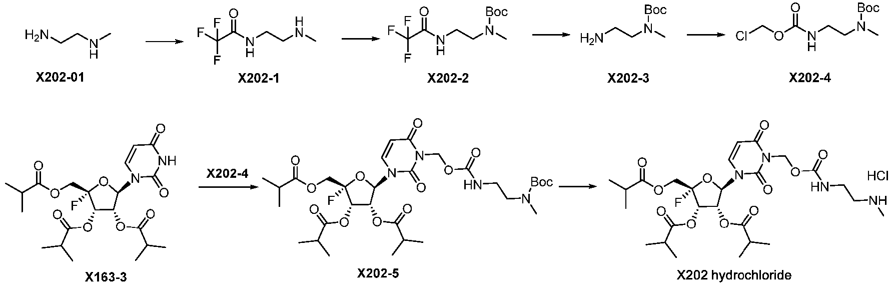

X202-01 (3 g, 40.47 mmol) was dissolved in acetonitrile (45 mL), then ethyl trifluoroacetate (5.75 g, 40.47 mmol) was added dropwise, and the mixture was heated to 80 °C and reacted for 1 h. After the reaction was completed as detected by TLC, the reaction solution was concentrated to give compound X202-1 (crude product, 6.75 g), which was directly used in the next step.

X202-1 (6.75 g, 39.67 mmol) was dissolved in tetrahydrofuran (33 mL), then a solution of di-*tert*-butyl dicarbonate (10.39 g, 47.60 mmol) in tetrahydrofuran (33 mL) was added dropwise, and the mixture was reacted at room temperature for 1 h. After the reaction was completed as detected by TLC, the reaction solution was concentrated to give compound X202-2 (crude product, 10.50 g), which was directly used in the next step.

X202-2 (10.50 g, 39.67 mmol) was dissolved in methanol (105 mL) and water (31.5 mL), then solid sodium hydroxide (6.35 g, 158.68 mmol) was added, and the mixture was reacted at room temperature for 1 h. After the reaction was completed as detected by TLC, the reaction solution was concentrated. Water and dichloromethane were added, and the mixture was left to stand for liquid separation. The organic phase was separated, washed with saturated brine, dried over anhydrous sodium sulfate, and concentrated to give compound X202-3 (crude product, 6.66 g), which was directly used in the next step.

X202-3 (6.65 g, 38.16 mmol) and triethylamine (7.72 g, 76.32 mmol) were dissolved in dichloromethane (66 mL), then chloromethyl chloroformate (4.92 g, 38.16 mmol) was added dropwise in an ice-water bath, and the mixture was stirred at room temperature for 1 h. After the reaction was completed as detected by TLC, water and dichloromethane were added to the reaction solution, and the mixture was left to stand for liquid separation. The organic phase was separated, dried over anhydrous sodium sulfate, and concentrated, and the residue was purified by silica gel column chromatography to give compound X202-4 (3.75 g, four-step yield: 35%) as an oil.

X163-3 (500 mg, 1.06 mmol), X202-4 (339 mg, 1.27 mmol), sodium iodide (159 mg, 1.06 mmol), and potassium carbonate (293 mg, 2.12 mmol) were added to acetone (8 mL), and the mixture was reacted at room temperature overnight. After the reaction was completed as detected by TLC, water and ethyl acetate were added to the reaction solution, and the mixture was left to stand for liquid separation. The organic phase was separated, dried over anhydrous sodium sulfate, concentrated, and purified by silica gel column chromatography to give compound X202-5 (220 mg, yield: 30%) as an oil.

X202-5 (220 mg, 0.31 mmol) was dissolved in ethyl acetate (2.20 mL), then a 4 M hydrogen chloride-ethyl acetate solution (2.20 mL) was added under nitrogen atmosphere, and the mixture was reacted at room temperature for 30 min. After the reaction was completed as detected by TLC, the reaction solution was concentrated, and the residue was slurried with diethyl ether and filtered to give compound X202 hydrochloride (100 mg, yield: 50%) as an off-white solid. ¹H NMR (400 MHz, DMSO-*d*₆) δ 8.77 (s, 2H), 7.92 (d, *J* = 8.1 Hz, 1H), 7.57 (t, *J* = 5.8 Hz, 1H), 6.11 (s, 1H), 5.92 (d, *J* = 8.1 Hz, 1H), 5.88 - 5.72 (m, 2H), 5.72 - 5.53 (m, 2H), 4.43 - 4.23 (m, 2H), 3.35 - 3.20 (m, 2H), 3.02 - 2.86 (m, 2H), 2.67 - 2.45 (m, 6H), 1.22 - 0.97 (m, 18H). ESI-MS m/z = 603.3 [M+1]⁺.

### Preparation Example 32: Synthesis of X203 Hydrochloride

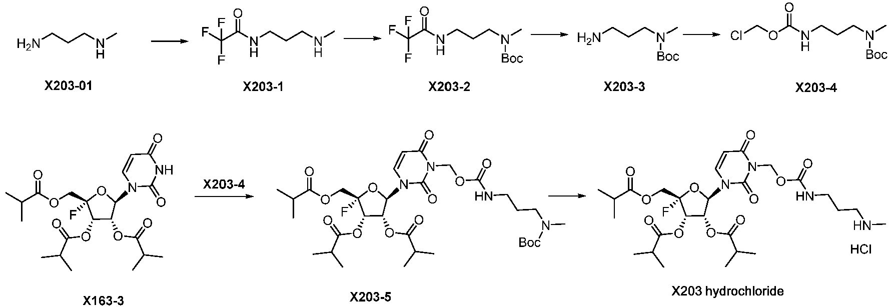

Referring to procedures in Preparation Example 31, X163-3 (500 mg, 1.06 mmol) was reacted with X203-4 (356 mg, 1.27 mg) to give intermediate X203-5; X203-5 was deprotected by hydrogen chloride-ethyl acetate to give compound X203 hydrochloride (75 mg, two-step yield: 11%) as a white solid. ¹H NMR (400 MHz, DMSO-*d*₆) δ 8.65 (s, 1H), 7.91 (d, *J* = 8.2 Hz, 1H), 7.49 (t, *J* = 5.8 Hz, 1H), 6.10 (s, 1H), 5.92 (d, *J* = 8.1 Hz, 1H), 5.88 - 5.71 (m, 2H), 5.70 - 5.54 (m, 2H), 4.42 - 4.24 (m, 2H), 3.13 - 2.98 (m, 2H), 2.92 - 2.78 (m, 2H), 2.68 - 2.45 (m, 6H), 1.79 - 1.65 (m, 2H), 1.18 - 1.01 (m, 18H). ESI-MS m/z = 617.0 [M+1]⁺.

### Preparation Example 33: Synthesis of X204 Hydrochloride

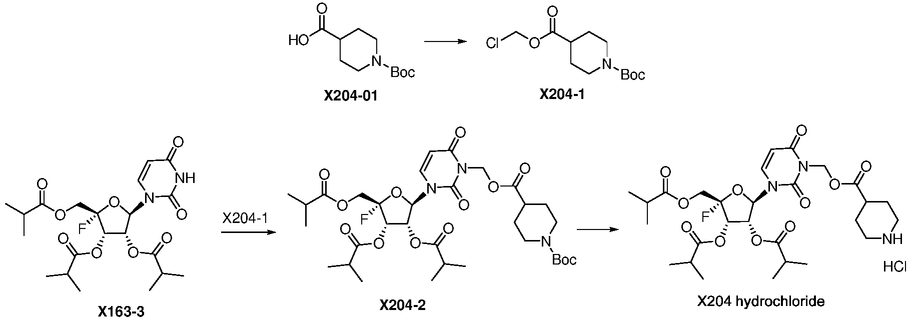

X204-01 (2.0 g, 8.72 mmol) and tetrabutylammonium hydrogen sulfate (295 mg, 0.87 mmol) were added to dichloromethane (20 mL) and water (20 mL), then solid sodium bicarbonate (2.78 g, 33.15 mmol) was added in batches, and the mixture was stirred for 10 min. Chloromethylchlorosulfonate (1.87 g, 11.34 mmol) was added dropwise in an ice-water bath. After the addition, the mixture was heated to room temperature and reacted for 3 h. After there were no starting materials remaining as detected by TLC, water and dichloromethane were added and left to stand for liquid separation. The organic phase was separated, dried over anhydrous sodium sulfate, filtered, concentrated, and purified by column chromatography to give compound X204-1 (2.40 g, yield: 99%) as an oil.

X163-3 (300 mg, 0.63 mmol), potassium carbonate (174 mg, 1.26 mmol), potassium iodide (105 mg, 0.63 mmol), and X204-1 (350 mg, 1.26 mmol) were added to acetone (5 mL), and the mixture was reacted at room temperature overnight. After there were no starting materials remaining as monitored by TLC, the reaction solution was filtered, and the filtrate was concentrated to dryness. The residue was purified by column chromatography to give compound X204-2 (260 mg, yield: 57%) as an oil.

X204-2 (200 mg, 0.28 mmol) was dissolved in 4 M hydrogen chloride-ethyl acetate (2 mL), and the mixture was reacted for 30 min under an ice water bath. After the reaction was completed as detected by TLC, the reaction solution was concentrated to dryness. Diethyl ether was added, and the mixture was stirred. The diethyl ether layer was removed, and the residue was concentrated to dryness to give compound X204 hydrochloride (130 mg, yield: 71%) as a solid. ¹H NMR (400 MHz, DMSO-*d*₆) δ 8.93 (s, 1H), 8.59 (s, 1H), 7.92 (d, *J* = 8.2 Hz, 1H), 6.12 (s, 1H), 5.93 (d, *J* = 8.1 Hz, 1H), 5.86 - 5.75 (m, 3H), 5.60 (dd, *J* = 7.3, 2.0 Hz, 1H), 4.41 - 4.27 (m, 2H), 3.26 - 3.16 (m, 2H), 2.96 - 2.83 (m, 2H), 2.77 - 2.52 (m, 4H), 2.01 - 1.90 (m, 2H), 1.80 - 1.65 (m, 2H), 1.17 - 1.04 (m, 18H). ESI-MS m/z = 614.4 [M+1]⁺.

### Preparation Example 34: Synthesis of X205 Hydrochloride

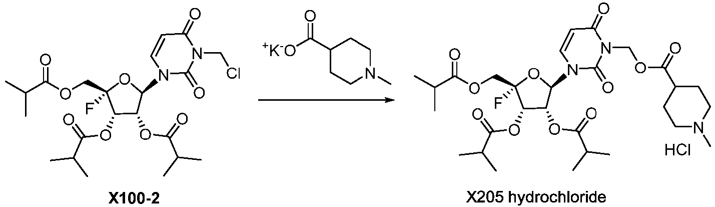

Referring to Preparation Example 24, compound X100-2 (300 mg, 0.63 mmol) was reacted with a N-methylpiperidine-4-carboxylate potassium salt (342 mg, 1.89 mmol) to give compound X205, which was treated with hydrogen chloride-ethyl acetate to give X205 hydrochloride (240 mg, yield: 63%) as a solid. ¹H NMR (400 MHz, DMSO-*d*₆) δ 9.82 (s, 1H), 7.91 (d, *J* = 8.1 Hz, 1H), 6.11 (s, 1H), 5.92 (d, *J* = 8.2 Hz, 1H), 5.86 - 5.74 (m, 3H), 5.60 (d, *J* = 7.3 Hz, 1H), 4.41 - 4.26 (m, 2H), 3.46 - 3.35 (m, 2H), 3.01 - 2.82 (m, 2H), 2.71 (s, 3H), 2.67 - 2.51 (m, 4H), 2.08 - 1.95 (m, 2H), 1.85 - 1.67 (m, 2H), 1.18 - 1.03 (m, 18H). ESI-MS m/z = 628.4 [M+1]⁺.

### Preparation Example 35: Synthesis of X206 and X206 Hydrochloride

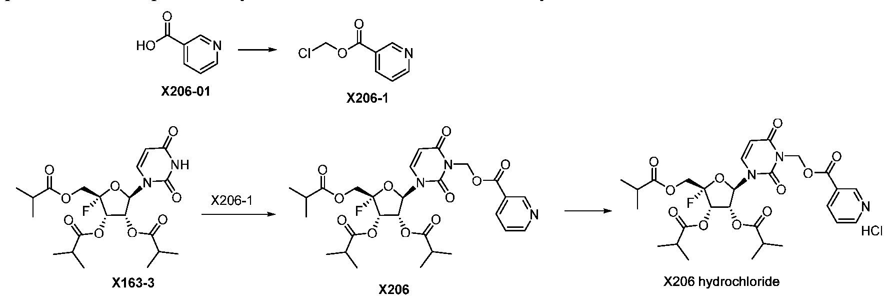

Referring to Preparation Example 33, compound X206-01 (5 g, 40.61 mmol) was reacted with chloromethyl chlorosulfonate (7.37 g, 44.67 mmol) to give compound X206-1 (crude product, 4 g), which was directly used in the next step; compound X163-3 (300 mg, 0.63 mmol) was reacted with X206-1 (218 mg, 1.27 mmol), and the reaction solution was purified by silica gel column chromatography to give X206 (104 mg, yield: 28%) as an off-white solid. ¹H NMR (X206, 400 MHz, DMSO-*d*₆) δ 9.06 (s, 1H), 8.83 (d, *J* = 4.4 Hz, 1H), 8.27 (d, *J* = 8.0 Hz, 1H), 7.94 (d, *J* = 8.2 Hz, 1H), 7.57 (dd, *J* = 8.0, 4.9 Hz, 1H), 6.14 (s, 1H), 6.11 - 6.01 (m, 2H), 5.96 (d, *J* = 8.1 Hz, 1H), 5.83 (dd, *J* = 20.1, 7.2 Hz, 1H), 5.66 (d, *J* = 7.2 Hz, 1H), 4.39 - 4.26 (m, 2H), 2.68 - 2.53 (m, 2H), 2.49 - 2.40 (m, 1H), 1.16 - 1.08 (m, 12H), 1.02 (d, *J* = 7.0 Hz, 3H), 0.98 (d, *J* = 7.0 Hz, 3H).

X206 (104 mg, 0.17 mmol) was added to 4 M hydrogen chloride-ethyl acetate (1 mL), and the mixture was concentrated to dryness to give compound X206 hydrochloride (110 mg). ¹H NMR (X206 hydrochloride, 400 MHz, DMSO-*d*₆) δ 9.07 (d, *J* = 2.2 Hz, 1H), 8.84 (dd, *J* = 4.9, 1.7 Hz, 1H), 8.32 - 8.27 (m, 1H), 7.94 (d, *J* = 8.2 Hz, 1H), 7.59 (dd, *J* = 8.0, 4.8 Hz, 1H), 6.13 (d, *J* = 2.1 Hz, 1H), 6.10 - 6.01 (m, 2H), 5.96 (d, *J* = 8.1 Hz, 1H), 5.82 (dd, *J* = 20.1, 7.2 Hz, 1H), 5.65 (dd, *J* = 7.3, 2.0 Hz, 1H), 4.38 - 4.26 (m, 2H), 2.68 - 2.54 (m, 2H), 2.48 - 2.40 (m, 1H), 1.14 - 1.08 (m, 12H), 1.02 (d, *J* = 7.0 Hz, 3H), 0.98 (d, *J* = 7.0 Hz, 3H). ESI-MS m/z = 608.3 [M+1]⁺.

### Preparation Example 36: Synthesis of X207

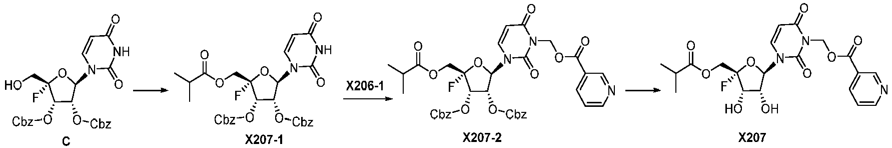

Referring to procedures in Preparation Example 18, C (500 mg, 0.94 mmol) was reacted with isobutyryl chloride (120 mg, 1.13 mmol) to give intermediate X207-1; X207-1 was reacted with X206-1 to give intermediate X207-2; X207-2 was deprotected by hydrogen/palladium on carbon to give compound X207 (65 mg, three-step yield: 15%). ¹H NMR (400 MHz, DMSO-*d*₆) δ 9.06 (d, *J* = 1.9 Hz, 1H), 8.83 (dd, *J* = 4.8, 1.7 Hz, 1H), 8.30 - 8.25 (m, 1H), 7.81 (d, *J* = 8.2 Hz, 1H), 7.57 (dd, *J* = 8.0, 4.8 Hz, 1H), 6.10 - 6.01 (m, 2H), 5.97 (d, *J* = 2.2 Hz, 1H), 5.90 (d, *J* = 8.2 Hz, 1H), 5.76 (d, *J* = 4.8 Hz, 1H), 5.35 (d, *J* = 8.7 Hz, 1H), 4.44 - 4.18 (m, 4H), 2.59 - 2.50 (m, 1H), 1.10 - 1.01 (m, 6H). ESI-MS m/z = 468.2 [M+1]⁺.

### Preparation Example 37: Synthesis of X208

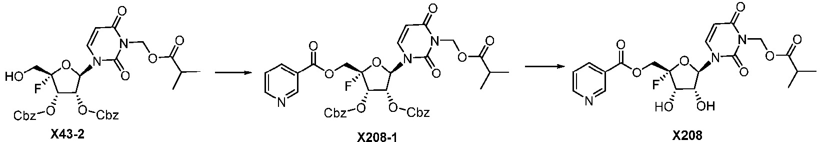

To a 50 mL reaction flask were sequentially added X43-2 (800 mg, 1.27 mmol), nicotinic acid (237 mg, 1.91 mmol), O-(7-azabenzotriazol-1-yl)-*N,N,N',N'*-tetramethyluronium hexafluorophosphate (HATU) (966 mg, 2.54 mmol), *N*,*N*-diisopropylethylamine (DIEA) (492 mg, 2.54 mmol), DMAP (155 mg, 1.27 mmol), and dichloromethane (16 mL), and the mixture was reacted at room temperature for 1 h under nitrogen atmosphere. After the reaction was completed as detected by TLC, the reaction solution was concentrated, and the residue was purified by silica gel column chromatography to give compound X208-1 (730 mg, yield: 78%) as an oil.

To a 25 mL reaction flask were sequentially added X208-1 (200 mg), 10% palladium on carbon (200 mg), and tetrahydrofuran (4 mL), and the mixture was purged with hydrogen three times and reacted for 3 h under normal pressure. After the reaction was completed as detected by TLC, the reaction solution was filtered, and the filtrate was concentrated. The residue was purified by silica gel column chromatography to give compound X208 (100 mg, yield: 79%) as a white solid. ¹H NMR (400 MHz, DMSO-*d*₆) δ 9.14 (d, *J* = 2.2 Hz, 1H), 8.85 (dd, *J* = 4.6Hz, 1.5 Hz, 1H), 8.34 (d, *J* = 7.9 Hz, 1H), 7.84 (d, *J* = 8.2 Hz, 1H), 7.59 (dd, *J* = 7.9, 4.9 Hz, 1H), 5.98 (d, J = 2.3 Hz, 1H), 5.81 - 5.75 (m, 2H), 5.72 (s, 2H), 5.42 (d, *J* = 9.0 Hz, 1H), 4.69 - 4.47 (m, 3H), 4.38 - 4.28 (m, 1H), 2.50 - 2.43 (m, 1H), 1.03 (d, *J* = 7.0 Hz, 6H). ESI-MS m/z = 468.2 [M+1]⁺.

### Preparation Example 38: Synthesis of X209

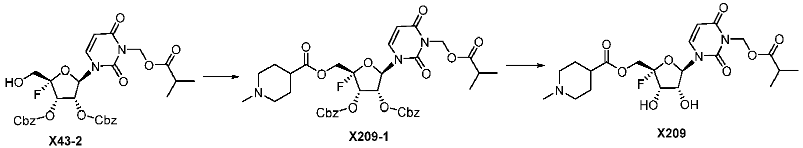

Referring to procedures in Preparation Example 37, compound X43-2 (750 mg, 1.19 mmol) was reacted with *N*-methylpiperidine-4-carboxylic acid (340 mg, 2.38 mmol) to give intermediate X209-1; X209-1 was deprotected by hydrogen/palladium on carbon, and the obtained product was purified by preparative liquid phase chromatography to give compound X209 (40 mg, two-step yield: 7%). ¹H NMR (400 MHz, Chloroform-*d*) δ 7.31 (d, *J* = 8.2 Hz, 1H), 5.92 (s, 2H), 5.82 (d, *J* = 8.2 Hz, 1H), 5.76 (s, 1H), 4.59 - 4.48 (m, 1H), 4.48 - 4.35 (m, 3H), 2.99 - 2.88 (m, 2H), 2.61 - 2.49 (m, 2H), 2.44 - 2.26 (m, 5H), 2.18 - 2.04 (m, 2H), 1.92 - 1.77 (m, 2H), 1.16 (d, *J* = 7.0 Hz, 6H). ESI-MS m/z = 488.3 [M+1]⁺.

### Preparation Example 39: Preparation of X210

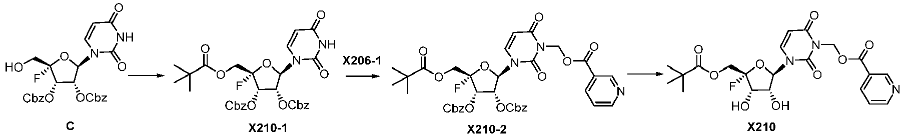

Referring to procedures in Preparation Example 18, C (800 mg, 1.51 mmol) was reacted with pivaloyl chloride (218 mg, 1.81 mmol) to give intermediate X210-1; X210-1 was reacted with X206-1 to give intermediate X210-2; X210-2 was deprotected by hydrogen/palladium on carbon to give compound X210 (220 mg, three-step yield: 30%) as an off-white solid. ¹H NMR (400 MHz, DMSO-*d*₆) δ 9.06 (d, *J* = 2.2 Hz, 1H), 8.83 (dd, *J* = 4.8, 1.6 Hz, 1H), 8.30 - 8.24 (m, 1H), 7.82 (d, *J* = 8.2 Hz, 1H), 7.56 (dd, *J* = 8.0, 4.8 Hz, 1H), 6.10 - 6.00 (m, 2H), 5.95 (s, 1H), 5.89 (d, *J* = 8.2 Hz, 1H), 5.76 (d, *J* = 4.7 Hz, 1H), 5.34 (d, *J* = 8.6 Hz, 1H), 4.43 - 4.27 (m, 3H), 4.24 - 4.16 (m, 1H), 1.11 (s, 9H). ESI-MS m/z = 482.2 [M+1]⁺.

### Preparation Example 40: Preparation of X211

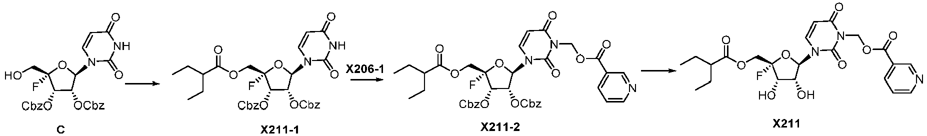

Referring to procedures in Preparation Example 18, C (800 mg, 1.51 mmol) was reacted with 2-ethylbutyrylchloride (244 mg, 1.81 mmol) to give intermediate X211-1; X211-1 was reacted with X206-1 to give intermediate X211-2; X211-2 was deprotected by hydrogen/palladium on carbon to give compound X211 (150 mg, three-step yield: 20%) as a solid. ¹H NMR (400 MHz, DMSO-*d*₆) δ 9.06 (d, *J* = 2.3 Hz, 1H), 8.83 (dd, *J* = 4.9, 1.7 Hz, 1H), 8.30 - 8.24 (m, 1H), 7.82 (d, *J =* 8.2 Hz, 1H), 7.57 (dd, *J =* 7.9, 4.8 Hz, 1H), 6.11 - 6.01 (m, 2H), 5.97 (s, 1H), 5.91 (d, *J* = 8.2 Hz, 1H), 5.77 (d, *J* = 4.7 Hz, 1H), 5.37 (d, *J* = 8.4 Hz, 1H), 4.42 - 4.29 (m, 3H), 4.29 - 4.20 (m, 1H), 2.27 - 2.14 (m, 1H), 1.58 - 1.36 (m, 4H), 0.83 - 0.74 (m, 6H). ESI-MS m/z = 496.2 [M+1]⁺.

### Preparation Example 41: Preparation of X214

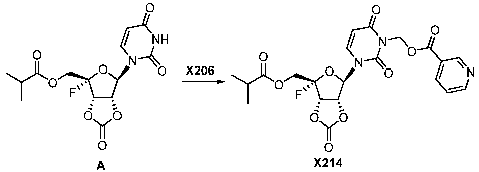

Referring to procedures in Preparation Example 3, A (400 mg, 1.12 mmol) was reacted with X206-1 (384 mg, 2.24 mmol) to give compound X214 (340 mg, yield: 62%) as an off-white solid. ¹H NMR (400 MHz, DMSO-*d*₆) δ 9.07 (d, *J* = 2.2 Hz, 1H), 8.84 (dd, *J* = 4.8, 1.7 Hz, 1H), 8.31 - 8.24 (m, 1H), 7.95 (d, *J* = 8.1 Hz, 1H), 7.57 (dd, *J* = 8.0, 4.9 Hz, 1H), 6.44 (s, 1H), 6.06 - 5.95 (m, 3H), 5.83 (d, *J* = 7.4 Hz, 1H), 5.68 (dd, *J* = 12.0, 7.4 Hz, 1H), 4.50 - 4.32 (m, 2H), 2.64 - 2.51 (m, 1H), 1.10 - 1.03 (m, 6H). ESI-MS m/z = 494.2 [M+1]⁺.

### Preparation Example 42: Preparation of X222

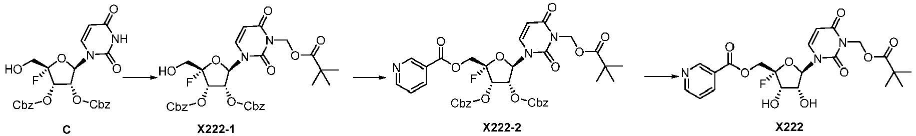

C (1 g, 1.89 mmol), potassium carbonate (261 mg, 1.89 mmol), and sodium iodide (283 mg, 1.89 mmol) were dissolved in acetone (10 mL), then chloromethyl pivalate (427 mg, 2.84 mmol) was added dropwise, and the mixture was reacted at room temperature overnight. After the reaction was completed as detected by TLC, the reaction solution was filtered, and the filtrate was concentrated. The residue was purified by silica gel column chromatography to give intermediate X222-1 (900 mg, yield: 74%) as an oil.

Referring to procedures in Preparation Example 37, X222-1 (800 mg, 1.24 mmol) was reacted with nicotinic acid (229 mg, 1.86 mmol) to give intermediate X222-2; X222-2 was deprotected by hydrogen/palladium on carbon to give compound X222 (50 mg, two-step yield: 8%) as an off-white solid. ¹H NMR (400 MHz, DMSO-*d*₆) δ 9.14 (d, *J* = 2.0 Hz, 1H), 8.85 (d, *J* = 4.6 Hz, 1H), 8.34 (d, *J* = 7.6 Hz, 1H), 7.85 (d, *J* = 8.1 Hz, 1H), 7.60 (dd, *J* = 7.9, 4.8 Hz, 1H), 5.98 (d, *J* = 1.6 Hz, 1H), 5.83 - 5.75 (m, 2H), 5.72 (s, 2H), 5.44 (d, *J* = 9.0 Hz, 1H), 4.67 - 4.48 (m, 3H), 4.38 - 4.30 (m, 1H), 1.08 (s, 9H). ESI-MS m/z = 482.2 [M+1]⁺.

### Preparation Example 43: Preparation of X223

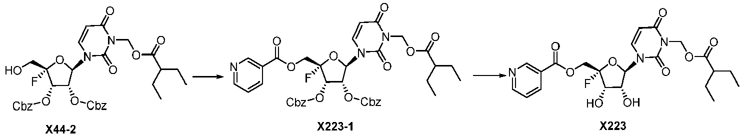

Referring to procedures in Preparation Example 37, X44-2 (800 mg, 1.21 mmol) was reacted with nicotinic acid (224 mg, 1.82 mmol) to give intermediate X223-1; X223-1 was deprotected by hydrogen/palladium on carbon to give compound X223 (70 mg, two-step yield: 12%) as an off-white solid. ¹H NMR (400 MHz, DMSO-*d*₆) δ 9.14 (d, *J* = 2.2 Hz, 1H), 8.85 (dd, *J* = 4.8, 1.7 Hz, 1H), 8.38 - 8.31 (m, 1H), 7.85 (d, *J* = 8.1 Hz, 1H), 7.60 (dd, *J* = 7.9, 4.9 Hz, 1H), 5.97 (d, *J* = 2.4 Hz, 1H), 5.84 - 5.71 (m, 4H), 5.44 (d, *J* = 9.1 Hz, 1H), 4.66 - 4.47 (m, 3H), 4.37 - 4.31 (m, 1H), 2.20 - 2.10 (m, 1H), 1.55 - 1.37 (m, 4H), 0.79 (t, *J* = 7.4 Hz, 6H). ESI-MS m/z = 496.3 [M+1]⁺.

### Preparation Example 44: Synthesis of X231

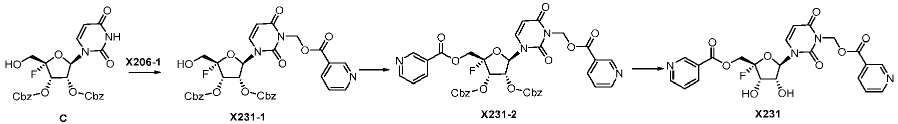

Referring to procedures in Preparation Examples 37 and 42, C (1 g, 1.89 mmol) was reacted with X206-1 (486 mg, 2.83 mmol) to give intermediate X231-1; X231-1 was reacted with nicotinic acid to give intermediate X231-2; X231-2 was deprotected by hydrogen/palladium on carbon to give compound X231 (40 mg, total three-step yield: 4%) as an off-white solid. ¹H NMR (400 MHz, DMSO-*d*₆) δ 9.13 (s, 1H), 9.02 (s, 1H), 8.81 (d, *J* = 4.9 Hz, 1H), 8.76 (d, *J* = 4.9 Hz, 1H), 8.33 (d, *J* = 8.0 Hz, 1H), 8.22 (d, *J* = 8.1 Hz, 1H), 7.90 (d, *J* = 8.1 Hz, 1H), 7.59 - 7.49 (m, 2H), 6.08 - 5.96 (m, 3H), 5.85 - 5.78 (m, 2H), 5.45 (d, *J* = 9.0 Hz, 1H), 4.70 - 4.46 (m, 3H), 4.43 - 4.36 (m, 1H). ESI-MS m/z = 503.2 [M+1]⁺.

### Preparation Example 45: Synthesis of X232

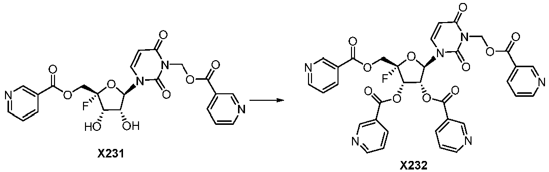

X231 (50 mg, 0.10 mmol), nicotinic acid (37 mg, 0.25 mmol), *N*,*N*-diisopropylethylamine (52 mg, 0.40 mmol), and *p*-dimethylaminopyridine (12 mg, 0.10 mmol) were dissolved in tetrahydrofuran (2 mL), then O-(7-azabenzotriazol-1-yl)-*N,N,N',N'*-tetramethyluronium hexafluorophosphate (HATU) (114 mg, 0.30 mmol) was added in batches, and the mixture was reacted at room temperature for 1-2 h. After the reaction was completed as detected by TLC, the reaction solution was concentrated to dryness, and the residue was purified by silica gel column chromatography to give compound X232 (45 mg, yield: 63%) as an off-white solid. ¹H NMR (400 MHz, DMSO-*d*₆) δ 9.08 (d, *J* = 2.2 Hz, 1H), 9.05 (d, *J* = 2.2 Hz, 1H), 9.00 (d, *J* = 2.2 Hz, 1H), 8.91 (d, *J* = 2.3 Hz, 1H), 8.85 (dd, *J* = 4.9, 1.7 Hz, 1H), 8.83 - 8.75 (m, 2H), 8.63 (dd, *J* = 4.9, 1.7 Hz, 1H), 8.32 - 8.10 (m, 4H), 8.00 (d, *J* = 8.2 Hz, 1H), 7.60 (dd, *J* = 8.0, 4.9 Hz, 1H), 7.56 - 7.44 (m, 2H), 7.40 (dd, *J* = 8.0, 4.8 Hz, 1H), 6.58 (s, 1H), 6.51 (dd, *J* = 20.1, 7.2 Hz, 1H), 6.17 (dd, *J* = 7.1, 1.8 Hz, 1H), 6.10 - 6.03 (m, 2H), 6.00 (d, *J* = 8.1 Hz, 1H), 4.87 - 4.69(m, 2H). ESI-MS m/z = 713.2 [M+1]⁺.

### Preparation Example 46: Synthesis of X233

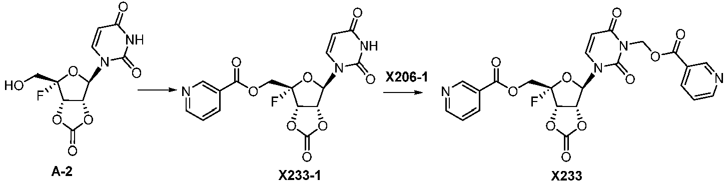

A-2 (400 mg, 1.39 mmol), nicotinic acid (257 mg, 2.09 mmol), *N*,*N*-diisopropylethylamine (539 mg, 4.17 mmol), and *p*-dimethylaminopyridine (170 mg, 1.39 mmol) were dissolved in dichloromethane (4 mL), then O-(7-azabenzotriazol-1-yl)-*N,N,N',N*'-tetramethyluronium hexafluorophosphate (HATU) (1.06 g, 2.78 mmol) was added in batches, and the mixture was reacted at room temperature for 1-2 h. After the reaction was completed as detected by TLC, the reaction solution was concentrated to dryness, and the residue was purified by silica gel column chromatography to give compound X233-1 (380 mg, yield: 70%) as an off-white solid. Referring to procedures in Preparation Example 3, X233-1 (380 mg, 0.97 mmol) was reacted with X206-1 (333 mg, 1.94 mmol) to give compound X233 (120 mg, yield: 23%) as an off-white solid. ¹H NMR (400 MHz, DMSO-*d*₆) δ 9.13 (d, *J* = 1.4 Hz, 1H), 9.02 (d, *J* = 1.4 Hz, 1H), 8.81 (dd, *J* = 4.9, 1.7 Hz, 1H), 8.76 (dd, *J* = 4.8, 1.7 Hz, 1H), 8.35 - 8.28 (m, 1H), 8.24 - 8.19 (m, 1H), 7.98 (d, *J* = 8.1 Hz, 1H), 7.57 - 7.50 (m, 2H), 6.50 (s, 1H), 6.01 - 5.86 (m, 5H), 4.76 - 4.60 (m, 2H). ESI-MS m/z = 529.2 [M+1]⁺.

### Preparation Example 47: Synthesis of X260 Hydrochloride

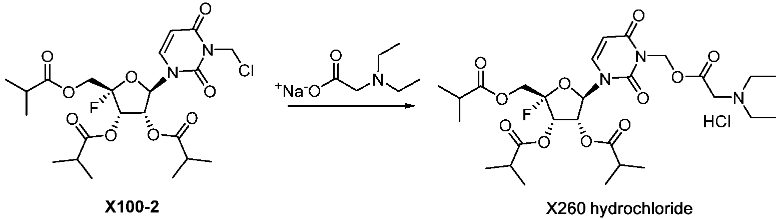

Referring to Preparation Example 24, X100-2 (100 mg, 0.19 mmol) was reacted with a *N*,*N*-diethylglycine sodium salt (44 mg, 0.29 mmol) to give compound X260, which was treated with hydrogen chloride-ethyl acetate to give X260 hydrochloride (60 mg, yield: 48%) as an off-white solid. ¹H NMR (400 MHz, DMSO-*d*₆) δ 10.09 (s, 1H), 7.95 (d, *J* = 8.2 Hz, 1H), 6.14 (d, *J* = 2.0 Hz, 1H), 6.00 - 5.90 (m, 3H), 5.80 (dd, *J* = 20.1, 7.2 Hz, 1H), 5.61 (dd, *J* = 7.3, 2.0 Hz, 1H), 4.43 - 4.15 (m, 4H), 3.26 - 3.10 (m, 4H), 2.66 - 2.52 (m, 3H), 1.20 (t, *J* = 7.2 Hz, 6H), 1.15 - 1.05 (m, 18H). ESI-MS m/z = 616.3 [M+1]⁺.

### Example 48: Synthesis of X219

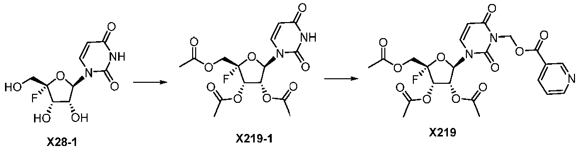

X28-1 (500 mg, 1.91 mmol), p-dimethylaminopyridine (47 mg, 0.38 mmol), and triethylamine (966 mg, 9.55 mmol) were dissolved in tetrahydrofuran (10 mL), then acetic anhydride (780 mg, 7.64 mmol) was added dropwise, and the mixture was reacted at room temperature for 2 h. After the reaction was completed as detected by TLC, the reaction solution was concentrated to dryness. Water was added, and the mixture was stirred to precipitate a solid, and filtered. The solid was dried at 60 °C to give intermediate X219-1 (550 mg, yield: 74%) as an off-white solid. ¹H NMR (400 MHz, DMSO-*d*₆) δ 11.60 (s, 1H), 7.76 (d, *J* = 8.0 Hz, 1H), 6.06 (d, *J* = 2.3 Hz, 1H), 5.79 (dd, *J* = 20.2, 7.5 Hz, 1H), 5.70 (d, *J* = 8.0 Hz, 1H), 5.55 (dd, *J* = 7.4, 2.2 Hz, 1H), 4.48 - 4.38 (m, 1H), 4.30 - 4.20 (m, 1H), 2.10 (s, 3H), 2.09 (s, 3H), 2.04 (s, 3H).

X219-1 (550 mg, 1.42 mmol), X206-1 (487 mg, 2.84 mmol), cesium carbonate (925 mg, 2.84 mmol), and sodium iodide (213 mg, 1.42 mmol) were added to *N*,*N*-dimethylformamide (11 mL), and the mixture was stirred at 70 °C for 2 h under nitrogen atmosphere. After the starting materials were completely reacted as detected by TLC, water and ethyl acetate were added to the reaction solution, and the mixture was left to stand for liquid separation. The organic phase was separated, washed with water, dried over anhydrous sodium sulfate, filtered, concentrated, and purified by silica gel column chromatography to give compound X219 (560 mg, yield: 75%) as a foamy solid. ¹H NMR (400 MHz, DMSO-*d*₆) δ 9.06 (d, *J* = 2.2 Hz, 1H), 8.83 (dd, *J* = 4.9, 1.7 Hz, 1H), 8.27 (dt, *J* = 8.1, 2.0 Hz, 1H), 7.91 (d, *J* = 8.2 Hz, 1H), 7.57 (dd, *J* = 8.0, 4.9 Hz, 1H), 6.16 (d, *J* = 2.1 Hz, 1H), 6.11 - 6.01 (m, 2H), 5.96 (d, *J* = 8.1 Hz, 1H), 5.80 (dd, *J* = 20.2, 7.4 Hz, 1H), 5.62 (dd, *J* = 7.3, 2.1 Hz, 1H), 4.48 - 4.39 (m, 1H), 4.24 (dd, *J* = 12.0, 10.0 Hz, 1H), 2.11 (s, 3H), 2.09 (s, 3H), 1.95 (s, 3H). ESI-MS m/z = 524.2 [M+1]⁺.

### Example 49: Synthesis of X220

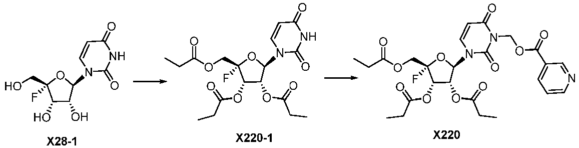

Referring to procedures in Preparation Example 48, X28-1 (200 mg, 0.76 mmol) was reacted with propionic anhydride (396 mg, 3.04 mmol) to give intermediate X220-1; X220-1 was reacted with X206-1 to give compound X220 (105 mg, two-step yield: 24%) as a foam. ¹H NMR (400 MHz, DMSO-*d*₆) δ 9.07 (d, *J* = 2.3, 0.9 Hz, 1H), 8.84 (dd, *J* = 4.8, 1.7 Hz, 1H), 8.28 (dt, *J* = 8.0, 2.0 Hz, 1H), 7.93 (d, *J* = 8.1 Hz, 1H), 7.62 - 7.54 (m, 1H), 6.16 (d, *J* = 2.1 Hz, 1H), 6.12 - 6.01 (m, 2H), 5.97 (d, *J* = 8.1 Hz, 1H), 5.83 (dd, *J* = 20.2, 7.4 Hz, 1H), 5.65 (dd, *J* = 7.3, 2.1 Hz, 1H), 4.48 - 4.36 (m, 1H), 4.28 (dd, *J* = 12.3, 9.2 Hz, 1H), 2.47 - 2.16 (m, 6H), 1.05 (t, *J* = 7.5 Hz, 6H), 0.94 (t, *J* = 7.5 Hz, 3H).ESI-MS m/z = 566.2 [M+1]⁺.

### Example 50: Synthesis of X257

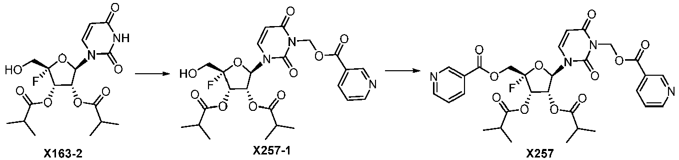

Referring to procedures in Preparation Example 44, X163-2 (300 mg, 0.75 mmol) was reacted with X206-1 (257 mg, 1.50 mmol) to give intermediate X257-1; X257-1 was reacted with nicotinic acid to give compound X257 (55 mg, two-step yield: 11%) as a foam. ¹H NMR (400 MHz, DMSO-*d*₆) δ 9.11 (d, *J* = 2.2 Hz, 1H), 8.98 (d, *J* = 2.2 Hz, 1H), 8.79 (dd, *J* = 4.9, 1.7 Hz, 1H), 8.69 (dd, *J* = 4.9, 1.7 Hz, 1H), 8.35 - 8.27 (m, 1H), 8.22 - 8.14 (m, 1H), 7.97 (d, *J* = 8.2 Hz, 1H), 7.55 - 7.45 (m, 2H), 6.19 (s, 1H), 6.14 - 5.91 (m, 4H), 5.71 (dd, *J* = 7.2, 2.0 Hz, 1H), 4.68 (dd, *J* = 12.1, 8.1 Hz, 1H), 4.55 (dd, *J* = 12.2, 6.4 Hz, 1H), 2.68 - 2.52 (m, 2H), 1.16 - 1.09 (m, 6H), 1.09 - 1.01 (m, 6H). ESI-MS m/z = 643.3 [M+1]⁺.

### Example 51: Synthesis of X265

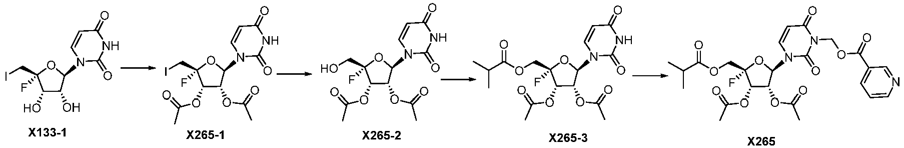

Referring to procedures in Preparation Example 7 and Example 48, X133-1 (1 g, 2.69 mmol) was reacted with acetic anhydride (824 mg, 8.07 mmol) to give intermediate X265-1; X265-1 was reacted with *m*-chloroperoxybenzoic acid to give X265-2; X265-2 was reacted with isobutyric anhydride to give X265-3; X265-3 was reacted with X206-1 to give compound X265 (110 mg, four-step yield: 10%) as a foam. ¹H NMR (400 MHz, DMSO-*d*₆) δ 9.06 (d, *J* = 2.2 Hz, 1H), 8.83 (dd, *J* = 4.8, 1.7 Hz, 1H), 8.27 (dt, *J* = 8.1, 2.0 Hz, 1H), 7.92 (d, *J* = 8.2 Hz, 1H), 7.57 (dd, *J* = 7.9, 5.0 Hz, 1H), 6.16 (d, *J* = 2.1 Hz, 1H), 6.11 - 5.99 (m, 2H), 5.96 (d, *J* = 8.1 Hz, 1H), 5.83 (dd, *J* = 20.4, 7.3 Hz, 1H), 5.65 (dd, *J* = 7.3, 2.1 Hz, 1H), 4.41 - 4.25 (m, 2H), 2.49 - 2.38 (m, 1H), 2.11 (s, 3H), 2.08 (s, 3H), 1.01 (d, *J* = 7.0 Hz, 3H), 0.97 (d, *J* = 7.0 Hz, 3H). ESI-MS m/z = 552.3 [M+1]⁺.

### Example 52: Synthesis of X266 Hydrochloride

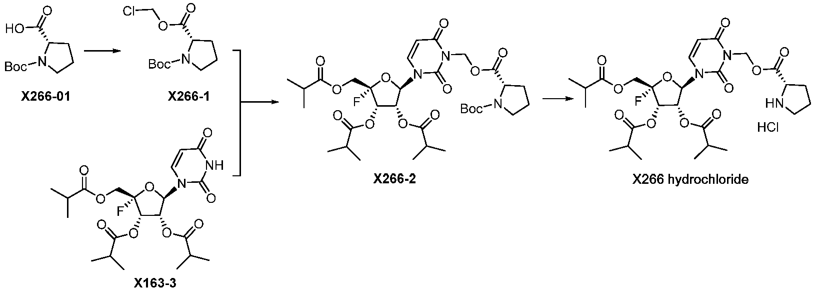

Referring to procedures in Preparation Example 33, X266-01 (5 g, 23.23 mmol) was reacted with chloromethyl chlorosulfonate (4.95 g, 30.20 mmol) to give intermediate X266-1 (4.7 g, yield: 77%) as an oil; X163-3 (300 mg, 0.63 mmol) was reacted with X266-1 (332 mg, 1.26 mmol) to give X266-2; X266-2 was reacted with a hydrogen chloride-ethyl acetate solution to give compound X266 hydrochloride (145 mg, two-step yield: 36%) as a solid. ¹H NMR (400 MHz, DMSO-*d*₆) δ 7.93 (d, *J =* 8.2 Hz, 1H), 6.14 (d, *J =* 2.0 Hz, 1H), 6.01 - 5.87 (m, 3H), 5.80 (dd, *J =* 20.2, 7.2 Hz, 1H), 5.60 (dd, *J =* 7.2, 2.0 Hz, 1H), 4.46 - 4.25 (m, 3H), 3.27 - 3.12 (m, 2H), 2.69 - 2.53 (m, 3H), 2.31 - 2.18 (m, 1H), 2.03 - 1.84 (m, 3H), 1.22 - 1.00 (m, 18H).ESI-MS m/z = 600.3 [M+1]⁺.

### Example 53: Synthesis of X267 Hydrochloride

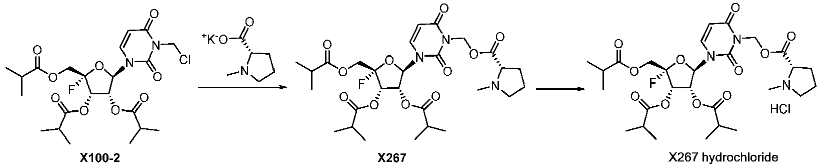

Referring to Preparation Example 24, compound X100-2 (300 mg, 0.63 mmol) was reacted with a N-methyl-L-proline potassium salt (211 mg, 1.26 mmol) to give compound X267, which was treated with hydrogen chloride-ethyl acetate to give compound X267 hydrochloride (150 mg, yield: 37%) as a solid. ¹H NMR (400 MHz, DMSO-*d*₆) δ 10.47 (s, 1H), 7.94 (d, *J =* 8.2 Hz, 1H), 6.14 (d, *J* = 2.1 Hz, 1H), 6.01 - 5.87 (m, 3H), 5.81 (dd, *J* = 20.1, 7.2 Hz, 1H), 5.61 (dd, *J* = 7.2, 2.0 Hz, 1H), 4.44 - 4.25 (m, 3H), 3.70 - 3.48 (m, 1H), 3.22 - 3.02 (m, 1H), 2.87 (s, 3H), 2.67 - 2.52 (m, 3H), 2.47 - 2.33 (m, 1H), 2.09 - 1.85 (m, 3H), 1.19 - 1.00 (m, 18H). ESI-MS m/z = 614.3 [M+1]⁺.

### Example 54: Synthesis of X206 Hydrobromide, X206 Methanesulfonate and X206 Sulfate

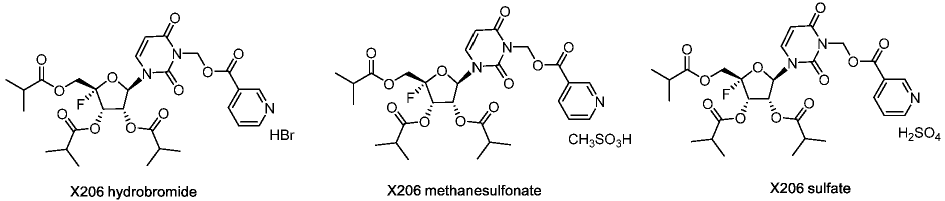

A solution of X206 (100 mg, 0.17 mmol) and 48% hydrobromic acid (29 mg, 0.17 mmol) in water were added to acetonitrile (1 mL), and the mixture was concentrated to dryness to give compound X206 hydrobromide (115 mg). ¹H NMR (400 MHz, DMSO-*d*₆) δ 9.08 (d, *J* = 2.3 Hz, 1H), 8.85 (dd, *J =* 4.9, 1.7 Hz, 1H), 8.31 (dt, *J =* 8.1, 2.0 Hz, 1H), 7.95 (d, *J* = 8.2 Hz, 1H), 7.61 (dd, *J* = 8.0, 4.9 Hz, 1H), 6.14 (d, *J* = 2.0 Hz, 1H), 6.11 - 6.00 (m, 2H), 5.97 (d, *J* = 8.1 Hz, 1H), 5.83 (dd, *J* = 20.3, 7.2 Hz, 1H), 5.65 (dd, *J* = 7.2, 2.0 Hz, 1H), 4.39 - 4.24 (m, 2H), 2.66 - 2.53 (m, 2H), 2.48 - 2.39 (m, 1H), 1.16 - 1.06 (m, 12H), 1.01 (d, *J* = 7.0 Hz, 3H), 0.98 (d, *J* = 7.0 Hz, 3H).

X206 (100 mg, 0.17 mmol) and methanesulfonic acid (16 mg, 0.17 mmol) were added to acetonitrile (1 mL), and the mixture was concentrated to dryness to give compound X206 methanesulfonate (117 mg). ¹H NMR (400 MHz, DMSO-*d*₆) δ 9.11 (dd, *J* = 2.2, 0.9 Hz, 1H), 8.88 (dd, *J* = 5.0, 1.7 Hz, 1H), 8.37 (dt, *J* = 8.1, 1.9 Hz, 1H), 7.95 (d, *J* = 8.2 Hz, 1H), 7.69 - 7.63 (m, 1H), 6.14 (d, *J* = 2.0 Hz, 1H), 6.12 - 6.00 (m, 2H), 5.97 (d, *J* = 8.1 Hz, 1H), 5.83 (dd, *J =* 20.2, 7.2 Hz, 1H), 5.65 (dd, *J =* 7.2, 2.0 Hz, 1H), 4.39 - 4.24 (m, 2H), 2.66 - 2.53 (m, 2H), 2.48 - 2.38 (m, 1H), 2.36 (s, 3H), 1.16 - 1.07 (m, 12H), 1.02 (d, *J* = 7.0 Hz, 3H), 0.98 (d, *J* = 7.0 Hz, 3H).

X206 (100 mg, 0.17 mmol) and sulfuric acid (17 mg, 0.17 mmol) were added to acetonitrile (1 mL), and the mixture was concentrated to dryness to give compound X206 sulfate (113 mg). ¹H NMR (400 MHz, DMSO-*d*₆) δ 9.12 (dd, *J* = 2.2, 0.8 Hz, 1H), 8.88 (dd, *J* = 4.9, 1.7 Hz, 1H), 8.39 (dt, *J* = 8.0, 1.9 Hz, 1H), 7.95 (d, *J* = 8.2 Hz, 1H), 7.71 - 7.64 (m, 1H), 6.14 (d, *J* = 2.0 Hz, 1H), 6.12 - 6.02 (m, 2H), 5.97 (d, *J* = 8.2 Hz, 1H), 5.83 (dd, *J* = 20.3, 7.2 Hz, 1H), 5.65 (dd, *J =* 7.2, 2.0 Hz, 1H), 4.39 - 4.24 (m, 2H), 2.69 - 2.52 (m, 2H), 2.49 - 2.39 (m, 1H), 1.19 - 1.07 (m, 12H), 1.02 (d, *J =* 7.0 Hz, 3H), 0.98 (d, *J =* 7.0 Hz, 3H).

### Example 55: Synthesis of X219 Hydrochloride, X219 Hydrobromide, X219 Methanesulfonate, X219 Hemisulfate, and X219 Sulfate

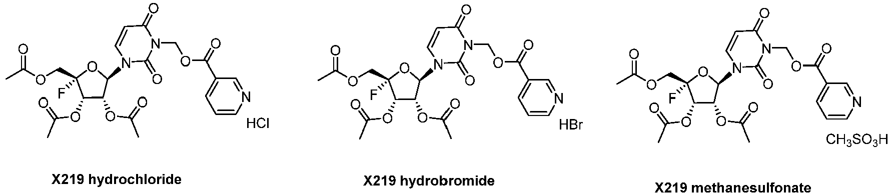

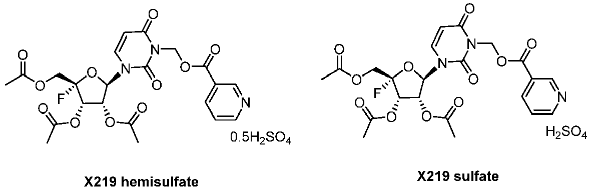

X219 (100 mg, 0.19 mmol) was dissolved in tetrahydrofuran (1 mL), then 37% concentrated hydrochloric acid (19 mg, 0.19 mmol) was added, and the mixture was stirred for 10 min. Isopropyl ether (1 mL) was added, and the mixture was filtered and dried at 60 °C to give compound X219 hydrochloride (95 mg, yield: 90%). ¹H NMR (400 MHz, DMSO-*d*₆) δ 9.10 (d, *J =* 2.2 Hz, 1H), 8.88 (dd, *J =* 5.1, 1.7 Hz, 1H), 8.41 - 8.33 (m, 1H), 7.93 (d, *J =* 8.2 Hz, 1H), 7.66 (dd, *J =* 8.0, 5.0 Hz, 1H), 6.17 (d, *J =* 2.2 Hz, 1H), 6.12 - 6.02 (m, 2H), 5.96 (d, *J =* 8.1 Hz, 1H), 5.80 (dd, *J =* 20.2, 7.4 Hz, 1H), 5.62 (dd, *J =* 7.3, 2.1 Hz, 1H), 4.44 (dd, *J =* 12.2, 10.3 Hz, 1H), 4.25 (dd, *J =* 12.2, 9.8 Hz, 1H), 2.11 (s, 3H), 2.09 (s, 3H), 1.96 (s, 3H).

X219 (100 mg, 0.19 mmol) was dissolved in tetrahydrofuran (1 mL), then a 48% aqueous hydrogen bromide solution (19 mg, 0.19 mmol) was added, and the mixture was stirred for 10 min. Isopropyl ether (1 mL) was added, and the mixture was filtered and dried at 60 °C to give compound X219 hydrobromide (105 mg, yield: 91%). ¹H NMR (400 MHz, DMSO-*d*₆) δ 9.13 (d, *J =* 2.2, 1H), 8.90 (dd, *J =* 5.0, 1.6 Hz, 1H), 8.43 (dt, *J =* 8.1, 1.9 Hz, 1H), 7.93 (d, *J =* 8.2 Hz, 1H), 7.75 - 7.67 (m, 1H), 6.17 (d, *J =* 2.1 Hz, 1H), 6.13 - 6.02 (m, 2H), 5.97 (d, *J =* 8.1 Hz, 1H), 5.80 (dd, *J =* 20.2, 7.4 Hz, 1H), 5.62 (dd, *J =* 7.4, 2.1 Hz, 1H), 4.44 (dd, *J =* 12.2, 10.4 Hz, 1H), 4.25 (dd, *J* = 12.3, 9.9 Hz, 1H), 2.11 (s, 3H), 2.09 (s, 3H), 1.96 (s, 3H).

X219 (100 mg, 0.19 mmol) was dissolved in tetrahydrofuran (1 mL), then methanesulfonic acid (18 mg, 0.19 mmol) was added, and the mixture was stirred for 10 min. Isopropyl ether (1 mL) was added, and the mixture was filtered and dried at 45 °C to give compound X219 methanesulfonate (80 mg, yield: 68%). ¹H NMR (400 MHz, DMSO-*d*₆) δ 9.11 (dd, *J* = 2.3, 0.9 Hz, 1H), 8.88 (dd, *J =* 5.0, 1.7 Hz, 1H), 8.38 (dt, *J =* 8.0, 1.9 Hz, 1H), 7.92 (d, *J =* 8.2 Hz, 1H), 7.71 - 7.63 (m, 1H), 6.16 (d, *J* = 2.2 Hz, 1H), 6.11 - 6.02 (m, 2H), 5.96 (d, *J =* 8.2 Hz, 1H), 5.80 (dd, *J =* 20.2, 7.4 Hz, 1H), 5.62 (dd, *J =* 7.4, 2.2 Hz, 1H), 4.44 (dd, *J =* 12.2, 10.3 Hz, 1H), 4.25 (dd, *J* = 12.3, 9.9 Hz, 1H), 2.36 (s, 3H), 2.11 (s, 3H), 2.09 (s, 3H), 1.96 (s, 3H).

X219 (100 mg, 0.19 mmol) was dissolved in tetrahydrofuran (1 mL), then sulfuric acid (10 mg, 0.10 mmol) was added, and the mixture was stirred for 10 min. Isopropyl ether (1 mL) was added, and the mixture was filtered and dried at 60 °C to give compound X219 hemisulfate (70 mg, yield: 64%). ¹H NMR (400 MHz, DMSO-*d*₆) δ 9.10 (d, *J* = 2.2, 0.9 Hz, 1H), 8.87 (dd, *J* = 5.0, 1.7 Hz, 1H), 8.37 (dt, *J* = 8.0, 1.9 Hz, 1H), 7.92 (d, *J* = 8.2 Hz, 1H), 7.70 - 7.62 (m, 1H), 6.16 (d, *J* = 2.1 Hz, 1H), 6.12 - 6.02 (m, 2H), 5.96 (d, *J =* 8.1 Hz, 1H), 5.80 (dd, *J* = 20.2, 7.4 Hz, 1H), 5.62 (dd, *J =* 7.4, 2.2 Hz, 1H), 4.44 (dd, *J =* 12.2, 10.3 Hz, 1H), 4.25 (dd, *J =* 12.3, 9.9 Hz, 1H), 2.11 (s, 3H), 2.09 (s, 3H), 1.96 (s, 3H).

X219 (100 mg, 0.19 mmol) was dissolved in tetrahydrofuran (1 mL), then sulfuric acid (19 mg, 0.19 mmol) was added, and the mixture was stirred for 10 min. Isopropyl ether (1 mL) was added, and the mixture was filtered and dried at 60 °C to give compound X219 sulfate (100 mg, yield: 84%). ¹H NMR (400 MHz, DMSO-*d*₆) δ 9.10 (dd, J= 2.2, 0.9 Hz, 1H), 8.87 (dd, *J* = 5.0, 1.7 Hz, 1H), 8.36 (dt, *J* = 8.0, 2.0 Hz, 1H), 7.92 (d, *J* = 8.2 Hz, 1H), 7.69 - 7.63 (m, 1H), 6.16 (d, *J* = 2.1 Hz, 1H), 6.11 - 6.02 (m, 2H), 5.96 (d, *J =* 8.2 Hz, 1H), 5.80 (dd, *J* = 20.2, 7.4 Hz, 1H), 5.62 (dd, *J =* 7.4, 2.2 Hz, 1H), 4.44 (dd, *J =* 12.2, 10.4 Hz, 1H), 4.25 (dd, *J =* 12.3, 9.8 Hz, 1H), 2.11 (s, 3H), 2.09 (s, 3H), 1.96 (s, 3H).

### Example 56: Synthesis of X265 Hydrochloride and X265 Hydrobromide

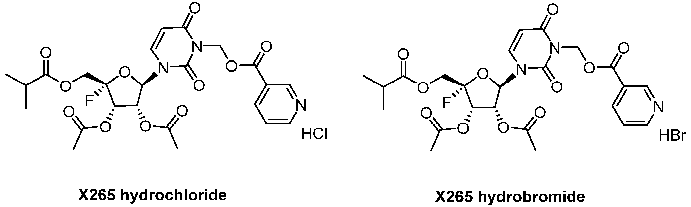

X265 (250 mg, 0.45 mmol) was dissolved in ethyl acetate (2 mL), then a 4 M hydrogen chloride-ethyl acetate solution (125 µL, 0.50 mmol) was added, and the mixture was stirred for 30 min. The reaction solution was filtered and dried at 45 °C to give compound X265 hydrochloride (150 mg, yield: 56%). ¹H NMR (400 MHz, DMSO-*d*₆) δ 9.12 (d, *J* = 2.1 Hz, 1H), 8.89 (dd, *J* = 5.0, 1.6 Hz, 1H), 8.41 (dt, *J* = 8.0, 1.9 Hz, 1H), 7.94 (d, *J* = 8.1 Hz, 1H), 7.69 (dd, *J =* 8.0, 5.0 Hz, 1H), 6.18 (d, *J =* 2.1 Hz, 1H), 6.12 - 6.01 (m, 2H), 5.96 (d, *J =* 8.1 Hz, 1H), 5.83 (dd, *J =* 20.4, 7.3 Hz, 1H), 5.65 (dd, *J =* 7.3, 2.1 Hz, 1H), 4.41 - 4.27 (m, 2H), 2.50 - 2.39 (m, 1H), 2.12 (s, 3H), 2.08 (s, 3H), 1.02 (d, *J =* 7.0 Hz, 3H), 0.98 (d, *J =* 7.0 Hz, 3H).

X265 (250 mg, 0.45 mmol) was dissolved in ethyl acetate (2 mL), then a 33% hydrogen bromide-acetic acid solution (123 mg, 0.50 mmol) was added, and the mixture was stirred for 30 min. The reaction solution was filtered and dried at 45 °C to give compound X265 hydrobromide (245 mg, yield: 85%). ¹H NMR (400 MHz, DMSO-*d*₆) δ 9.11 (d, *J* = 2.1 Hz, 1H), 8.88 (dd, *J* = 5.0, 1.6 Hz, 1H), 8.38 (dt, *J* = 8.1, 1.9 Hz, 1H), 7.93 (d, *J* = 8.2 Hz, 1H), 7.67 (dd, *J =* 8.0, 4.9 Hz, 1H), 6.17 (d, *J =* 2.1 Hz, 1H), 6.12 - 6.01 (m, 2H), 5.96 (d, *J =* 8.1 Hz, 1H), 5.83 (dd, *J =* 20.4, 7.3 Hz, 1H), 5.65 (dd, *J =* 7.3, 2.0 Hz, 1H), 4.41 - 4.26 (m, 2H), 2.50 - 2.40 (m, 1H), 2.12 (s, 3H), 2.08 (s, 3H), 1.02 (d, *J =* 7.0 Hz, 3H), 0.98 (d, *J =* 7.0 Hz, 3H).

### Example 57: Synthesis of X275

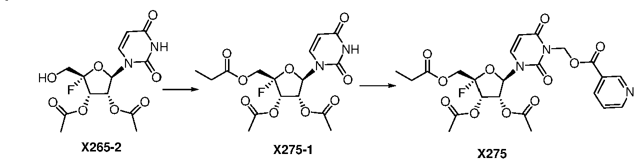

Referring to procedures in Preparation Example 51, compound X265-2 (3.0 g, 8.67 mmmol) was reacted with propionic anhydride (1.69 g, 13.01 mmol) to give compound X275-1; X275-1 was reacted with X206-1 to give compound X275 (2.30 g, two-step yield: 49%) as a foam. ¹H NMR (400 MHz, DMSO-*d*₆) δ 9.11 (d, *J* = 2.2 Hz, 1H), 8.88 (dd, *J* = 4.9, 1.7 Hz, 1H), 8.32 (dt, *J =* 8.0, 2.0 Hz, 1H), 7.97 (d, *J =* 8.2 Hz, 1H), 7.62 (dd, *J =* 8.0, 4.8 Hz, 1H), 6.22 (d, *J =* 2.1 Hz, 1H), 6.17 - 6.06 (m, 2H), 6.01 (d, *J* = 8.1 Hz, 1H), 5.86 (dd, *J* = 20.3, 7.4 Hz, 1H), 5.68 (dd, *J =* 7.3, 2.1 Hz, 1H), 4.47 (dd, *J =* 12.2, 10.3 Hz, 1H), 4.34 (dd, *J =* 12.2, 9.1 Hz, 1H), 2.43 - 2.20 (m, 2H), 2.17 (s, 3H), 2.14 (s, 3H), 0.98 (t, *J* = 7.5 Hz, 3H). ESI-MS m/z = 538.3 [M+1]⁺.

### Example 58: Synthesis of X275 Hydrochloride and X275 Hydrobromide

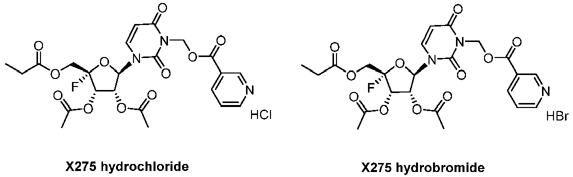

X275 (300 mg, 0.56 mmol) was dissolved in ethyl acetate (1.2 mL), then a 4 M hydrogen chloride-ethyl acetate solution (155 µL, 0.62 mmol) was added dropwise, and the mixture was stirred for 30 min. The reaction solution was filtered and dried at 45 °C to give compound X275 hydrochloride (200 mg, yield: 63%). ¹H NMR (400 MHz, DMSO-*d*₆) δ 9.11 (d, *J* = 2.2 Hz, 1H), 8.89 (dd, *J =* 5.0, 1.7 Hz, 1H), 8.40 (dt, *J* = 8.1, 1.9 Hz, 1H), 7.93 (d, *J* = 8.2 Hz, 1H), 7.69 (dd, *J* = 8.0, 5.0 Hz, 1H), 6.17 (d, *J* = 2.1 Hz, 1H), 6.12 - 6.02 (m, 2H), 5.96 (d, *J* = 8.1 Hz, 1H), 5.80 (dd, *J =* 20.3, 7.3 Hz, 1H), 5.62 (dd, *J =* 7.3, 2.1 Hz, 1H), 4.41 (dd, *J =* 12.2, 10.5 Hz, 1H), 4.29 (dd, *J* = 12.2, 9.3 Hz, 1H), 2.34 - 2.16 (m, 2H), 2.11 (s, 3H), 2.08 (s, 3H), 0.93 (t, *J* = 7.5 Hz, 3H).

X275 (900 mg, 1.67 mmol) was dissolved in ethyl acetate (3.6 mL), then a 33% hydrogen bromide-acetic acid solution (451 mg, 1.84 mmol) was added dropwise, and the mixture was stirred for 30 min. The reaction solution was filtered and dried at 45 °C to give compound X275 hydrobromide (820 mg, yield: 79%). ¹H NMR (400 MHz, DMSO-*d*₆) δ 9.16 (d, *J* = 2.1 Hz, 1H), 8.93 (dd, *J =* 5.1, 1.6 Hz, 1H), 8.48 (dt, *J =* 8.1, 1.9 Hz, 1H), 7.94 (d, *J* = 8.2 Hz, 1H), 7.76 (dd, *J* = 8.1, 5.1 Hz, 1H), 6.18 (d, *J* = 2.1 Hz, 1H), 6.13 - 6.02 (m, 2H), 5.97 (d, *J* = 8.1 Hz, 1H), 5.80 (dd, *J =* 20.2, 7.4 Hz, 1H), 5.62 (dd, *J =* 7.4, 2.1 Hz, 1H), 4.42 (dd, *J =* 12.2, 10.6 Hz, 1H), 4.29 (dd, *J* = 12.2, 9.4 Hz, 1H), 2.37 - 2.16 (m, 2H), 2.11 (s, 3H), 2.09 (s, 3H), 0.94 (t, *J* = 7.5 Hz, 3H).

### Example 59: Synthesis of X220 Hydrochloride and X220 Hydrobromide

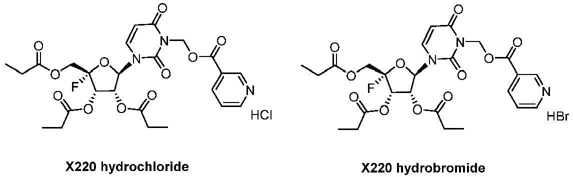

X220 (120 mg, 0.21 mmol) was dissolved in ethyl acetate (0.5 mL), then a 4 M hydrogen chloride-ethyl acetate solution (58 µL, 0.23 mmol) was added dropwise, and the mixture was stirred for 30 min. The reaction solution was concentrated to give compound X220 hydrochloride (130 mg, yield: 100%). ¹H NMR (400 MHz, DMSO-*d*₆) δ 9.07 (d, *J* = 2.3 Hz, 1H), 8.84 (dd, *J* = 4.9, 1.7 Hz, 1H), 8.29 (dt, *J* = 8.0, 2.0 Hz, 1H), 7.93 (d, *J* = 8.2 Hz, 1H), 7.59 (dd, *J* = 8.0, 4.9 Hz, 1H), 6.16 (d, *J* = 2.1 Hz, 1H), 6.11 - 6.00 (m, 2H), 5.96 (d, *J* = 8.1 Hz, 1H), 5.83 (dd, *J* = 20.2, 7.3 Hz, 1H), 5.64 (dd, *J* = 7.3, 2.1 Hz, 1H), 4.46 - 4.33 (m, 1H), 4.33 - 4.23 (m, 1H), 2.45 - 2.20 (m, 6H), 1.04 (t, *J =* 7.5 Hz, 6H), 0.93 (t, *J =* 7.5 Hz, 3H). X220 (500 mg, 0.88 mmol) was dissolved in ethyl acetate (2 mL), then a 33% hydrogen bromide-acetic acid solution (237 mg, 0.97 mmol) was added dropwise, and the mixture was stirred for 30 min. The reaction solution was filtered and dried at 45 °C to give compound X220 hydrobromide (290 mg, yield: 51%). ¹H NMR (400 MHz, DMSO-*d*₆) δ 9.10 (d, *J* = 2.2 Hz, 1H), 8.87 (dd, *J =* 5.0, 1.7 Hz, 1H), 8.35 (dt, *J =* 8.0, 2.0 Hz, 1H), 7.93 (d, *J =* 8.2 Hz, 1H), 7.64 (dd, *J* = 8.0, 5.0 Hz, 1H), 6.16 (d, *J* = 2.1 Hz, 1H), 6.12 - 6.01 (m, 2H), 5.96 (d, *J* = 8.1 Hz, 1H), 5.83 (dd, *J* = 20.2, 7.3 Hz, 1H), 5.64 (dd, *J* = 7.3, 2.1 Hz, 1H), 4.53 - 4.35 (m, 1H), 4.28 (dd, *J* = 12.2, 9.3 Hz, 1H), 2.46 - 2.19 (m, 6H), 1.04 (t, *J* = 7.5 Hz, 6H), 0.93 (t, *J* = 7.5 Hz, 3H).

### Test Example 1: Pharmacokinetic Evaluation in Rats

Male SD rats, divided into intravenous (iv) and intragastric (po) groups, were fasted for 16 h before the experiment (the intravenous group was not fasted), with free access to water, and were fed together 2 h after administration. Compound X28-1 was intravenously injected at a dose of 10.0 mg/kg (n = 3), and compounds X22, X51, and X102 (n = 3) were intragastrically administered in equimolar amounts to X28-1, which were 15.5 mg/kg, 16.5 mg/kg, and 16.6 mg/kg, respectively, with a vehicle of 5% DMSO + 5% enthanol + 40% PEG400 + 50% saline. 0.2-0.3 mL of blood was collected via the jugular vein at 5 min (intravenous only), 0.25 h, 0.5 h, 1.0 h, 2.0 h, 4.0 h, 6.0 h, 8.0 h, and 24 h after administration. The blood samples were transferred into heparin sodium anticoagulation tubes, gently mixed uniformly, and centrifuged at 2000 g for 10 min. Plasma was isolated and frozen in a refrigerator at -70 °C for later detection. The concentration of nucleoside X28-1 in the plasma was determined by the LC-MS-MS method and pharmacokinetic parameters were calculated. The results are shown in Table 1.

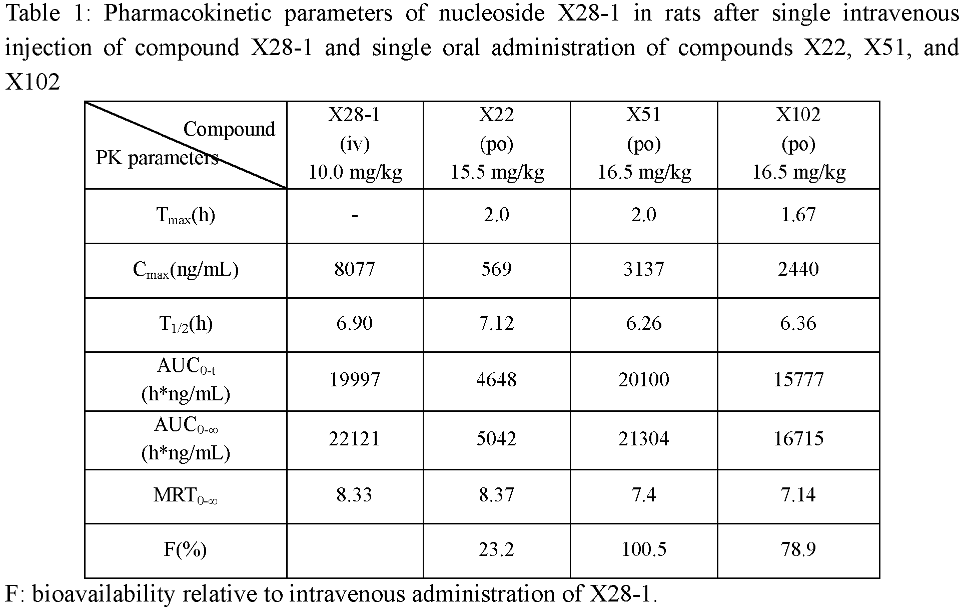

As can be seen from Table 1, nucleoside metabolite X28-1 had high exposure in rats after oral administration of compounds X51 and X102. Compared with intravenous injection of nucleoside X28-1, compounds X51 and X102 had oral bioavailability of up to 100.5% and 78.9%, respectively.

### Test Example 2: Pharmacokinetic Evaluation in Rats

A total of 12 male SD rats, divided into intravenous (IV) and intragastric (PO) groups, were fasted for 12 h before the experiment (the intravenous group was not fasted), with free access to water, and were fed together 2 h after administration. Compound X28-1 was intragastrically administered at a dose of 10.0 mg/kg (n = 3) and intravenously injected at a dose of 5.0 mg/kg (n = 3), and both compound X52 and compound X55 were intragastrically administered at a dose of 18.0 mg/kg (n = 3), with a vehicle of 5% DMSO + 5% enthanol + 40% PEG400 + 50% saline. For the intravenous group, 0.2-0.3 mL of blood was collected via the jugular vein at 5 min, 0.25 h, 0.5 h, 1.0 h, 2.0 h, 4.0 h, 8.0 h, and 24.0 h after administration, and for the intragastric group, at 5 min, 0.5 h, 1.0 h, 2.0 h, 4.0 h, 6.0 h, 8.0 h, and 24.0 h after administration. The blood samples were transferred into heparin sodium anticoagulation tubes, gently mixed uniformly, and centrifuged at 2000 g for 10 min. Plasma was isolated and frozen in a refrigerator at -70 °C for later detection. The concentration of nucleoside X28-1 in the plasma was determined by the LC-MS-MS method and pharmacokinetic parameters were calculated. The results are shown in Table 2.

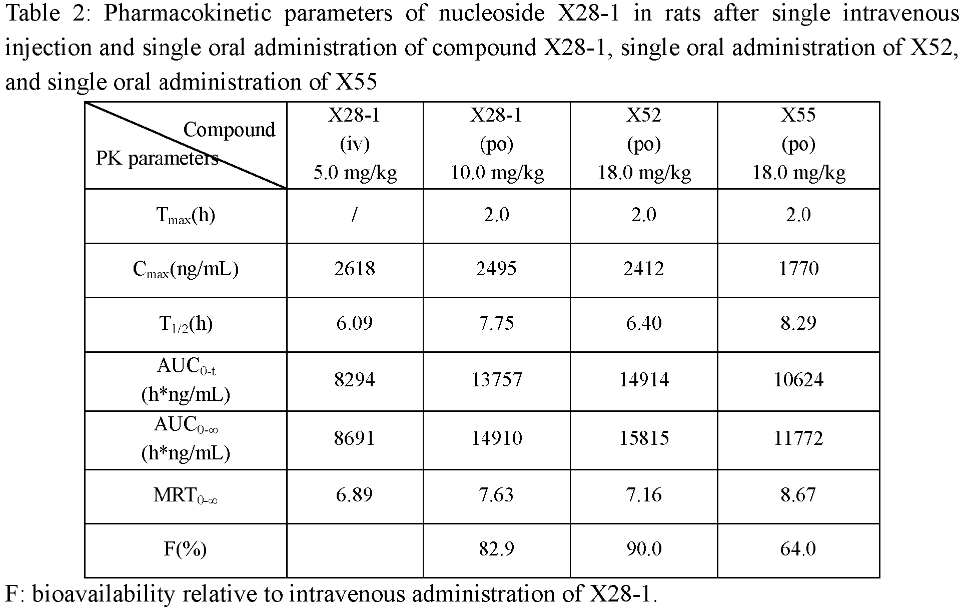

As can be seen from Table 2, nucleoside metabolite X28-1 had high exposure in rats after oral administration of compounds X52 and X55. Compared with intravenous injection of nucleoside X28-1, compounds X52 and X55 had oral bioavailability of up to 90.0% and 64.0%, respectively, with the oral bioavailability of compound X52 higher than that of X28-1.

### Test Example 3: Pharmacokinetic Evaluation in Rats

A total of 12 male SD rats, divided into 4 groups, were fasted for 12 h before the experiment, with free access to water, and were fed together 4 h after administration. Compounds X28-1, X100 hydrochloride, X206 hydrochloride, and X207 were intragastrically administered in equimolar amounts at doses of 10.0 mg/kg (n = 3), 24.0 mg/kg (n = 3), 25.0 mg/kg (n = 3), and 18.0 mg/kg (n = 3), respectively, with a vehicle of 5% DMSO + 5% enthanol + 40% PEG400 + 50% saline. 0.2-0.3 mL of blood was collected via the jugular vein at 5 min, 0.25 h, 0.5 h, 1.0 h, 2.0 h, 4.0 h, 6.0 h, 8.0 h, and 24.0 h after administration. The blood samples were transferred into heparin sodium anticoagulation tubes, and subsequent steps were the same as in Example 2. The pharmacokinetic parameters obtained by calculation are shown in Table 3.

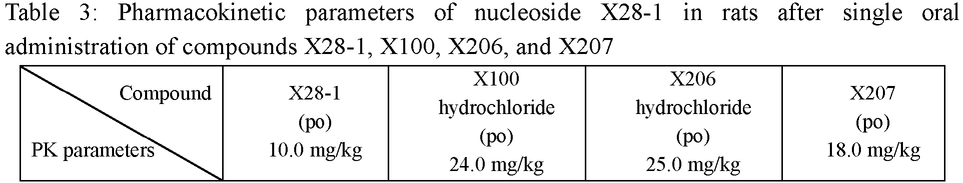

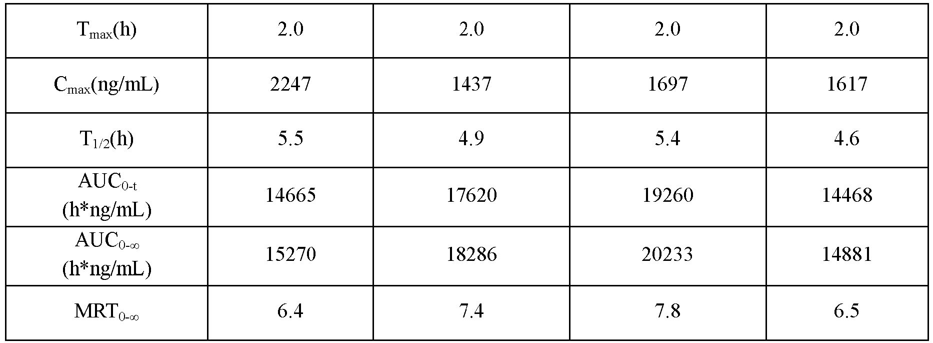

As can be seen from Table 3, in SD rats, after X28-1, X100 hydrochloride, X206 hydrochloride, and X207 were intragastrically administered in equimolar amounts, the levels of exposure of the nucleoside in rat plasma were ranked as X206 hydrochloride > X100 hydrochloride > X28-1 > X207, with the exposure of nucleoside after intragastrical administration of X206 hydrochloride being the highest and 1.3 times that of X28-1.

### Test Example 4: Pharmacokinetic Evaluation in Rats

Male SD rats were fasted for 12 h before the experiment, with free access to water, and were fed together 2 h after administration. Compounds X28-1, X219, and X219 hydrochloride were intragastrically administered at equimolar doses of 10.0 mg/kg (n = 3), 20.0 mg/kg (n = 3), and 21.0 mg/kg (n = 3), respectively, with a vehicle of 5% DMSO + 5% enthanol + 40% PEG400 + 50% saline. 0.2 mL of blood was collected via the jugular vein at 0.25 h, 0.5 h, 1.0 h, 2.0 h, 4.0 h, 6.0 h, 8.0 h, and 24.0 h after administration. The blood samples were transferred into heparin sodium anticoagulation tubes, gently mixed uniformly, put on ice, and immediately centrifuged (4 °C, 2000 g, 10 min). 30 µL of plasma was accurately pipetted and added to a plastic tube containing 600 µL of methanol solution with an internal standard, temporarily stored on dry ice after being vortexed, and then stored in an ultra-low temperature refrigerator for later detection. The concentration of nucleoside X28-1 in the plasma was determined by the LC-MS-MS method and pharmacokinetic parameters were calculated. The results are shown in Table 4.

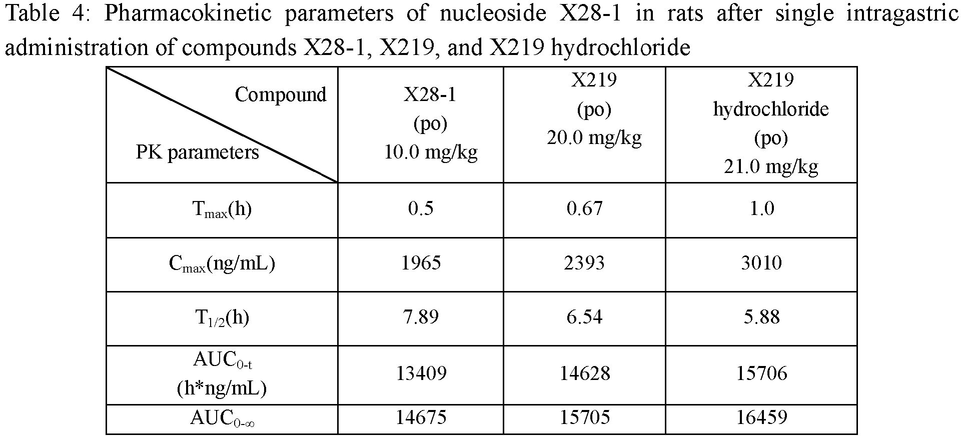

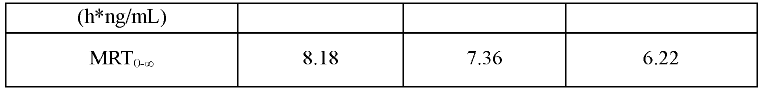

As can be seen from Table 4, in SD rats, after X28-1, X219, and X219 hydrochloride were intragastrically administered at equimolar doses, Cₘₐₓ and exposure (AUC₀₋ₜ) of the nucleoside in rat plasma in the X219 and X219 hydrochloride groups were significantly higher than those in the X28-1 group.

### Test Example 5: Pharmacokinetic Evaluation in Cynomolgus Monkeys

Male cynomolgus monkeys were fasted for 12 h before intragastric administration, with free access to water, and were fed together 2 h after administration. X28-1 was intravenously administered at a dose of 1.0 mg/kg (n = 3) and intragastrically administered at a dose of 5.0 mg/kg (n = 3), and X219 hydrochloride was intragastrically administered at a dose of 10.7 mg/kg (n = 3), with a vehicle for the compounds of 5% DMSO + 5% enthanol + 40% PEG400 + 50% saline. 0.5 mL of blood was collected via the forelimb vein at 5 min (intravenous only), 0.25 h, 0.5 h, 1.0 h, 2.0 h, 4.0 h, 6.0 h, 8.0 h, and 24.0 h after administration. The blood samples were transferred into heparin sodium anticoagulation tubes, gently shaken and mixed uniformly, put on ice, and immediately centrifuged (4 °C, 2000 g, 10 min). After centrifugation, 100 µL of plasma was accurately pipetted and added to a plastic tube containing 400 µL of methanol solution with an internal standard, temporarily stored on dry ice after being vortexed, and then stored in an ultra-low temperature refrigerator for later detection. The concentration of nucleoside X28-1 in the plasma was determined by the LC-MS-MS method and pharmacokinetic parameters were calculated. The results are shown in Table 5.

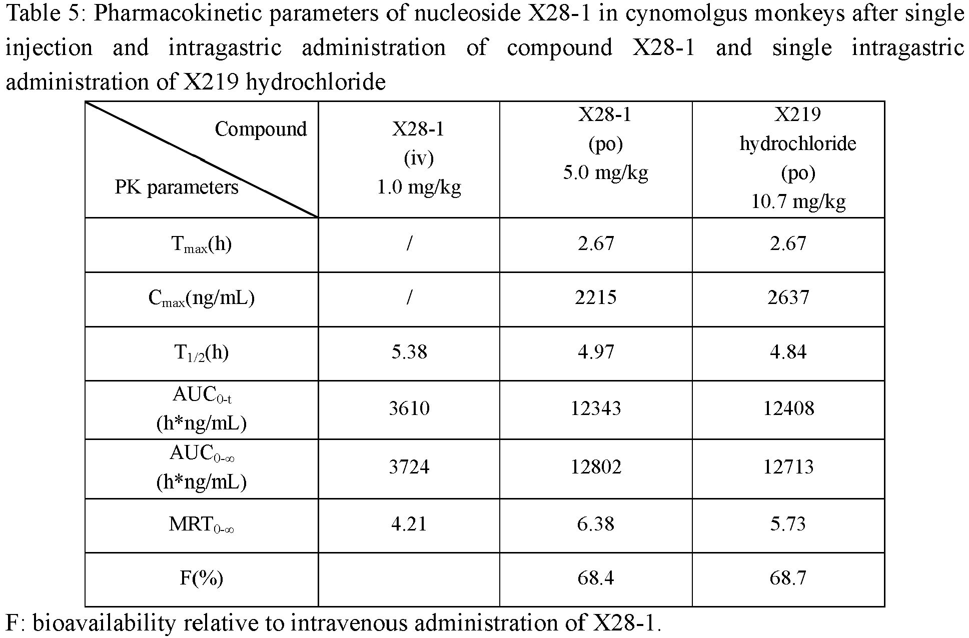

As can be seen from Table 5, X219 hydrochloride had good oral pharmacological properties in cynomolgus monkeys, with oral bioavailability of up to 68.7%.

### Test Example 6: Stability Test of Compounds in Solutions at Different pH Values

About 0.001 g of each of the compounds was taken, weighed precisely, and dissolved in buffered saline solutions at different pH values. Preparation method for a sodium acetate buffered saline solution at pH 4.0: 1.22 g of sodium acetate was taken, 20.5 mL of 2 mol/L aqueous acetic acid solution was added, and the mixture was diluted to 1000 mL with water. Preparation method for a potassium dihydrogen phosphate buffered saline solution at pH 6.8: 250 mL of 0.2 mol/L potassium dihydrogen phosphate solution was taken and mixed with 112.0 mL of 0.2 mol/L sodium hydroxide solution, and the mixture was diluted to 1000 mL with water and shaken uniformly. The stability of the compounds was evaluated by analyzing changes in the content of the compounds in solutions at pH 4.0 and pH 6.8 by liquid chromatography. The results are shown in Table 6.

**Table 6: Reduction in the content of the compounds in buffered saline solutions at different pH values within 24 h**

| pH value | Time (h) | Content reduction (%) | | | | | | |
|---|---|---|---|---|---|---|---|---|
| | | **X28-1** | **X51** | **X52** | **X55** | **X219** | **X219 hydrochloride** | **X206** |
| | 0 | / | / | / | / | / | / | / |
| | 3 | 5.7 | 1.6 | 0.9 | 2.4 | 0.22 | 0.24 | 0.01 |
| | 6 | 12.2 | 2.5 | 1.4 | 4.9 | 0.34 | 0.28 | 0.03 |
| | 9 | 17.5 | 3.1 | 1.9 | 6.9 | 0.44 | 0.34 | 0.02 |
| pH4.0 | 12 | 22.2 | 3.4 | 2.2 | 9.0 | 0.62 | 0.55 | 0.03 |
| | 15 | 26.9 | 3.9 | 2.6 | 10.8 | 0.70 | 0.64 | 0.03 |
| | 18 | 30.3 | 4.6 | 3.0 | 12.7 | 0.78 | 0.72 | 0.01 |
| | 21 | 35.9 | 5.2 | 3.4 | 14.2 | 0.93 | 0.73 | 0.03 |
| | 24 | 40.1 | 5.6 | 3.6 | 15.4 | 1.02 | 0.87 | 0.00 |
| | 0 | / | / | / | / | / | / | / |
| | 3 | 8.0 | 1.1 | 1.2 | 1.0 | 1.06 | 0.98 | 1.93 |
| | 6 | 15.3 | 2.0 | 2.3 | 2.3 | 1.86 | 2.03 | 3.09 |
| | 9 | 22.0 | 3.0 | 3.3 | 3.3 | 2.92 | 3.09 | 3.84 |
| pH6.8 | 12 | 28.3 | 3.8 | 4.3 | 4.4 | 3.96 | 4.10 | 4.06 |
| | 15 | 33.4 | 4.7 | 5.4 | 5.4 | 5.27 | 5.13 | 5.72 |
| | 18 | 38.0 | 5.6 | 6.6 | 6.4 | 6.01 | 6.13 | 6.34 |
| | 21 | 42.9 | 6.5 | 7.6 | 7.7 | 7.02 | 7.07 | 7.28 |
| | 24 | 46.9 | 7.4 | 8.7 | 8.9 | 8.38 | 8.02 | 8.16 |

As can be seen from the table above, compound X28-1 had poor stability in solutions at pH 4.0 and pH 6.8, with the content reduced by 40.1% and 46.9% in 24 h. In contrast, X51, X52, X219, X219 hydrochloride, and X206 had better stability, with the content changed within 10% in 24 h; particularly in the solution at pH 4.0, there were changes of 1.02%, 0.87%, and 0.0% in the content of X219, X219 hydrochloride, and X206, respectively, showing good stability. The content of X55 in the solution at pH 6.8 changed within 10% in 24 h, and therefore X55 also had better overall stability than X28-1.

### Test Example 7: Evaluation of In Vitro Activity of Compounds Against Respiratory Syncytial Virus and Influenza Virus

Assay of activity against respiratory syncytial virus: HEp-2 cells were seeded into a 96-well assay plate at a density of 6000 cells in 100 µL of medium per well and cultured overnight in an incubator at 37 °C with 5% CO₂. The compounds were dissolved in DMSO at a starting concentration of 10 µM or 5 µM. The compounds at different concentrations (10 µM as initial concentration, 3-fold dilution, unless otherwise specified) and a respiratory syncytial virus (RSV) A long strain (MOI = 0.02) were added to the assay plate, and a cell control (cells, no compound treatment or viral infection), a virus control (cells infected with virus, no compound treatment), and a culture solution control (culture solution only) were set. The final concentration of DMSO in the culture solution was 0.5%. The cells were cultured in an incubator at 37 °C with 5% CO₂ for 5 days.

Assay of activity against influenza virus: MDCK cells were seeded into a 96-well assay plate at a density of 15,000 cells in 100 µL of medium per well and cultured overnight in an incubator at 37 °C with 5% CO₂. The compounds were dissolved in DMSO at a starting concentration of 10 µM or 5 µM. The compounds at different concentrations (10 µM as initial concentration, 3-fold dilution, unless otherwise specified) and an influenza virus (IFV) A/California/07/2009 (H1N1) pdm09 strain (MOI = 0.02) were added to the assay plate, and a cell control (cells, no compound treatment or viral infection), a virus control (cells infected with virus, no compound treatment), and a culture solution control (culture solution only) were set. The final concentration of DMSO in the culture solution was 0.5%. The cells were cultured in an incubator at 37 °C with 5% CO₂ for 5 days.

Cell viability was determined using the cell viability assay kit CCK8. The antiviral activity and cytotoxicity of the test samples were represented by the inhibition rate (%) and the cell viability (%) of the samples at different concentrations for the virus-induced cytopathic effect, respectively. Inhibition rate (%) = (test well reading - mean of virus control)/(mean of cell control - mean of virus control) × 100; cell viability (%) = (test well reading - mean of medium control)/(mean of cell control - mean of medium control) × 100.

The inhibition rate and cell viability of the samples were analyzed by nonlinear fitting using GraphPad Prism, and values of 50% effective concentration (EC₅₀) and 50% cytotoxicity concentration (CC₅₀) of the samples were calculated.

**Table 7: In vitro inhibitory activity of the compounds against respiratory syncytial virus and influenza virus**

| Compou nd | Structure | IFV (MDCK) | | RSV (HEp-2) | |
|---|---|---|---|---|---|
| | | EC₅₀ (µM) | CC₅₀ (µM) | EC₅₀ (µM) | CC₅₀ (µM) |
| X43 | 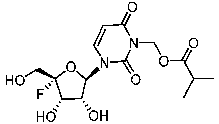 | 0.50 | > 10 | 2.32 | > 10 |
| X51 | 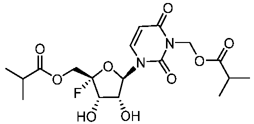 | < 0.37 | > 10 | 2.15 | > 10 |
| X52 | 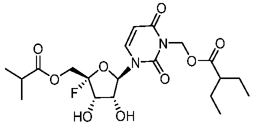 | 0.57 | > 10 | 4.85 | > 10 |
| X55 | 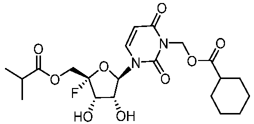 | < 0.37 | > 10 | 1.63 | > 10 |
| X57 | 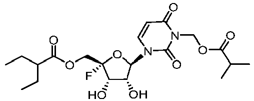 | 90.5%^{a} | > 5 | 50.3%^{a} | > 5 |
| X61 | 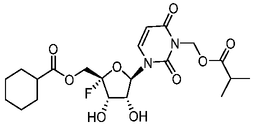 | < 0.37 | > 10 | 2.04 | > 10 |
| X63 | 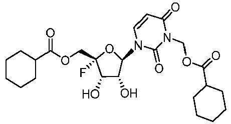 | < 0.37 | > 10 | 1.51 | > 10 |
| X79^{b} | 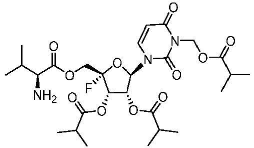 | 0.48 | > 10 | 1.46 | > 10 |
| X98^{b} | 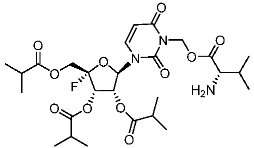 | 0.50 | > 10 | 1.42 | > 10 |
| X100^{b} | 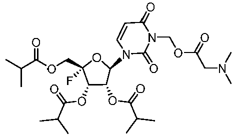 | 0.41 | > 10 | 1.79 | > 10 |
| X113 | 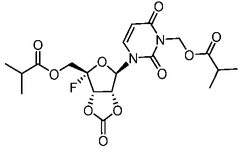 | < 0.37 | > 10 | 1.97 | > 10 |
| X134 | 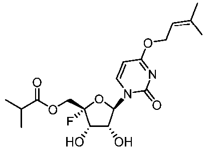 | 0.46 | > 10 | 1.20 | > 10 |
| X173 | 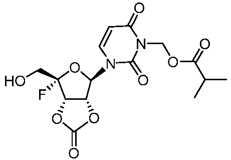 | 0.46 | > 10 | 2.62 | > 10 |
| X185^{c} | 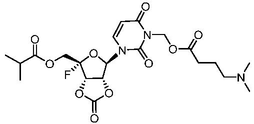 | NT | NT | 94.7%^{a} | > 5 |
| X198 | 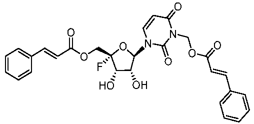 | NT | NT | 2.45 | > 10 |
| X199 | 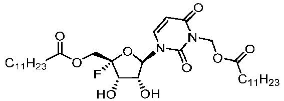 | 0.41 | > 10 | 0.46 | > 10 |
| X200 | 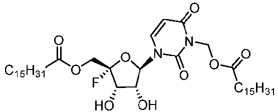 | 2.29 | > 10 | 1.17 | > 10 |
| X201 | 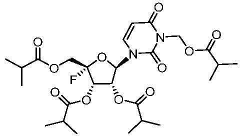 | 0.56 | > 10 | 1.51 | > 10 |
| X204^{b} | 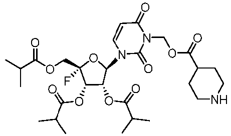 | 0.65 | >10 | 1.52 | > 10 |
| X205^{b} | 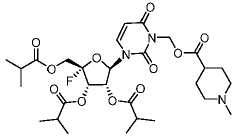 | < 0.37 | > 10 | 2.56 | > 10 |
| X206 | 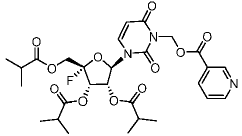 | 0.38 | > 10 | 2.43 | > 10 |
| X207 | 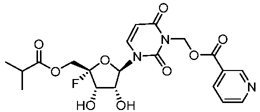 | 0.38 | > 10 | 4.09 | > 10 |
| X208 | 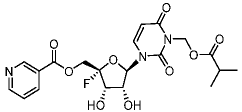 | < 0.37 | > 10 | 1.09 | > 10 |
| X210 | 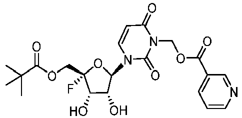 | < 0.37 | > 10 | 1.88 | > 10 |
| X211 | 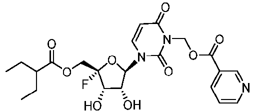 | 3.34 | > 10 | 5.82 | > 10 |
| X214 | 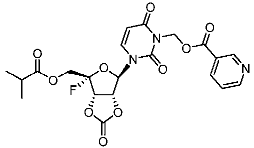 | < 0.37 | > 10 | 2.09 | > 10 |
| X219 | 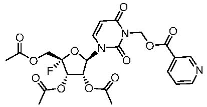 | 3.920 | > 10 | 2.60 | > 10 |
| X220 | 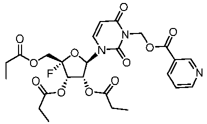 | 0.819 | > 10 | 1.29 | > 10 |
| X222 | 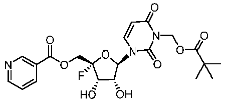 | < 0.37 | > 10 | 0.77 | > 10 |
| X223 | 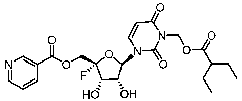 | 0.82 | > 10 | 2.14 | > 10 |
| X231 | 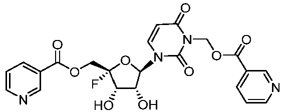 | 0.51 | > 10 | 1.16 | > 10 |
| X232 | 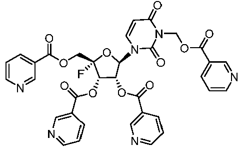 | 0.85 | > 10 | 1.13 | > 10 |
| X233 | 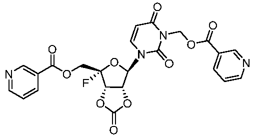 | 0.46 | > 10 | 1.20 | > 10 |
| X257 | 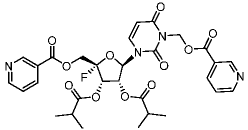 | 1.381 | > 10 | 1.64 | > 10 |
| X260^{b} | 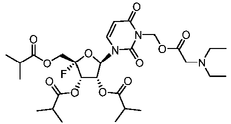 | < 0.37 | > 10 | 2.34 | > 10 |
| X265 | 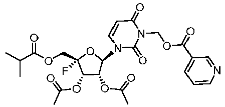 | 1.662 | > 10 | 2.78 | > 10 |
| X266^{b} | 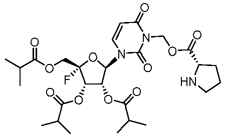 | 0.894 | > 10 | 1.02 | > 10 |
| X267^{b} | 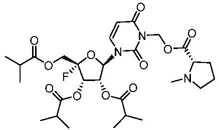 | 0.500 | > 10 | 0.88 | > 10 |
| X28-1 | 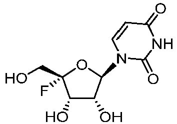 | < 0.37 | > 10 | 6.06 | > 10 |

| | | | | | |
|---|---|---|---|---|---|
| ^{a}: inhibition rate at a concentration of 5 µM; ^{b}: hydrochloride; ^{c}: hemimaleate; NT: not tested | | | | | |

*In vitro* antiviral activity assay showed that the compounds listed in Table 7 had significant activity against respiratory syncytial virus and influenza virus. Compounds X51, X55, X61, X63, X113, 205 hydrochloride, X208, X210, X214, X222, and X260 hydrochloride had an EC₅₀ of less than 0.37 µM for influenza virus, which was comparable to EC₅₀ for X28-1; most of the compounds in the table had better inhibitory activity against respiratory syncytial virus than X28-1.

### Test Example 8: Evaluation of In Vitro Activity of Compounds Against Severe Fever with Thrombocytopenia Syndrome Virus (SFTSV)

Vero (ATCC) cells were seeded into a 96-well assay plate at a density of 3 × 10⁴ cells in 100 µL of medium per well and cultured for 24 h in an incubator at 37 °C with 5% CO₂. The compounds were dissolved in DMSO, diluted with a culture solution containing 2% FBS, and tested for the inhibition rate for viruses at a concentration of 10 µM. The concentration gradients for the EC₅₀ test were set to be 10 µM, 5 µM, 2.5 µM, 1.25 µM, 0.625 µM, and 0.3125 µM, a cell control (no compound treatment and no viral infection) and a MOCK group (containing a certain concentration of DMSO, with viral infection) were set, and the content of DMSO in the MOCK group was determined according to the concentration of the drug tested and was consistent with that of the drug group. The compounds diluted with the culture solution were added to the assay plate, and two or three duplicate wells were set for each concentration. After being cultured for 2 h, the cells were infected with SFTSV-Nluc (self-constructed SFTSV virus with an Nluc fluorescent label), with the multiplicity of infection MOI = 0.1, and cultured for 48 h. The supernatant was discarded. The cells were lysed with a Passive lysis buffer (100 µL/well), and the lysate was shaken and mixed uniformly. After 20 min, 20 µL of the lysate was taken and determined for the luc value. An equal volume of working solution was added, and the mixture was assayed on a platform. The inhibition rate (inhibition rate = (1 - sample value/control value) × 100%) was calculated using the MOCK group as a control group, and the 50% effective concentration (EC₅₀) of the samples was calculated using GraphPad Prism.

**Table 8: In vitro inhibitory activity of compounds against SFTSV**

| Compound | Structure | Inhibition rate (10 µM) | EC₅₀ (µM) |
|---|---|---|---|
| X51 | 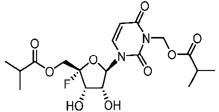 | 94.1% | 0.43 |
| X55 | 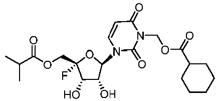 | 93.6% | 1.04 |
| X61 | 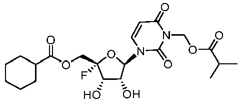 | 96.1% | 0.59 |
| X79^{a} | 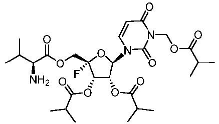 | < 10% | NT |
| X98^{a} | 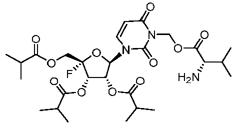 | 95.7% | 1.16 |
| X199 | 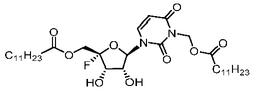 | 96.1% | 2.79 |
| X206 | 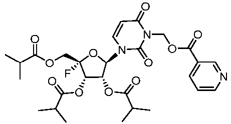 | 93.7% | 1.73 |
| X207 | 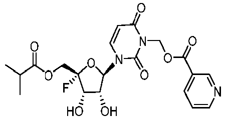 | 91.8% | 1.89 |
| X219 | 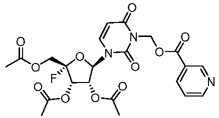 | 91.7% | 3.39 |
| X222 | 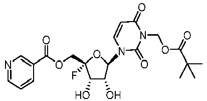 | 90.3% | 3.30 |
| X266^{a} | 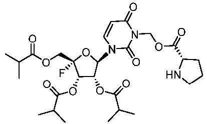 | 94.6% | 2.07 |
| X267^{a} | 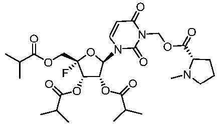 | 94.1% | 2.00 |
| X28-1 | 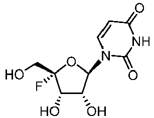 | 95.3% | 0.80 |

| | | | |
|---|---|---|---|
| ^{a}: hydrochloride; NT: not tested. | | | |

As can be seen from Table 8, the test compounds, except X79, were all able to significantly inhibit the replication of SFTSV-Nluc in Vero cells, all with inhibition rates above 90% at a concentration of 10 µM. The EC₅₀ values of the compounds X51 and X61 were 0.43 µM and 0.59 µM, respectively, and therefore the two compounds had better activity against SFTSV-Nluc than X28-1.

### Test Example 9: Comparison of Solubility of Compounds

The compounds X219-1, X219, X265, and salts thereof were selected and compared for solubility. 5 mg of each of the test samples was weighed and placed in a glass test tube, 1 mL of deionized water was added, and the mixture was ultrasonically dissolved for 15 min, during which the test sample was added gradually and progressively until it could not be completely dissolved. After filtration, the saturated test sample solution was subjected to a 50-fold dilution, and the solubility of each of the compounds in water was determined.

**Table 9: Solubility of compounds in water**

| Compound | Structure | Solubility (mg/mL) |
|---|---|---|
| X219 | 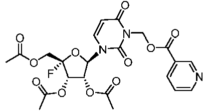 | 0.70 |
| X219 hydrochloride | 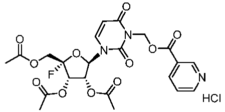 | 4.74 |
| X219 hydrobromide | 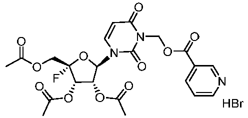 | 17.63 |
| X219-1 | 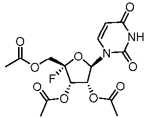 | 0.15 |
| X265 | 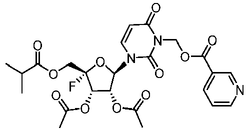 | 0.41 |
| X265 hydrochloride | 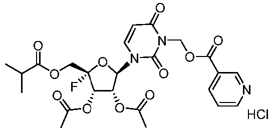 | 19.50 |
| X265 hydrobromide | 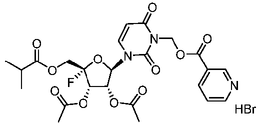 | 8.05 |
| X265-3 | 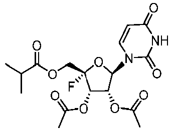 | 0.10 |

Water solubility is an important parameter that affects the absorption and bioavailability of a drug in the body. As can be seen from the table above, compound X219 had better water solubility than X219-1, and compounds X219 and X265 had significantly improved water solubility after salification.

### Test Example 10: Evaluation of In Vivo Efficacy of Compound X219 Hydrochloride for Influenza Virus in Mice

### Experimental procedures:

Balb/C male mice of 3-4 weeks old were divided into 6 groups of 7 mice each, including a blank control group, a vehicle group, a positive drug group (favipiravir 200 mpk, mpk = mg/kg), and three dose groups (10 mpk, 20 mpk, and 30 mpk) of X219 hydrochloride. The mice were infected with influenza virus Influenza A/PR/8/34 (PR8) (3 × 10⁴ PFU, nasal drip volume of 30 µL) by nasal drip. A vehicle for the compounds was 5% DMSO + 5% enthanol + 40% PEG400 + 50% saline. The blank control group was free of virus treatment and compound or vehicle treatment. Except for the blank control group, the mice were challenged on Day 0 and subjected to intragastric administration of the compounds or vehicle once 1 h after the challenge. On Days 1-3, the mice in each group were subjected to administration of the compounds or vehicle once every day. Changes in the body weight of the mice were recorded every day. Mice were sacrificed on Day 4 and dissected for samples. The left lungs of the mice were fixed in a tissue fixative solution and the right lungs were soaked in DMEM for tissue homogenization. The viral copies (viral load) and the expression level of inflammatory factors were determined.

### Experimental results:

The results are shown in FIGs. 1 and 2. After 4 days of infection, the body weight of the mice in the PR8 + vehicle group was reduced by more than 20% compared with the initial body weight, and the body weight of the mice in the positive drug group (PR8 + favipiravir 200 mpk) and PR8 + X219 hydrochloride group (10 mpk, 20 mpk, and 30 mpk) did not change significantly (panel A in FIG. 1); the pulmonary viral load of the mice after administration of X219 hydrochloride was significantly reduced compared with that in the PR8 + vehicle group, by greater than 4 log₁₀ at doses of 20 mg/kg and 30 mg/kg, and therefore, X219 hydrochloride had a significantly better inhibitory effect on virus than the positive drug favipiravir (200 mpk) (panel B in FIG. 1); the levels of inflammatory factors (IL-6, IL-8, IL-1β, TNF-α, ISG-15, and TNF-1β) in the lungs of mice in the X219 hydrochloride group were all significantly lower than those in the PR8 + vehicle group. The above experimental results showed that the X219 hydrochloride had a good effect in resisting influenza virus PR8 in mice (FIG. 2).

### Test Example 11: Evaluation of Efficacy of Compound X28-1 for Resisting Severe Fever with Thrombocytopenia Syndrome Virus (SFTSV) in Mice

### Experimental procedures:

A129 mice of 6-8 weeks old were divided into 7 groups of 5 mice each. A wild-type SFSV virus (SFTSV-WT) and a self-constructed SFTSV virus with an Nluc fluorescent label (SFTSV-Nluc) were used, and the following groups were set: a blank control vehicle group (no virus treatment, vehicle: 5% DMSO + 5% enthanol + 40% PEG400 + 50% saline), an SFTSV-WT vehicle group, an SFTSV-WT X28-1 20 mpk group, an SFTSV-WT positive drug group (favipiravir 150 mpk), an SFTSV-Nluc vehicle group, an SFTSV-Nluc X28-1 20 mpk group, and an SFTSV-Nluc positive drug group (favipiravir 150 mpk). Mice were anesthetized with a mixture of isoflurane and oxygen for blood sampling and virus inoculation experiments. On Day 0, the mice in the SFTSV-WT group were intraperitoneally injected with SFTSV-WT (DMEM solution, 1 × 10³ PFU/mouse) and the mice in the SFTSV-Nluc group were intraperitoneally injected with SFTSV-Nluc (DMEM solution, 1 × 10⁴ PFU/mouse), both at an injection volume of 100 µL. Except for the blank control group and the vehicle control group, animals in the groups were subjected to intragastric administration of the test compounds or favipiravir once daily from Day 0 to Day 6 after the viral infection, and changes in the body weight and the survival of the mice were monitored.

The test compounds were further evaluated for their inhibitory effect on SFTSV-Nluc in mice.

The experimental procedures were similar to those described above, except that the administration was performed only from Day 0 to Day 2 after viral infection, and animals of the groups were subjected to intragastric administration of the test compounds or favipiravir once daily. All mice were anesthetized on Day 1 and Day 3, followed by fluorescence imaging. All mice were euthanized on Day 3 after infection, and tissues (brain, lungs, heart, spleen, kidneys, testis, liver, intestine, and femur) were collected, imaged *in vitro,* divided into two portions, and stored at -80 °C for later use or paraffin-embedded for subsequent pathological analysis.

### Experimental results:

The results are shown in FIGs. 3-5. The mice in the SFTSV-WT vehicle group and the SFTSV-Nluc vehicle group began to show a significant reduction in the body weight on the second day after infection, and all died from day 3 to day 4 after infection (reduction of more than 20% in weight was determined to be death); in the X28-1 treatment group, all mice infected with SFTSV-WT or SFTSV-Nluc showed no significant change in the body weight (panel A in FIG. 3), and had a survival rate of up to 100% (panel B in FIG. 3).

Fluorescence imaging showed that the mice treated with X28-1 all exhibited negligible fluorescence intensity, and individual mice treated with favipiravir exhibited weak fluorescence intensity on the ventral side (panels A and B in FIG. 4) and the dorsal side (panels C and D in FIG. 4) on Day 1 and Day 3. *In vitro* imaging of the spleen, kidneys, liver, and intestine of the mice showed that fluorescence intensity in all organs of the mice in the SFTSV-Nluc X28-1 20 mpk group was significantly weaker than that in the SFTSV-Nluc vehicle group (panels E and F in FIG. 4).

The spleen/body weight ratio and the kidney/body weight ratio of the mice in the SFTSV-Nluc vehicle group were significantly increased compared with those in the blank control group, and the spleen/body weight ratio and the kidney/body weight ratio of the mice treated with X28-1 were consistent with those in the blank control group (panel A in FIG. 5). The mice in the SFTSV-Nluc X28-1 20 mpk group showed significantly reduced viral titer and copy number in the spleen and kidney compared with the mice in the SFTSV-Nluc vehicle group (panel B in FIG. 5). The experimental results showed that X28-1 had good efficacy for resisting SFTSV in A129 mice.

### Test Example 12: Evaluation of In Vivo Efficacy of Compound X219 Hydrochloride for Respiratory Syncytial Virus (RSV) in Mice

### Experimental procedures:

BALB/C female mice of 6-8 weeks old were divided into 3 groups of 3 mice each. The mice were infected with respiratory syncytial virus (RSV A2) by nasal drip, with a challenge dose of 4 × 10⁶ FFU and a nasal drip volume of no more than 50 µL/mouse. The animals were divided into an X219 hydrochloride (RSV A2 + X219 hydrochloride group), an X28-1 group (RSV A2 + X28-1 group), and a vehicle group (RSV A2 + vehicle group). A vehicle for the compounds was 5% DMSO + 5% ethanol + 40% PEG400 + 50% saline. On Day 0, after 1-2 h of challenge, the groups were each subjected to intragastric administration of the vehicle, X219 hydrochloride (20 mpk), or X28-1 (10 mpk), at 200 µL/mouse; on Day 1 to Day 4, the mice was subjected to intragastric administration once daily. Changes in the body weight of the mice in the groups were recorded every day. On Day 4, after weighing, the mice were sacrificed by cervical dislocation and dissected to take lungs. The right lungs were each immersed in a tissue grinding tube containing PBS for the detection of viral load in the lungs.

### Experimental results:

The results are shown in FIG. 6. Four days after infection, the mice in the vehicle group showed the greatest reduction in the body weight by approximately 20%. The mice in the X219 hydrochloride and X28-1 groups began to show an increase in the body weight on Day 3 after challenge (panel A in FIG. 6). Compared with the vehicle group, the mice in the RSV A2 + X219 hydrochloride 20 mpk group showed a significant reduction in the pulmonary viral load by about 0.7 log₁₀; the pulmonary viral load of the mice in the RSV A2 + X28-1 10 mpk group was reduced by about 0.4 log₁₀ (panel B in FIG. 6).

## Claims

1. A compound represented by formula (I), or a pharmaceutically acceptable salt, a solvate, a stereoisomer, a geometric isomer, an isotopically labeled compound or a prodrug thereof: 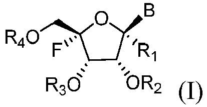 wherein,
B is selected from 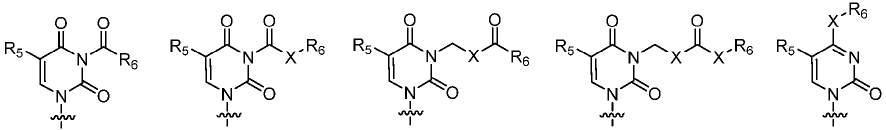 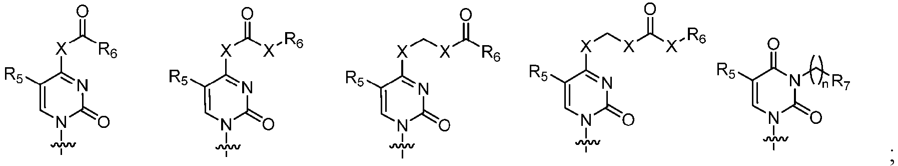
X is selected from O, NH, and S;
R₅ is selected from hydrogen, deuterium, and halogen;
R₆ is selected from C₁₋₂₀ alkyl, C₃₋₂₀ cycloalkyl, 3- to 20-membered heterocycloalkyl, C₂₋₂₀ alkenyl, C₂₋₂₀ alkynyl, C₆₋₂₀ aryl, 5- to 15-membered heteroaryl, amino C₁₋₂₀ alkyl, C₃₋₂₀ cycloalkyl C₁₋₂₀ alkyl, C₁₋₂₀ alkylamino C₁₋₂₀ alkyl, C₁₋₂₀ dialkylamino C₁₋₂₀ alkyl, C₃₋₂₀ cycloalkylamino C₁₋₂₀ alkyl, C₁₋₂₀ alkoxy C₁₋₂₀ alkyl, C₃₋₂₀ cycloalkoxy C₁₋₂₀ alkyl, a group after removal of carboxyl from an amino acid, R₂O-C₁₋₂₀ alkyl, R₂NH-C₁₋₂₀ alkyl, R₂O-C₃₋₂₀ cycloalkyl, and R₂NH-C₃₋₂₀ cycloalkyl;
n is selected from an integer of 1-6;
R₇ is 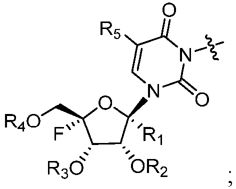 R₁ is selected from hydrogen and deuterium;
R₂ and R₃ are each independently selected from hydrogen, C₁₋₂₀ alkanoyl, C₃₋₂₀ cycloalkanoyl, 3- to 20-membered heterocycloalkanoyl, C₂₋₂₀ alkenylacyl, C₂₋₂₀ alkynylacyl, C₆₋₂₀ aroyl, 5- to 15-membered heteroaroyl, amino C₁₋₂₀ alkanoyl, C₁₋₂₀ alkylamino C₁₋₂₀ alkanoyl, C₁₋₂₀ dialkylamino C₁₋₂₀ alkanoyl, C₃₋₂₀ cycloalkylamino C₁₋₂₀ alkanoyl, C₁₋₂₀ alkoxy C₁₋₂₀ alkanoyl, C₃₋₂₀ cycloalkoxy C₁₋₂₀ alkanoyl, and a group after removal of hydroxyl in carboxyl from an amino acid, wherein the C₁₋₂₀ alkanoyl and the C₃₋₂₀ cycloalkanoyl are unsubstituted or substituted with halogen,
or R₂ and R₃ are connected with each other to form 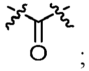
R₄ is selected from hydrogen, C₁₋₂₀ alkanoyl, C₃₋₂₀ cycloalkanoyl, 3- to 20-membered heterocycloalkanoyl, C₂₋₂₀ alkenylacyl, C₂₋₂₀ alkynylacyl, C₆₋₂₀ aroyl, 5- to 15-membered heteroaroyl, amino C₁₋₂₀ alkanoyl, C₁₋₂₀ alkylamino C₁₋₂₀ alkanoyl, C₁₋₂₀ dialkylamino C₁₋₂₀ alkanoyl, C₃₋₂₀ cycloalkylamino C₁₋₂₀ alkanoyl, C₁₋₂₀ alkoxy C₁₋₂₀ alkanoyl, C₃₋₂₀ cycloalkoxy C₁₋₂₀ alkanoyl, a group after removal of hydroxyl in carboxyl from an amino acid, 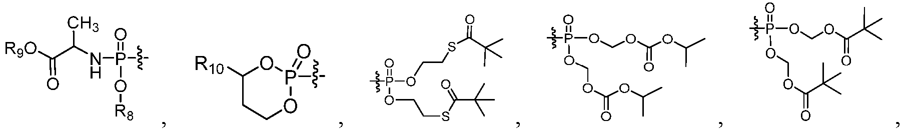 and 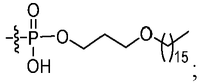
R₈ is selected from C₆₋₂₀ aryl and 5- to 15-membered heteroaryl;
R₉ is selected from C₁₋₂₀ alkyl and C₆₋₂₀ arylmethylene;
R₁₀ is selected from C₁₋₆ alkyl, C₃₋₆ cycloalkyl, C₆₋₂₀ aryl, and 5- to 15-membered heteroaryl.

2. The compound, or the pharmaceutically acceptable salt, the solvate, the stereoisomer, the geometric isomer, the isotopically labeled compound or the prodrug thereof according to claim 1, wherein,
B, X, R₁, and R₅ are as defined in claim 1;
R₆ is selected from C₁₋₂₀ alkyl, C₃₋₁₀ cycloalkyl, 3- to 10-membered heterocycloalkyl, C₂₋₁₀ alkenyl, C₃₋₁₀ alkynyl, C₆₋₁₂ aryl, 5- to 12-membered heteroaryl, amino C₁₋₁₀ alkyl, C₃₋₁₀ cycloalkyl C₁₋₁₀ alkyl, C₁₋₁₀ alkylamino C₁₋₁₀ alkyl, C₁₋₁₀ dialkylamino C₁₋₁₀ alkyl, C₃₋₁₀ cycloalkylamino C₁₋₁₀ alkyl, C₁₋₁₀ alkoxy C₁₋₁₀ alkyl, C₃₋₁₀ cycloalkoxy C₁₋₁₀ alkyl, a group after removal of carboxyl from an amino acid, R₂O-C₁₋₁₀ alkyl, R₂NH-C₁₋₁₀ alkyl, R₂O-C₃₋₁₀ cycloalkyl, and R₂NH-C₃₋₁₀ cycloalkyl;
n is selected from an integer of 1-4;
R₇ is 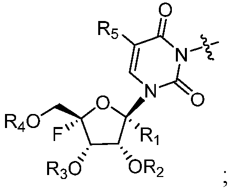
R₂ and R₃ are each independently selected from hydrogen, C₂₋₁₀ alkanoyl, C₃₋₁₀ cycloalkanoyl, 3- to 10-membered heterocycloalkanoyl, C₃₋₁₀ alkenylacyl, C₃₋₁₀ alkynylacyl, C₆₋₁₂ aroyl, 5- to 10-membered heteroaroyl, amino C₁₋₁₀ alkanoyl, C₁₋₁₀ alkylamino C₁₋₁₀ alkanoyl, C₁₋₁₀ dialkylamino C₁₋₁₀ alkanoyl, C₃₋₁₀ cycloalkylamino C₁₋₁₀ alkanoyl, C₁₋₁₀ alkoxy C₁₋₁₀ alkanoyl, C₃₋₁₀ cycloalkoxy C₁₋₁₀ alkanoyl, and a group after removal of hydroxyl in carboxyl from an amino acid, wherein the C₂₋₁₀ alkanoyl and the C₃₋₁₀ cycloalkanoyl are unsubstituted or substituted with halogen; or R₂ and R₃ are connected with each other to form 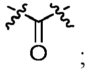
R₄ is selected from hydrogen, C₂₋₂₀ alkanoyl, C₃₋₁₀ cycloalkanoyl, 3- to 10-membered heterocycloalkanoyl, C₃₋₁₀ alkenylacyl, C₃₋₁₀ alkynylacyl, C₆₋₁₅ aroyl, 5- to 12-membered heteroaroyl, amino C₁₋₁₀ alkanoyl, C₁₋₁₀ alkylamino C₁₋₁₀ alkanoyl, C₁₋₁₀ dialkylamino C₁₋₁₀ alkanoyl, C₃₋₁₀ cycloalkylamino C₁₋₁₀ alkanoyl, C₁₋₁₀ alkoxy C₁₋₁₀ alkanoyl, C₃₋₁₀ cycloalkoxy C₁₋₁₀ alkanoyl, a group after removal of hydroxyl in carboxyl from an amino acid, 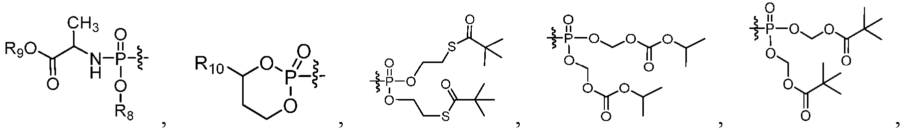 and 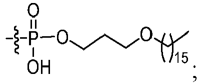
R₈ is selected from C₆₋₁₄ aryl and 5- to 12-membered aryl;
R₉ is selected from C₂₋₁₀ alkyl and C₆₋₁₅ arylmethylene;
R₁₀ is selected from C₁₋₆ alkyl, C₃₋₆ cycloalkyl, C₆₋₁₄ aryl, and 5- to 12-membered heteroaryl.

3. The compound, or the pharmaceutically acceptable salt, the solvate, the stereoisomer, the geometric isomer, the isotopically labeled compound or prodrug thereof according to claim 1 or 2, being represented by the following formula (II): 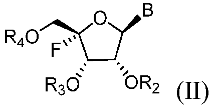 wherein,
B is selected from 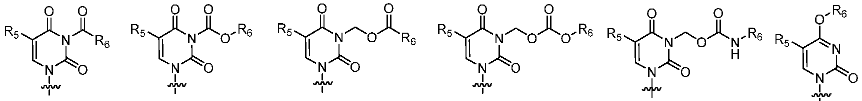 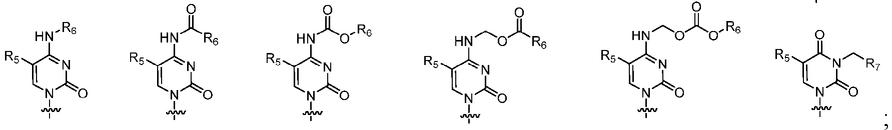
R₅ is selected from hydrogen and deuterium;
R₂, R₃, R₄, R₆, and R₇ are as defined in claim 1 or 2.

4. The compound, or the pharmaceutically acceptable salt, the solvate, the stereoisomer, the geometric isomer, the isotopically labeled compound or the prodrug thereof according to any one of claims 1-3, wherein,
R₆ is selected from C₁₋₂₀ alkyl, C₃₋₂₀ cycloalkyl, 3- to 20-membered heterocycloalkyl, C₂₋₂₀ alkenyl, C₆₋₂₀ aryl, 5- to 15-membered heteroaryl, C₃₋₂₀ cycloalkyl C₁₋₂₀ alkyl, C₁₋₂₀ alkylamino C₁₋₂₀ alkyl, C₁₋₂₀ dialkylamino C₁₋₂₀ alkyl, a group after removal of carboxyl from an amino acid, and R₂O-C₁₋₂₀ alkyl; wherein R₂ is C₁₋₂₀ alkanoyl; preferably, R₆ is selected from C₁₋₂₀ alkyl, C₃₋₁₀ cycloalkyl, 3- to 10-membered heterocycloalkyl, C₃₋₁₀ alkenyl, C₆₋₁₂ aryl, 5- to 12-membered heteroaryl, C₃₋₁₀ cycloalkyl C₁₋₁₀ alkyl, C₁₋₁₀ alkylamino C₁₋₁₀ alkyl, C₁₋₁₀ dialkylamino C₁₋₁₀ alkyl, a group after removal of carboxyl from an amino acid, and R₂O-C₁₋₁₀ alkyl; wherein R₂ is C₂₋₁₀ alkanoyl; more preferably, R₆ is selected from C₁₋₁₆ alkyl, C₃₋₈ cycloalkyl, 3- to 8-membered heterocycloalkyl, C₃₋₈ alkenyl, C₆₋₁₀ aryl, 5- to 10-membered heteroaryl, C₃₋₈ cycloalkyl C₁₋₈ alkyl, C₁₋₄ alkylamino C₁₋₈ alkyl, C₁₋₄ dialkylamino C₁₋₈ alkyl, a group after removal of carboxyl from an amino acid, and R₂O-C₁₋₈ alkyl; wherein R₂ is C₂₋₈ alkanoyl; preferably, the 3- to 8-membered heterocycloalkyl is azacycloalkyl, e.g., azetidin-3-yl, pyrrolidinyl, or piperidin-4-yl; preferably, the C₆₋₁₀ aryl is selected from phenyl and naphthyl; preferably, the 5- to 10-membered heteroaryl is 5- to 6-membered heteroaryl selected from pyridinyl, pyrimidinyl, pyrazinyl, furanyl, and thienyl, preferably N-containing heteroaryl, including pyridin-2-yl, pyridin-3-yl, pyridin-4-yl, pyrimidin-4-yl, pyrimidin-5-yl, and pyrazin-2-yl; the amino acid is preferably selected from L-alanine, L-valine, and L-phenylglycine; and/or
R₂ and R₃ are each independently selected from hydrogen, C₁₋₂₀ alkanoyl, C₆₋₂₀ aroyl, and 5- to 20-membered heteroaroyl, or R₂ and R₃ are connected with each other to form 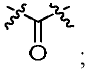 preferably, R₂ and R₃ are each independently selected from hydrogen, C₂₋₁₀ alkanoyl, C₆₋₁₂ aroyl, and 5- to 10-membered heteroaroyl, or R₂ and R₃ are connected with each other to form 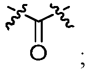 preferably, R₂ and R₃ are each independently selected from hydrogen, C₂₋₁₀ alkanoyl, C₆₋₁₀ aroyl, and 5- to 6-membered heteroaroyl, or R₂ and R₃ are connected with each other to form 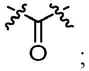 preferably, the C₆₋₁₀ aryl is selected from phenyl and naphthyl; the 5- to 6-membered heteroaryl is selected from pyridinyl, pyrimidinyl, pyrazinyl, furanyl, and thienyl, preferably N-containing heteroaryl, including pyridin-2-yl, pyridin-3-yl, pyridin-4-yl, pyrimidin-4-yl, pyrimidin-5-yl, and pyrazin-2-yl; and/or
R₄ is selected from hydrogen, C₁₋₂₀ alkanoyl, C₃₋₂₀ cycloalkanoyl, 3- to 20-membered heterocycloalkanoyl, C₆₋₂₀ aroyl, 5- to 20-membered heteroaroyl, and a group after removal of hydroxyl in carboxyl from an amino acid; preferably, R₄ is selected from hydrogen, C₂₋₁₆ alkanoyl, C₃₋₁₀ cycloalkanoyl, 3- to 10-membered heterocycloalkanoyl, C₆₋₁₂ aroyl, 5- to 10-membered heteroaroyl, and a group after removal of hydroxyl in carboxyl from an amino acid; preferably, R₄ is selected from hydrogen, C₂₋₁₆ alkanoyl, C₃₋₆ cycloalkanoyl, 3- to 6-membered heterocycloalkanoyl, C₆₋₁₀ aroyl, 5- to 6-membered heteroaroyl, and a group after removal of hydroxyl in carboxyl from an amino acid; preferably, the C₆₋₁₀ aryl is selected from phenyl and naphthyl; preferably, the 3- to 6-membered heterocycloalkanoyl is azacycloalkanoyl, e.g., azetidin-3-ylacyl or piperidin-4-ylacyl; preferably, the 5- to 6-membered heteroaryl is selected from pyridinyl, pyrimidinyl, pyrazinyl, furanyl, and thienyl, preferably N-containing heteroaryl, including pyridin-2-yl, pyridin-3-yl, pyridin-4-yl, pyrimidin-4-yl, pyrimidin-5-yl, and pyrazin-2-yl; the amino acid is selected from L-alanine, L-valine and L-phenylglycine;
wherein B, X, R₁, R₅, and R₇ are as defined in any one of claims 1-3.

5. The compound, or the pharmaceutically acceptable salt, the solvate, the stereoisomer, the geometric isomer, the isotopically labeled compound or the prodrug thereof according to any one of claims 1-4, wherein the compound has a structure represented by the following formula (III): 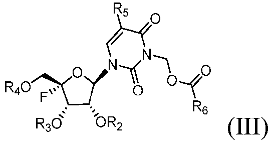
wherein, R₅ is selected from hydrogen and deuterium;
R₂, R₃, R₄, and R₆ are as defined in any one of claims 1-4.

6. Compound, or pharmaceutically acceptable salt, solvate, stereoisomer, geometric isomer, isotopically labeled compound or prodrug thereof, wherein the compound is any one of:

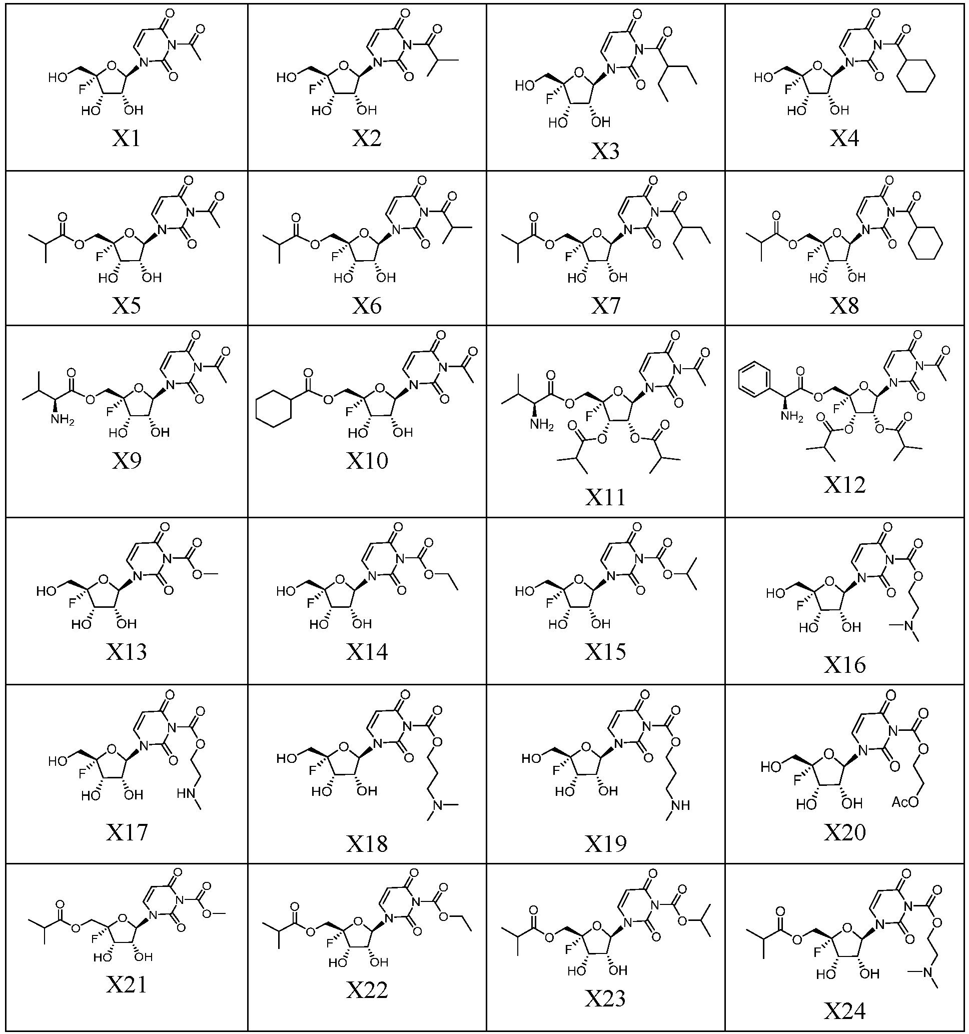

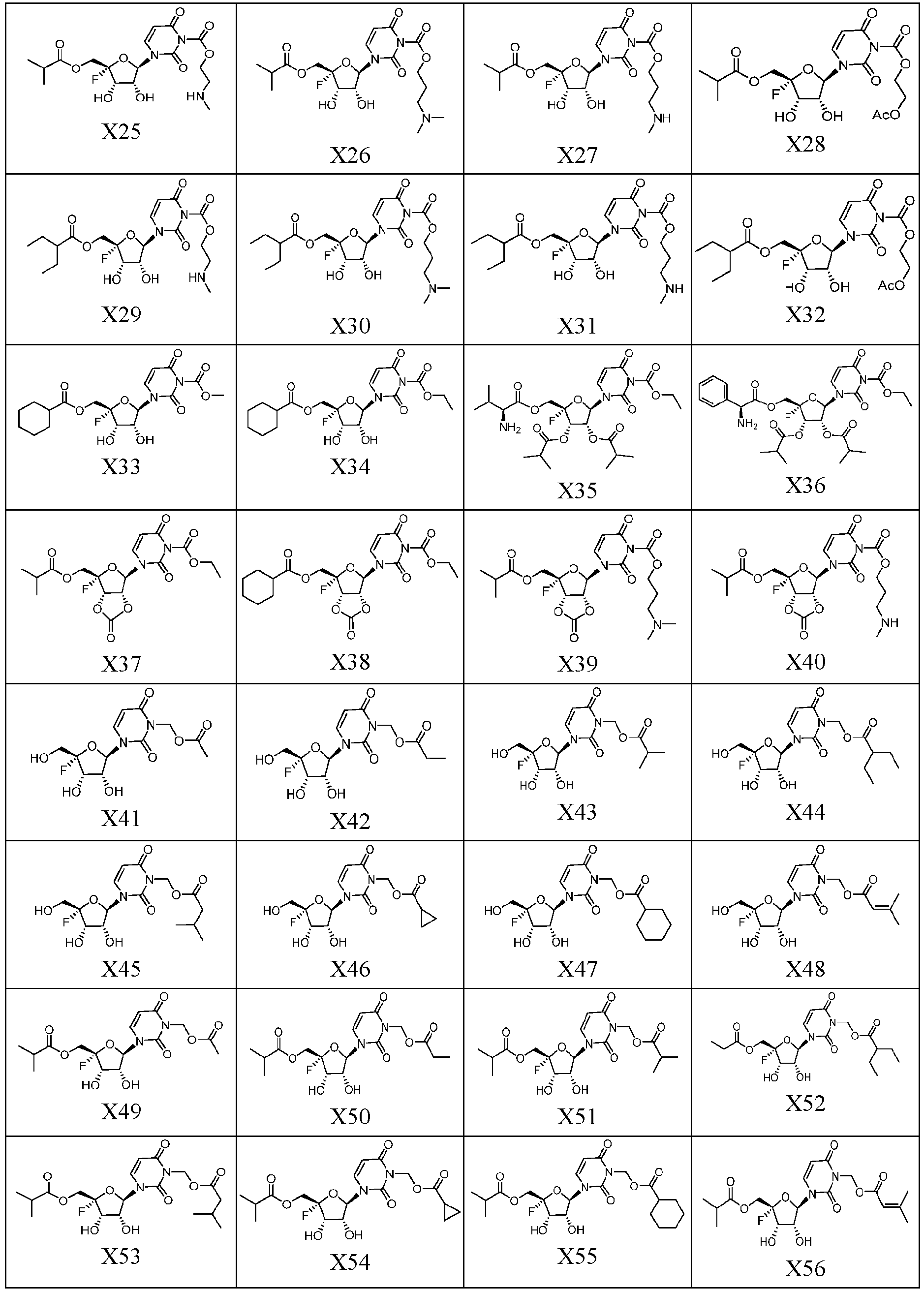

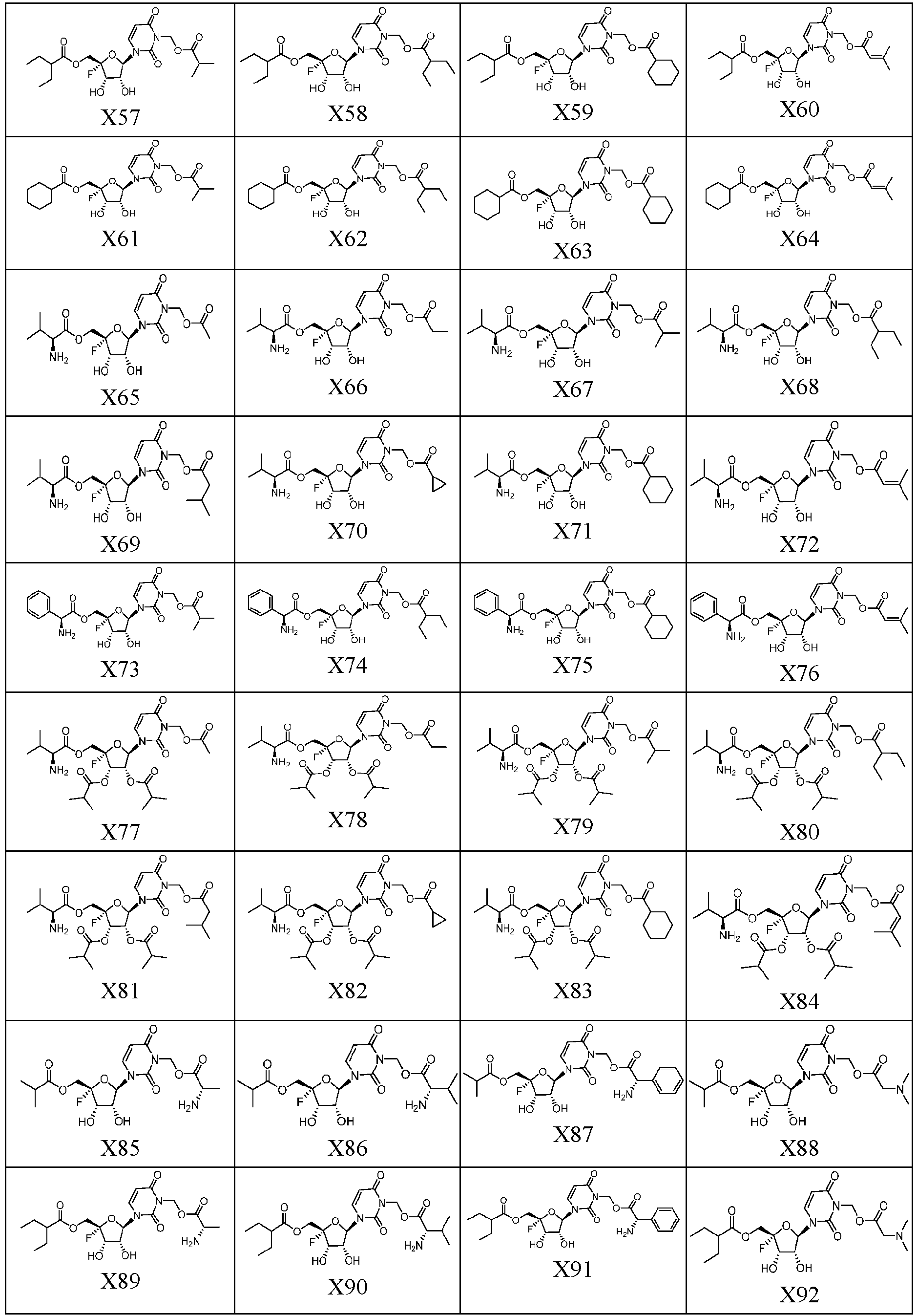

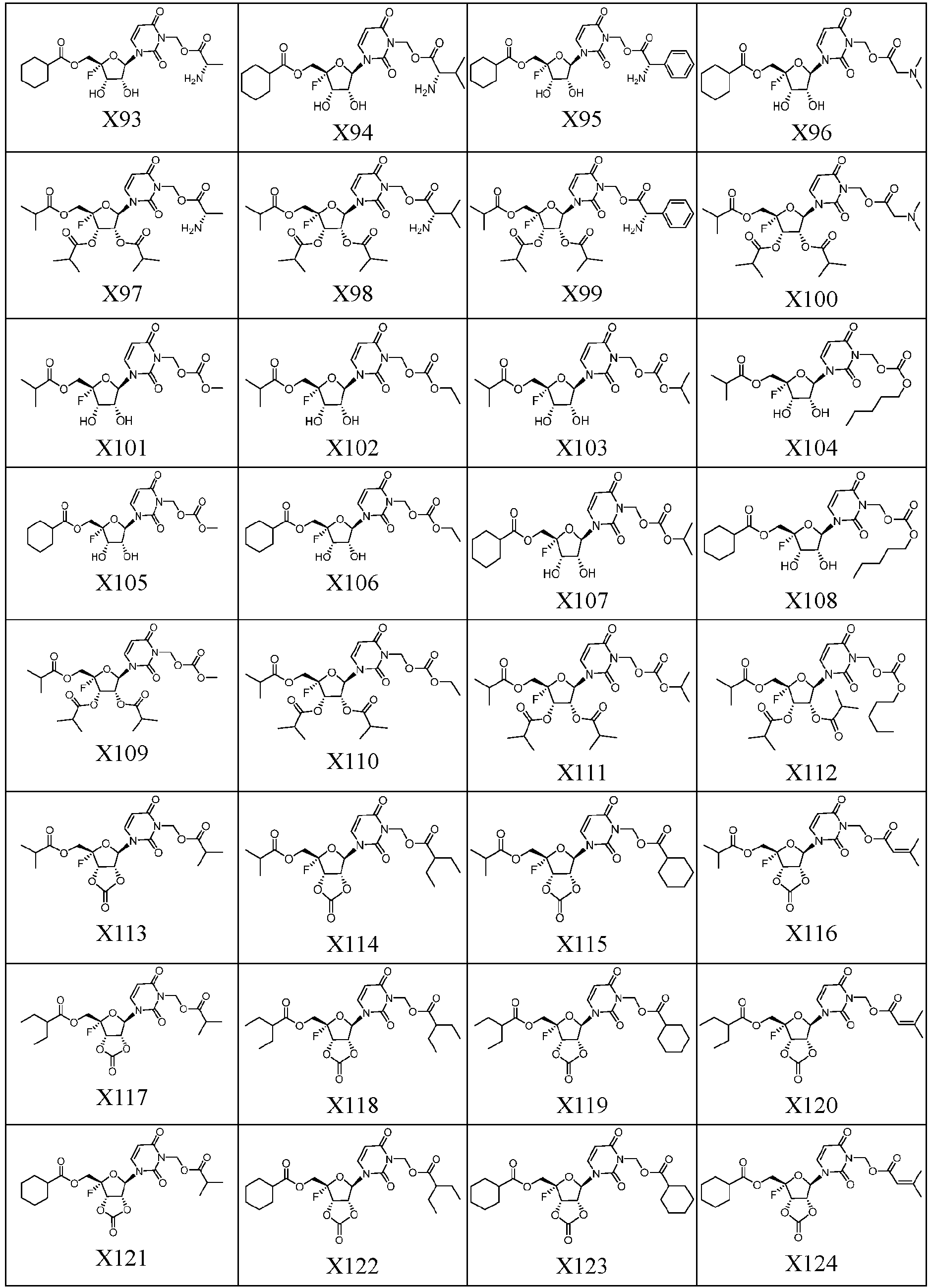

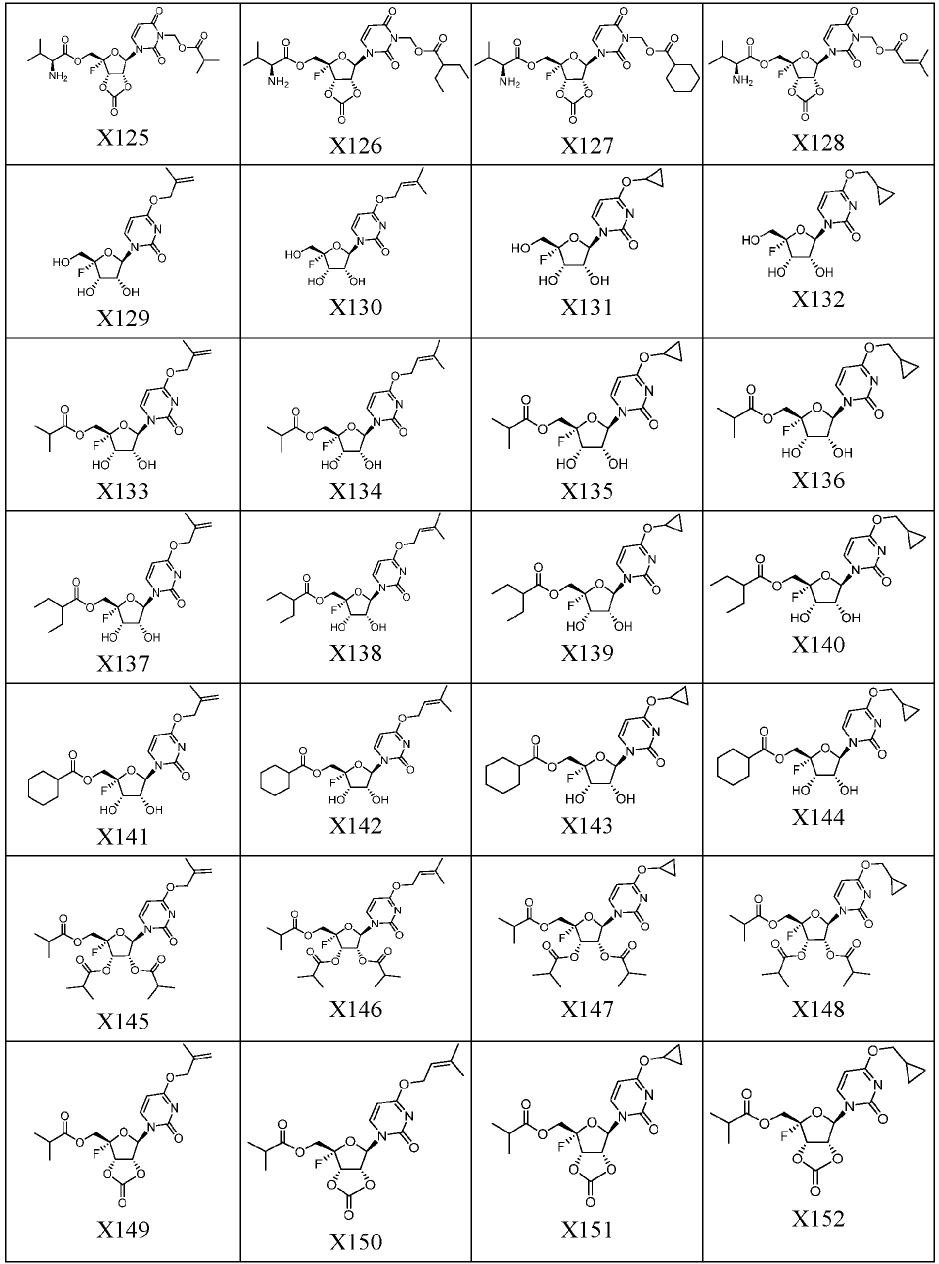

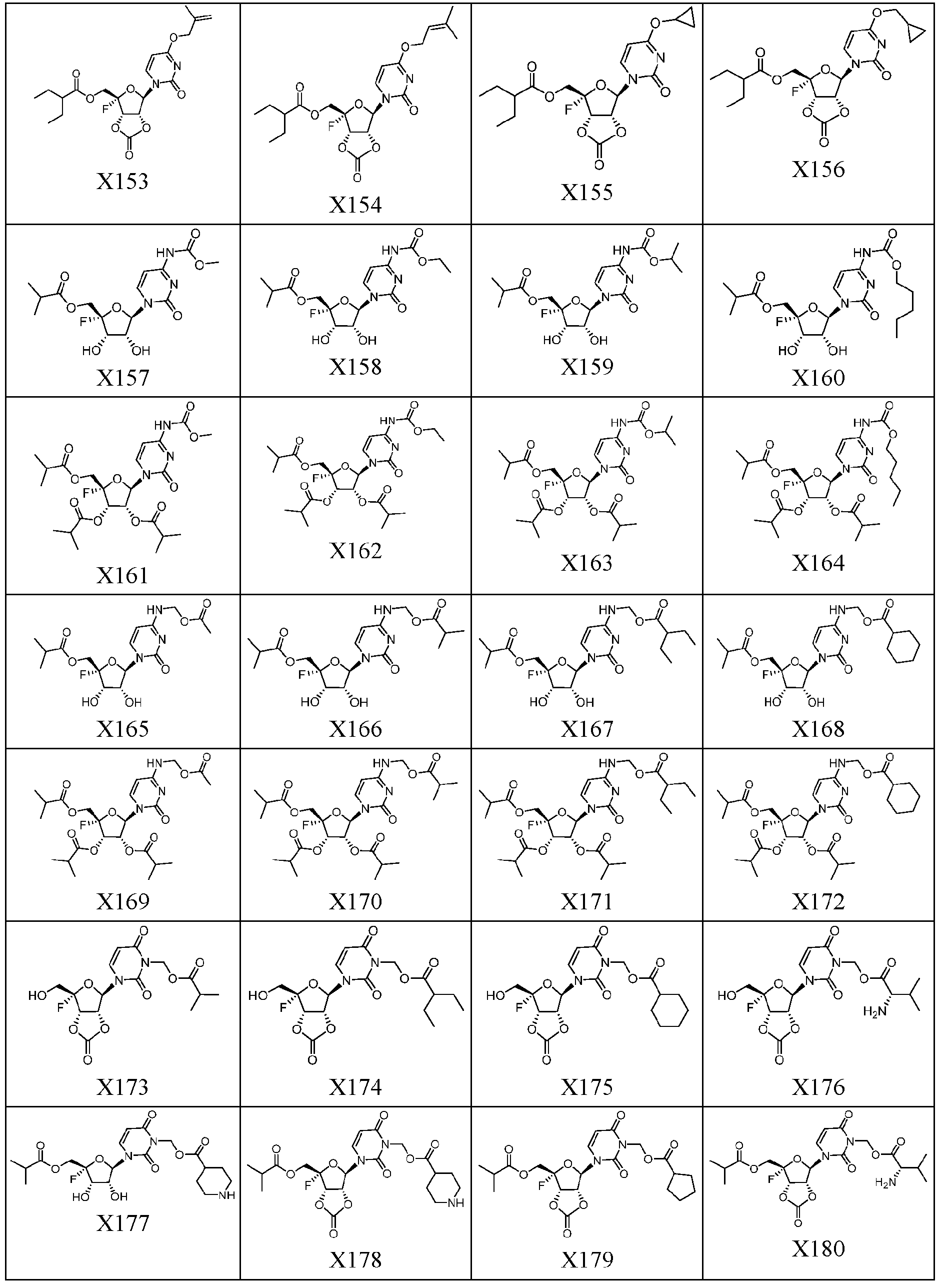

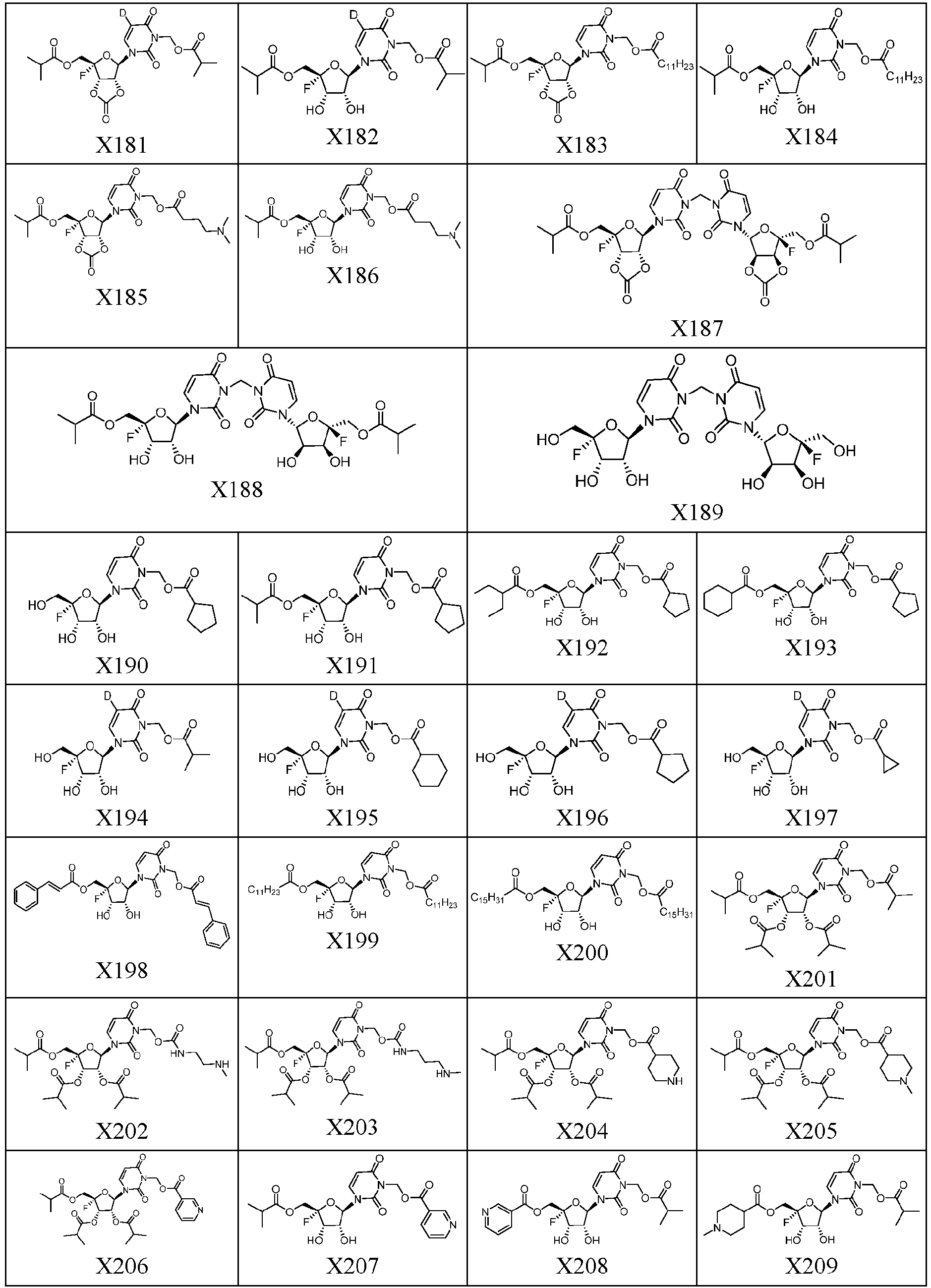

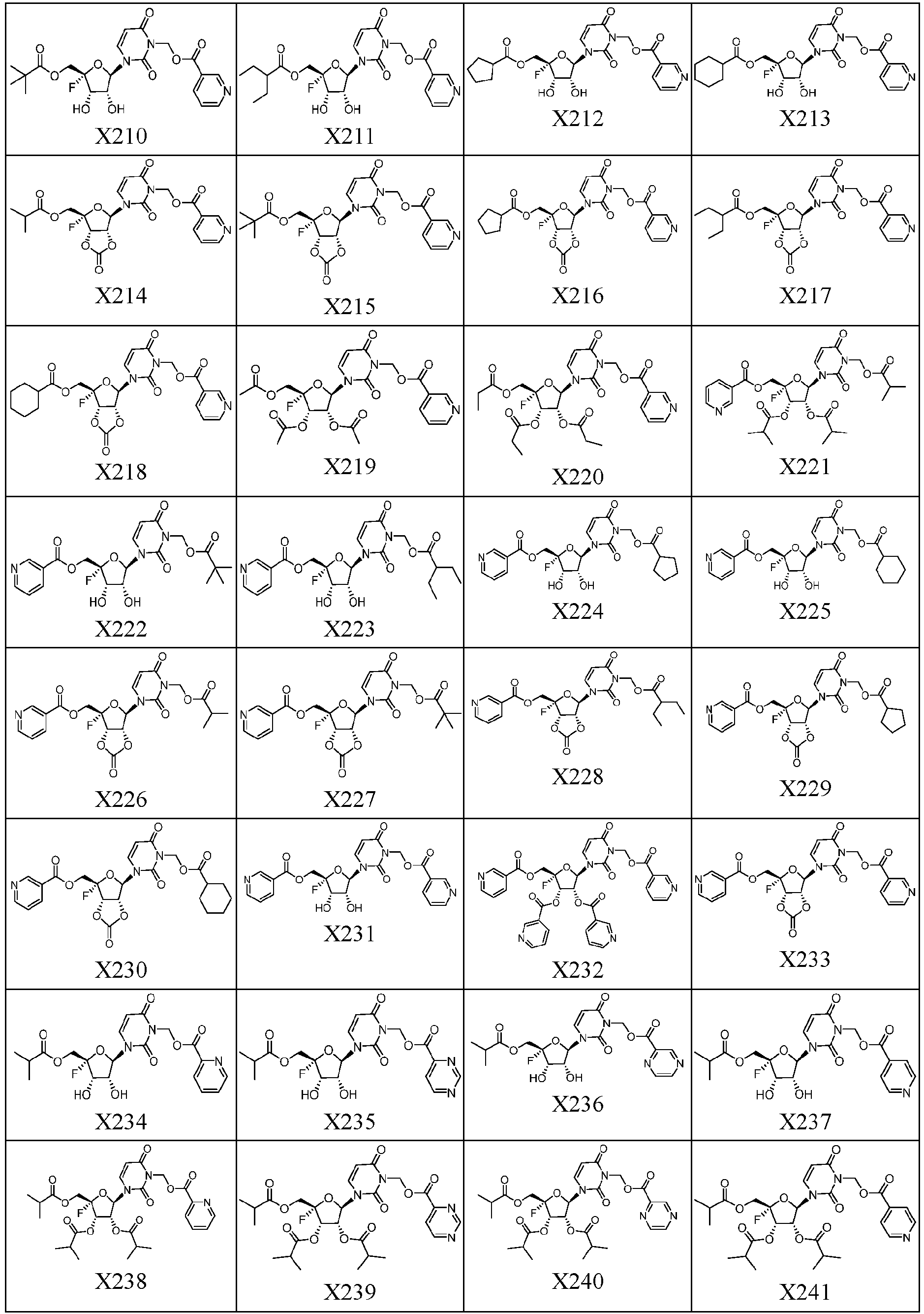

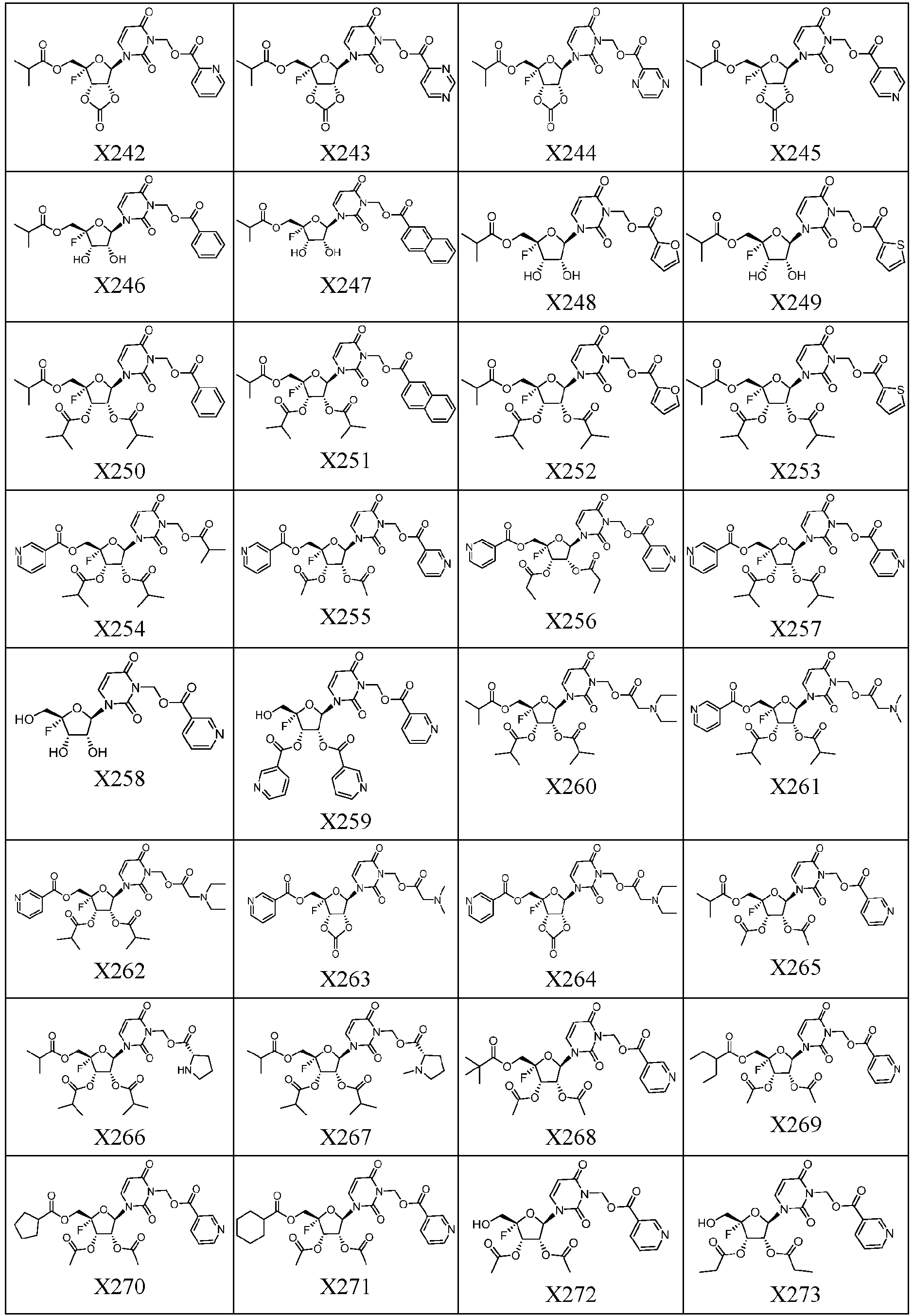

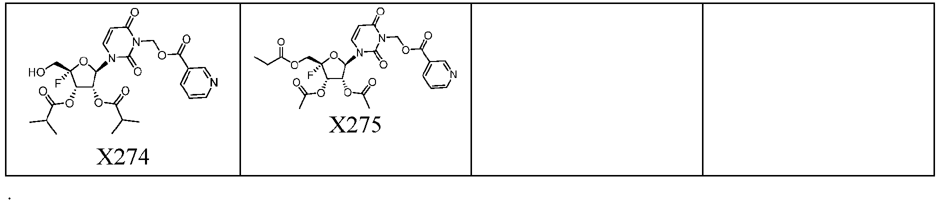

7. A pharmaceutically acceptable salt of a compound, being selected from the following structures: 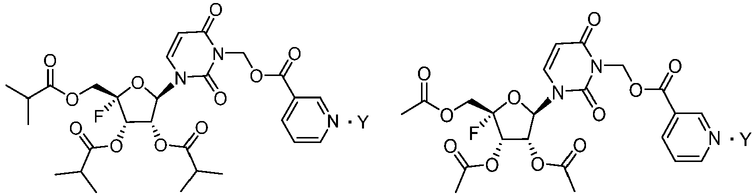 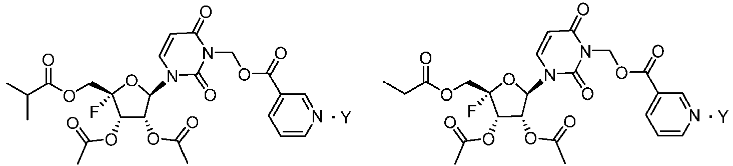 wherein Y is selected from hydrogen chloride, hydrogen bromide, hydrogen iodide, sulfuric acid, hemisulfuric acid, nitric acid, phosphoric acid, maleic acid, succinic acid, citric acid, tartaric acid, fumaric acid, formic acid, acetic acid, propionic acid, malonic acid, oxalic acid, benzoic acid, phthalic acid, methanesulfonic acid, ethanesulfonic acid, benzenesulfonic acid, toluenesulfonic acid, naphthalenesulfonic acid, 1,5-naphthalenedisulfonic acid, camphoric acid, camphorsulfonic acid, salicylic acid, acetylsalicylic acid, aspartic acid, glutamic acid, lactic acid, gluconic acid, ascorbic acid, gallic acid, mandelic acid, malic acid, sorbic acid, trifluoroacetic acid, taurine, homotaurine, 2-hydroxyethanesulfonic acid, cinnamic acid, or mucic acid; Y are each preferably hydrogen chloride, hydrogen bromide, sulfuric acid, hemisulfuric acid, or methanesulfonic acid, and more preferably hydrogen chloride or hydrogen bromide.

8. A pharmaceutical composition, comprising:
the compound, and the pharmaceutically acceptable salt, the solvate, the stereoisomer, the geometric isomer, the isotopically labeled compound or the prodrug thereof according to any one of claims 1-6, or one or more of the pharmaceutically acceptable salts of a compound according to claim 7, and a pharmaceutically acceptable carrier.

9. The compound, or the pharmaceutically acceptable salt, the solvate, the stereoisomer, the geometric isomer, the isotopically labeled compound or the prodrug thereof according to any one of claims 1-6, or the pharmaceutically acceptable salt of a compound according to claim 7, or the pharmaceutical composition according to claim 8, for use in inhibiting viral replication, and/or in preventing, alleviating, and/or treating a disease caused by a viral infection.

10. The compound, or the pharmaceutically acceptable salt, the solvate, the stereoisomer, the geometric isomer, the isotopically labeled compound or the prodrug thereof according to any one of claims 1-6, or the pharmaceutically acceptable salt of a compound according to claim 7, or the pharmaceutical composition according to claim 8, for use according to claim 9, wherein, the virus is selected from one or more of the following viruses:
a paramyxovirus, preferably a parainfluenza virus, a measles virus, a Nipah virus, or a respiratory syncytial virus RSV;
an influenza virus, preferably an influenza A virus, an influenza B virus, an influenza C virus, or an influenza D virus;
a virus of the family Bunyaviridae, preferably a virus of the genus *Bunyavirus,* the genus *Phlebovirus,* the genus *Nairovirus,* or the genus *Hantavirus;*
an arenavirus, preferably a Lassa fever virus LASV, a Junin virus JUNV, or a Machupo virus MACV;
a virus of the family Flaviviridae, preferably a hepatitis C virus HCV, a Dengue virus DENV, or a Zika virus ZIKV;
a filovirus, preferably a Marburg virus MBV, an Ebola virus EBV, or a Cuevavirus; and
a coronavirus, preferably a human-infected coronavirus or an animal-infected coronavirus, wherein more preferably, the human-infected coronavirus is severe acute respiratory syndrome coronavirus SARS-CoV, 2019 novel coronavirus SARS-CoV-2, Middle East respiratory syndrome coronavirus MERS-CoV, human coronavirus OC43, human coronavirus 229E, human coronavirus NL63, or human coronavirus HKUl, and the animal-infected coronavirus is porcine epidemic diarrhea virus PEDV or feline infectious peritonitis virus FIFV.

11. The compound, or the pharmaceutically acceptable salt, the solvate, the stereoisomer, the geometric isomer, the isotopically labeled compound or the prodrug thereof according to any one of claims 1-6, or the pharmaceutically acceptable salt of a compound according to claim 7, or the pharmaceutical composition according to claim 8, for use according to claim 9, wherein, the disease caused by a viral infection is selected from one or more of the following diseases:
common cold, a high-risk symptom infection, a respiratory tract infection, pneumonia, and complications thereof caused by a respiratory syncytial virus RSV infection;
common cold, a high-risk symptom infection, a respiratory tract infection, pneumonia, and complications thereof caused by an influenza virus infection;
an infection and complications thereof caused by a bunya virus infection;
an infection and complications thereof caused by an arenavirus infection;
chronic hepatitis C and complications thereof caused by a hepatitis C virus infection;
Dengue fever and complications thereof caused by a Dengue virus infection;
an infection and complications thereof caused by a Zika virus infection;
hemorrhagic fever and complications thereof caused by a Marburg virus or Ebola virus infection;
common cold, a high-risk symptom infection, a respiratory tract infection, pneumonia, and complications thereof caused by a human coronavirus infection;
porcine epidemic diarrhea caused by a porcine epidemic diarrhea virus infection; and
feline infectious peritonitis caused by a feline coronavirus infection.

12. The compound, or the pharmaceutically acceptable salt, the solvate, the stereoisomer, the geometric isomer, the isotopically labeled compound or the prodrug thereof according to any one of claims 1-6, or the pharmaceutically acceptable salt of a compound according to claim 7, or the pharmaceutical composition according to claim 8, for use according to claim 9, wherein, the disease caused by a viral infection is a disease caused by a respiratory syncytial virus RSV infection; preferably, the disease caused by a respiratory syncytial virus RSV infection is selected from one or more of the following diseases: a respiratory tract infection, pneumonia, and complications thereof; or
the disease caused by a viral infection is a disease caused by an influenza virus infection; preferably, the disease caused by an influenza virus infection is selected from one or more of the following diseases: common cold, a high-risk symptom infection, a respiratory tract infection, pneumonia, and complications thereof; or
the disease caused by a viral infection is a disease caused by a bunya virus infection; preferably, the disease caused by a bunya virus infection is selected from one or more of the following diseases: fever with thrombocytopenia syndrome, hemorrhagic fever with renal syndrome, influenza-like diseases, encephalitis, and complications thereof.
